Europäisches Patentamt

European Patent Office

Office européen des brevets

(19)

(11) EP 1 034 305 B1

(12) EUROPEAN PATENT SPECIFICATION

(45) Date of publication and mention
of the grant of the patent:
**12.03.2003 Bulletin 2003/11**

(21) Application number: **98960509.2**

(22) Date of filing: **25.11.1998**

(51) Int Cl.⁷: **C12Q 1/68**

(86) International application number:
**PCT/US98/25294**

(87) International publication number:
**WO 99/027140 (03.06.1999 Gazette 1999/22)**

(54) **INTEGRATED CIRCUIT BIOCHIP MICROSYSTEM**

MIKROSYSTEM MIT INTEGRIERTER BIOCHIPSCHALTUNG

MICROSYSTEME SOUS FORME DE BIOPUCE A CIRCUIT INTEGRE

(84) Designated Contracting States:
**AT BE CH CY DE DK ES FI FR GB GR IE IT LI LU
MC NL PT SE**
Designated Extension States:
**AL LT LV MK RO SI**

(30) Priority: **26.11.1997 US 979672**

(43) Date of publication of application:
**13.09.2000 Bulletin 2000/37**

(60) Divisional application:
**02009976.8 / 1 236 807**

(73) Proprietor: **UT-BATTELLE, LLC**
**Oak Ridge, Tennessee 37831-6498 (US)**

(72) Inventors:
• **VO-DINH, Tuan**
**Knoxville, TN 37922 (US)**
• **WINTENBERG, Alan**
**Knoxville, TN 37922 (US)**
• **ERICSON, Milton, N.**
**Knoxville, TN 37922 (US)**

(74) Representative: **Glawe, Delfs, Moll & Partner
Patentanwälte
Rothenbaumchaussee 58
20148 Hamburg (DE)**

(56) References cited:
**WO-A-93/22678          WO-A-97/12030
WO-A-97/12225**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

**1.1 FIELD OF THE INVENTION**

This invention relates to novel biochips that combine integrated circuit elements, electro-optical excitation and detection systems, and molecular receptor probes in a self-contained integrated microdevice.

**1.2 DESCRIPTION OF RELATES ART**

**1.2.1 BIOSENSORS**

Biosensors use recognition properties of living systems which possess extremely selective bioreceptors (*e. g.*, antibody, enzyme, gene probes *etc.*) which allow specific identification and detection of complex chemical and biological species. A wide variety of chemical sensors, biosensors, bioanalytical instrumentation using laser-induced fluorescence (Stevenson *et al.,* 1994), antibody-based immunofluorescence (Vo-Dinh *et al.* 1987; Vo-Dinh *et al.,* 1991), and gene probes Vo-Dinh *et al,* 1994; Isda *et al.,* 1996, Isola *et al.,* 1998) have been developed in recent years. Due to the exquisite specificity of the DNA hybridization process, there is an increasing interest in the development of DNA bioreceptor-based analytical systems (Vo-Dinh *et al.,* 1994; Isola *et al.,* 1996; Isola *et al.,* 1998; Schena *et al.,* 1995; Piunno *et al.,* 1995; Kumar *et al.,* 1994; Eggers *et al.,* 1994; Vo-Dinh *et al.,* 1998; Fodor *et al.,* 1991; McGall *et al.,* 1997). Gene probes using a detection scheme based on surface-enhanced Raman scattering (SERS) labels were recently developed to enhance both the selectivity and sensitivity of DNA biosensors (Vo-Dinh *et al.,* 1994; Isola *et al.,* 1998). Recently the development of a fluorescence-based fiberoptic genosensor for the *Mycobacterium tuberculosis* has been reported (Isola *et al.,* 1996), and a surface-enhanced Raman scattering (SERS) system for HIV detection (Isola *et al.,* 1998).

**1.2.2 BIOSENSORS BASED ON BIOCHIP DESIGNS**

A basic interest has been in the development of inexpensive biosensors for environmental and biomedical diagnostics. Biosensors have been investigated, mostly based on DNA probes and on various systems for analysis of oligonucleotide arrays, but there appears to be limited consideration and development of integrated circuit (IC) gene probe-based biosensors on microchips. Existing systems typically employ photomultipliers or 2-dimensional detectors such as charge-coupled device (CCD) systems which require bulky electronic and data conditioning accessories (Affymetrix® http, 1997; Schena *et al.,* 1995; Piunno *et al.,* 1995; Kurnar *et al.,* 1994; Eggars *et al.,* 1994; Graham *et al.,* 1992).

Despite significant strides in developing DNA chips, detection and analysis methods have not been well developed to take advantage of the amount of information that such chips can obtain in a short period of time. A common technique for detecting DNA probes involves labeling the probe with radioactive tags and detecting the probe target hybrids by autoradiography. Phosphorous-32 ($^{32}$P) is the most common radioactive label used because of its high-energy emission and, consequently short exposure time. Radioactive label techniques, however, suffer several disadvantages, such as limited shelf life. For example, $^{32}$P has a limited shelf life because it has a 14-day half-life.

Several optical detection systems based on surface-enhanced Raman fluorescence of visible and near-infrared (NIR) dye probe labels have been investigated (Vo-Dinh *et al.,* 1987; Isola *et al.,* 1996) for non-radioactive detection of tagged gene probes. Fluorescence detection is extremely sensitive when the target compounds or labeled systems are appropriately selected. Indeed, a zeptomole ($10^{-21}$ mole) detection limit has been achieved using fluorescence detection of dyes with laser excitation (Stevenson *et al.,* 1994). Even so, detection systems are macro compared to the micro world of DNA arrays, as many detection/analysis methods are mere adaptations from other systems. This means that analysis is relatively slow, compared to data accumulation.

**1.2.3 POLYNUCLEOTIDE-DETECTING BIOCHIPS**

Much interest has centered recently on the development of DNA chips based on high density oligonucleotide arrays and fluorescence analysis such as described by Hacia *et al.* (1996). This principle has been commercialized in the Affymetrix® GeneChip® which was developed to process large amounts of genetic information. GeneChip® probe arrays are arranged on single chips in the form of tens of thousands of DNA probes that are designed to fluorescence when hybridized to their targets. The light is scanned with laser light and the light intensity stored for later computations (July 23, 1997, Affymetrix, Inc., http://www.affymetrix.com/).

Unfortunately, the DNA chips, while much like the microprocessor chips that currently run today's technology, have yet to be successfully developed into integrated systems that conveniently interpret what information can be captured by DNA chips. Thus, an Affymetrix® chip that is stated to detect HIV mutations still requires an external scanning and interpretation of the signals that are generated by a DNA-captured nucleic acid.

### 1.2.4 MICROORGANISM DETECTION AND IDENTIFICATION USING BIOCHIPS

The detection of biological species in complex systems is important for many biomedical and environmental applications. In particular, there is a strong interest in developing detection techniques and sensors for use in such applications as infectious disease identification, medical diagnostics and therapy, as well as biotechnology and environmental bioremediation. An objective in developing new techniques and sensors is not only to be able to selectively identify target compounds but to be able to assay large numbers of samples. Yet, there remain problems in reproducibly detecting and measuring low levels of biological compounds conveniently, safely and quickly.

### 1.3 DEFICIENCIES IN THE PRIOR ART

There is currently a strong need for a truly integrated biochip system that comprises probes, samplers, detector as well as amplifier and logic circuitry on board. Such a system will be useful in many environments, including *inter alia*, physicians offices, and can be used by relatively low-skilled personnel. Until now, *most* DNA biosensors previously reported are based on fiberoptic probes, glass and silica plates used as the probe substrates which are externally connected to a photosensing system, which generally consists of a conventional detection device, such as a photomultiplier, or a charge-coupled device (CCD). Although the probes on the sampling platform are small (often referred to as a 'DNA chip' or 'gene chip'), the entire device containing excitation laser sources and detection systems (often a confocal microscope system) is relatively large, *e.g.*, table-top size systems. Although these systems have demonstrated their usefulness in genomics research and analysis, they are laboratory-oriented and involve relatively expensive equipment.

Likewise, there is a need to develop biochip based sensors useful in the detection and quantitation of macromolecules other than DNAs. The development of sensors useful in the detection of molecules such as RNAs, peptidenucleic acids (PNAs), ribozymes, polypeptides, antibodies, enzymes, peptide fragments, would represent a significant advance in the art and provide new methods and devices for the detection of molecules of biological importance. Furthermore, because of recent advances in the molecular sciences, the ability to detect and quantitate biomimetics, and new classes of biologically-active molecules such as DNA adaptamers (1), cyclodextrins (2), dendrimers (3 ), molecular imprints ( 4 ), and the like would benefit from the creation of biochip-based apparatus capable of detecting and quantitating these, and other biologically- and medically-relevant macromolecules.

>    (1) Erdeniz et al., 1997
>    (2) Topchieva et al., 1998
>    (3) Sakthievel et al., 1998; Bulte et al., 1998
>    (4) Mosbach, 1994

There is therefore a distinct need for development of systems, devices, and apparatus that will allow rapid, large-scale and cost effective analysis of these macromolecules, and permit the development of methods for detecting and quantitating biologically-relevant molecules.

WO-A-93/22678 discloses a microchip to detect DNA hybridization based on an array of test sites, which build a monolithically integrated structure formed on (or in) a semiconductor wafer using very large scale integrated circuit methods. Optical detection of the electromagnetic (UV, VIS or IR) response is achieved by a CCD sensor array monolithically integrated in the wafer.

WO-A-97/12225 discloses an integrated optic interferometric sensor in which the optical signal is enhanced and processed by (1) treating a portion of the planar waveguide structure and (2) integrating a beam processing region between the two regions of the waveguide. The integrated signal processing capabilities increase the signal-to-noise ratio by more than one order of magnitude over a similar multimode device.

### 2. Summary of the Invention

The invention comprises an integrated microchip biosensor device as defined in the claims.

Such a device may employ multiple optical sensing elements and microelectronics on a single integrated chip combined with one or more nucleic acid-based bioreceptors designed to detect sequence specific genetic constituents in complex samples. The microchips combine integrated circuit elements, electrooptics, excitation/detection systems and nucleic acid-based receptor probes in a self-contained and integrated microdevice. A basic biochip, for example, may include: (1) an excitation light source; (2) a bioreceptor probe; (3) a sampling element; (4) a detector; and (5) a signal amplification/treatment system.

The integrated circuit biomierochips of the present invention comprise an integrated circuit that includes an optical transducer and associated optics and circuitry for generating an electrical signal in response to light or other radiation indicative of the presence of a target biological species, particularly a nucleic acid. The chip may also include a support for immobilizing a bioprobe, which is preferably a nucleic acid. In particular embodiments, a target nucleic acid may be tagged or labeled with a substance that emits a detectable signal; for example, luminescence.

Alternatively, the bioprobe attached to the immobilized bioprobe may be tagged or labeled with a substance that emits a detectable or altered signal when combined with the target nucleic acid. The tagged or labeled species may be fluorescent, phosphorescent, or otherwise luminescent, or it may emit Raman energy or it may absorb energy.

The highly integrated biosensors of the present invention are advantageous in part because of fabricating multiple optical sensing elements and microelectronics on a single integrated circuit, and further combining the chip in preferred embodiments with a plurality of molecular hybridization probes (Geiger *et al.,* 1990; Aubert *et al.,* 1988). When the probes selectively bind to a targeted species, a signal is generated that is picked up by the chip. The signal may then be processed in several ways, depending on the nature of the signal.

In one aspect, the present invention concerns an integrated system that includes (1) a targeted nucleic acid sequence in combination with a biological probe which is modified to receive light or other radiation of a first frequency and thereby to emit light or other radiation of a different frequency than the first frequency, and (2) to detect the emitted radiation by means of a phototransducer. The target nucleic acid is typically a uniquely characteristic gene sequence of a pathogen such as a fungus, bacteria, or virus, or other distinct nucleic acid species such as may be found in mutant mammalian cells or in individuals with inherited errors of metabolism. The target nucleic acid is modified or labeled to include a tag or label that emits a signal upon exposure to an incident light or other radiation.

The target nucleic acid may be immobilized onto the integrated microchip that also supports a phototransducer and related detection circuitry. Alternatively, a gene probe may be immobilized onto a membrane or filter which is then attached to the microchip or to the detector surface itself, such as the transducer detector described herein. This approach avoids the need to bind the bioreceptor directly to the transducer and thus is attractive for simplifying large-scale production

In one preferred embodiment of the invention, light of a highly directional or focused nature is impinged on a target nucleic that inherently or by virtue of an appropriate tag or label will emit a detectable signal upon irradiation. The irradiation may be provided by a suitable light source, such as a laser beam or a light-emitting diode (LED). With the Raman, fluorescence and phosphoroscence detection modes, the incident light is further kept separate from the emitted light using different light paths and/or appropriate optical filters to block the incident light from the detector.

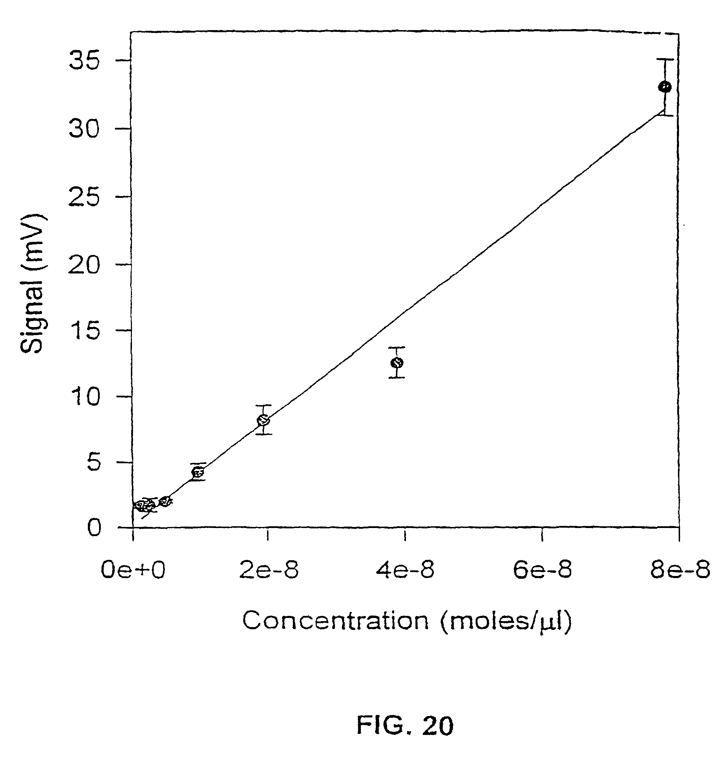

A target nucleic acid sequence is preferably hybridized with a nucleic acid sequence that is selected for that purpose (bioprobe). As stated earlier, the selected bioprobe is immobilized on a suitable substrate, either on the biochip itself or on a membrane type material that is then contacted or attached to the chip surface. The bioprobe may be labeled with a tag that is capable of emitting light or other non-radioactive energy. Upon hybridization with a target nucleic acid sequence, the hybrid product can be irradiated with light of suitable wavelength and will emit a signal in proportion to the amount of target nucleic acid hybridized, see FIG. 20. The labeled bioprobe may comprise a labeled molecular bioreceptor. Known receptors are advantageous to use because of their known ability to selectively bind with the target nucleic acid sequence. In certain particular examples, the bioreceptor itself may exhibit changes in light emission when its cognate is bound.

In certain applications, it may be desirable to increase the amount of biotarget when only trace quantities are present in a sample. The present invention is compatible with polymerase chain reaction (PCR), which is a technique to amplify DNA sequences.

There are several methods for selectively identifying biological species, including antibody detection and assay as in the well-known Enzyme-linked Immunosuppresent Assays (ELISA) employing molecular hybridization techniques. Generally speaking, it is possible to identify sequence-specific nucleic acid segments, and to design sequences complementary to those segments, thereby creating a specific probe for a target cell, such as different pathogen cells or even mammalian cells that have mutated from their normal counterparts. In principle, one can design complementary sequences to any identified nucleic acid segment. In many instances, unique sequences specific to an organism may be used as probes for a particular organism or cell type. The quantitative phenotypic analysis of yeast deletion mutants, for example, has utilized unique nucleic acid sequence identifiers to analyze deletion strains by hybridization with tagged probes using a high-density parallel array (Shoemaker *et al.*, 1996).

Hybridization involves joining a single strand of nucleic acid with a complementary probe sequence. Hybridization of a nucleic acid probe to nucleic acid sequences such as gene sequences from bacteria, or viral DNA offers a very high degree of accuracy for identifying nucleic acid sequences complementary to that of the probe. Nucleic acid strands tend to be paired to their complements in double-stranded structures. Thus, a single-stranded DNA molecule will seek out its complement in a complex mixture of DNA containing large numbers of other nucleic acid molecules. Hence, nucleic acid probe (*e.g.*, gene probe) detection methods are very specific to DNA sequences. Factors affecting the hybridization or reassociation of two complementary DNA strands include temperature, contact time, salt concentration, the degree of mismatch between the base pairs, and the length and concentration of the target and probe sequences. In perhaps the simplest procedure, hybridization is performed on an immobilized target or a probe molecule attached on a solid surface such as a nitrocellulose or nylon membrane or a glass plate.

## 3.0 BRIEF DESCRIPTION OF THE DRAWINGS

For a more complete understanding of the present invention and the advantages thereof, reference is now made to the following description taken in conjunction with the accompanying drawings in which like reference numbers indicate like features and wherein:

FIG. 1 illustrates a schematic, exploded view of an example of a DNA biochip of the invention.

FIG. 2 illustrates a schematic diagram of one possible optical detector and amplifier circuit that may be implemented on an integrated circuit in order to convert an optical signal into an electrical signal suitable for data digitization and capture by a computer.

FIG. 3 is a perspective, partially enlarged and exploded schematic view of a biochip having multiple arrays of exciting light sources and detectors.

FIG. 4 is a schematic, sectional view of an integrated circuit microchip system for a biochip with integrated light emitting diode excitation sources.

FIG. 5 illustrates a schematic circuit diagram for an integrated light emitting diode (LED) light source and phototransistor detection device.

FIG. 6 illustrates a schematic layout of an integrated circuit that implements the light source and detection device illustrated in FIG. 5.

FIG. 7 illustrates a physical layout of a large-area, 4 x 4 $n$-well integrated amplifier-photodiode array designed as a single, custom integrated circuit.

FIG. 8 illustrates a schematic cross-section of an n-well photodiode used in the photodiode array of FIG. 7.

FIG. 9 illustrates a detection circuit for use in conjunction with the photodiode array of FIG. 7.

FIG. 10 illustrates an alternative detection circuit for use in conjunction with the photodiode array of FIG. 7.

FIG. 11 illustrates schematically an analog multiplexer that enables any element in the photodiode array of FIG. 7 to be connected to an amplifier.

FIG. 12 illustrates one possible schematic implementation of the multiplexer in FIG. 11 for use with 16 cells.

FIG. 13 illustrates schematically a partially-parallel system that may be used to obtain data from the photodiode array shown in FIG. 7. The partially-parallel system has only a read-out system for every row of photodetectors.

FIG. 14 illustrates a fully-parallel system that may be used to obtain data from the photodiode array shown in FIG. 7. The fully-parallel device has a read-out system (amplifier, electronics) for every photodetector.

FIG. 15 shows a calibration curve for an NIR dye-labeled single-stranded DNA (5'-CCTCCTCCTTCCCAGCAGGG-3'; SEQ ID NO:1) over a concentration range from 1 pmol/$\mu$L to 3 fmol/$\mu$L.

FIG. 16 illustrates the results of measurements of gene probes tagged with fluorescein, a dye label emitting in the visible range.

FIG. 17 illustrates the performance of an integrated circuit microchip (ICM) phototransistor and amplifier circuit consisting of a 2 $\mu$m, $p$-well CMOS process occupying an area of 160,000 square microns and 220 phototransistor cells connected in parallel by showing the signal output response for various concentrations of the dye label Rhodamine-6G excited with a small helium-cadmium laser (8 mW, 325 nm).

FIG. 18 illustrates schematically an experimental setup that may be used to evaluate a 4 x 4 array amplifier/ phototransistor integrated circuit microchip device.

FIG. 19 illustrates the results when four sample spots of 1 $\mu$L of fluorescein labeled DNA were placed on a nitrocellulose membrane that was translated over a detection channel of the device shown in FIG. 18.

FIG. 20 illustrates the calibration curve of fluorescein labeled DNA using the device shown in FIG. 18.

FIG. 21 illustrates an embodiment of the present invention that is used for absorption and reflection measurements.

FIG. 22 is a schematic vertical section of a portion of a biochip of the invention that may operate with either luminescent or Raman radiation.

FIG. 23 is a schematic vertical section of a portion of a biochip that may employ luminescent or Raman energy for detection, or may operate by detecting the degree of absorption of a light beam.

FIG. 24 illustrates a plan view of one embodiment of the present invention that is used to detect samples from a microfluidic device $via$ a plurality of cells.

FIG. 25 illustrates a schematic vertical section of a microfluidic injection system for liquid and gas sample in and out of the ICM chip used in the embodiment illustrated by FIG. 24.

FIG. 26 illustrates an embodiment of the present invention that is used to detect samples from a microfluidic device using an imaging lens, binary optics, or a lens array to image each microfluidic channel onto a detector element of the ICM.

**FIG. 27** illustrates a micro-electromechanical system (MEMS) that is used to construct an ICM with Random Access Microsensing.

**FIG. 28** illustrates an overview of an ICM system that uses the MEMS depicted in FIG. 27.

**FIG. 29** illustrates an embodiment of the present invention that uses individually addressable, integrated vertical-cavity surface-emitting lasers (VCSEL) and on-axis and/or off-axis diffractive lenses.

**FIG. 30** is an LED driver circuit where $V_1$ is a DC voltage (5V, for example) and $V_2$ is a DC voltage (5V to turn on LED and 0V to turn off LED) or a pulse train with 5V levels turning on the LED and 0V turning off the LED.

**FIG. 31** shows an instrumental system to produce DNA sample microarrays. Identified are **601,** a pico-pump controller; **602,** the pico-pump; **603,** dispensing tip; **604,** sample; **605,** substrate; **606,** mesh frit; **607,** vacuum inlet; **608,** dual-axis translation stage; and **609,** computer controller.

**FIG. 32** shows an illustrative embodiment of the Biochip of the present invention.

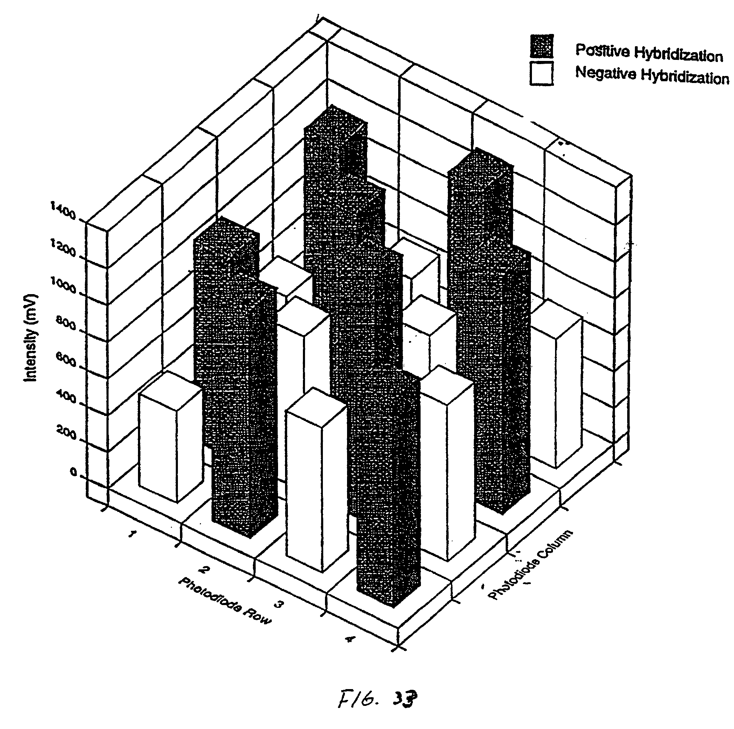

**FIG. 33** shows the results of hybridization occurring between complementary DNA sequences and the detection of fluorescence signals using a nucleic acid biochip. Fluorescence signals higher than the background were detected on the biochip channels where hybridization of labeled DNA HIV 1 gene probes with complementary bound-DNA fragments had occurred. The $4 \times 4$ array signals of the 3-dimensional plot demonstrated positive hybridization with the HIV 1 probes used. A reference system of negative blank samples (consisting of DNA probes which did not have the sequence of the HIV I gene) showed only weak background fluorescent signals.

## 4.0 DESCRIPTION OF ILLUSTRATIVE EMBODIMENTS

### 4.1 DEFINITIONS

An integrated circuit (IC) is a circuit comprised of elements such as transistors, resistors and capacitors fabricated in a single piece of semiconducting material, usually silicon or gallium arsenide. The "integrated circuit" may be utilized in highly integrated structures including, for example:

1) multichip modules where several IC's and other circuit elements including molecular target probes may be combined compactly on a polymer, quartz, glass, sliver, ceramic or other substrates. In some cases, one IC may be the substrate with other components, such as photodiodes or LEDs mounted on it;
2) Hybrid microcircuits where one or more IC's and other circuit elements are mounted on or several substrate(s); and,
3) Other compact electromechanical arrangements of a circuit comprising primarily one but possibly more IC's and other electronic components and microelectromechanised systems (MEMs).

### 4.2 SOME ADVANTAGES OF THE INVENTION

The DNA Biochip system offers a unique combination of performance capabilities and analytical features of merit not available in any other DNA analysis system currently available. With its multichannel capability, the DNA Biochip technology is the only current system that allows simultaneous detection of multiple DNA targets simultaneously. The present biochip also offers several advantages in size, performance, fabrication, analysis and production cost. The small sizes of the probes (microliter to nanoliter) minimize sample requirement and reduce reagent and waste requirement. Highly integrated systems lead to a reduction in noise and an increase in signal due to the improved efficiency of sample collection and the reduction of interfaces. The capability of large-scale production using low-cost IC technology is an important advantage. The assembly process of various components is made simple by integration of several elements on a single chip. For medical applications, this cost advantage will allow the development of extremely low cost, disposable biochips that can be used for in-home medical diagnostics of diseases without the need of sending samples to a laboratory for analysis.

### 4.3 DESIGN AND OPERATING PRINCIPLE OF THE DNA BIOCHIP

### 4.3. 1 INTEGRATED BIOCHIPS

Two fundamental operating principles of a biosensor are: (1) 'biological recognition'; and (2) 'sensing'. The basic principle of a biosensor is to detect this molecular recognition and to transform it into another type of signal using a transducer. There are different types of transducers which may produce either an optical signal (*i.e.* optical biosensors) or an electrochemical signal (*i.e.* electrochemical biosensors), or a mass-based signal (*e.g.*, microbalances, surface-acoustic wave devices, micro-cantilevers). There are also different types of bioreceptors which may consist of an enzyme, an antibody, a nucleic acid fragment, a chemoreceptor, a tissue, an organelle or a microorganism. Sometimes a synthetic molecule, often called biomimetic receptor (*e.g.*, synthetic antibody, molecular imprint) can be used to mimic the properties of biological receptors.

The inventors have developed an integrated DNA biochip which uses nucleic acid probes and detection system into a self-contained microdevice. The DNA biochip involves the combination of integrated circuit elements, electro-optics excitation/detection system, and DNA-based bioreceptor probes into a self-contained and integrated microdevice. A basic DNA biochip includes: (1) excitation light source with related optics; (2) a bioprobe; (3) a sampling platform and delivery system; (4) an optical detector with associated optics and dispersive device; and (5) a signal amplification/ treatment system.

Construction of a DNA biochip involves integration of several basic elements of very different natures. The basic steps include: (a) selection or development of the bioreceptor; (b) selection of the excitation source; (c) selection or development of the transducer; and (d) integration of the excitation source-bioreceptor-transducer system.

The development of the DNA biochip comprises three major elements. The first element involves the development of a bioreceptor probe system: a microarray of DNA probes on a multiarray sampling platform. The second element is focused on the development of non-radioactive methods for optical detection: the fluorescence technique. The third element involves the development of an integrated electro-optic IC system on a single chip for biosensing: photodiode-amplifier microchip using the CMOS technology.

## 4.3.2 OPERATING PRINCIPLE OF POLYNUCLEOTIDE PROBES:

### MOLECULAR HYBRIDIZATION

Whereas the use of immunological probes is based on antibody-antigen reactions, the operation of gene probes is based on the hybridization process, which is one of the most powerful and useful techniques in molecular genetics. Hybridization involves the joining of a single strand of nucleic acid with a complementary probe sequence. Hybridization of a nucleic acid probe to DNA biotargets (*e.g.*, gene sequences, bacteria, viral DNA, *p53* cancer gene mutation *etc.*) offers a very high degree of accuracy for identifying DNA sequences complementary to that of the probe. Nucleic acids strands tend to be paired to their sequence complements (*i.e.* adenine-thymine, guanine-cytosine pairing) in the corresponding double-stranded structure. A single-stranded DNA molecule will seek out its complement in a complex mixture of DNA containing large numbers of other nucleic acid molecules. Various types of gene probes have been developed that are labeled with fluorescent (Isola *et at.,* 1996; Vo-Dinh *et al.,* 1998; Vo-Dinh *et al.,* 1998) or Raman labels (Vo-Dinh *et al.,* 1998; Isola *et al.,* 1998).

## 4.3.3 MICROSAMPLING PLATFORM

DNA probes can be directly or indirectly immobilized onto the biochip transducer sensing element to ensure optimal contact and maximum detection. When immobilized onto a substrate, the gene probes can be reused repeatedly. In one embodiment, hybridization is performed on an immobilized target or a probe molecule attached on a solid surface. DNA can be bound to different types of support materials using several methods. The method commonly used for binding DNA to glass involves silanization of the glass surface followed by activation with carbodiimide or glutaraldehyde.

Another embodiment involves immobilizing the gene probes onto a membrane platform which is placed on the transducer detection surface. In this manner, there is no need to bind the bioreceptor onto the biochip transducer, which may facilitate easier large-scale production.

In still another embodiment, 5'-terminal protecting groups are selectively removed from growing polynucleotide chains in pre-defined regions of a support (such as glass) by controlled exposure to light through a photolithographic mask (Fodor *et al.*, 1991; McGall *et al.*, 1997). This method may be used to fabricate polynucleotide probe arrays with densities as high as about $10^6$ unique probe sequences per $cm^2$.

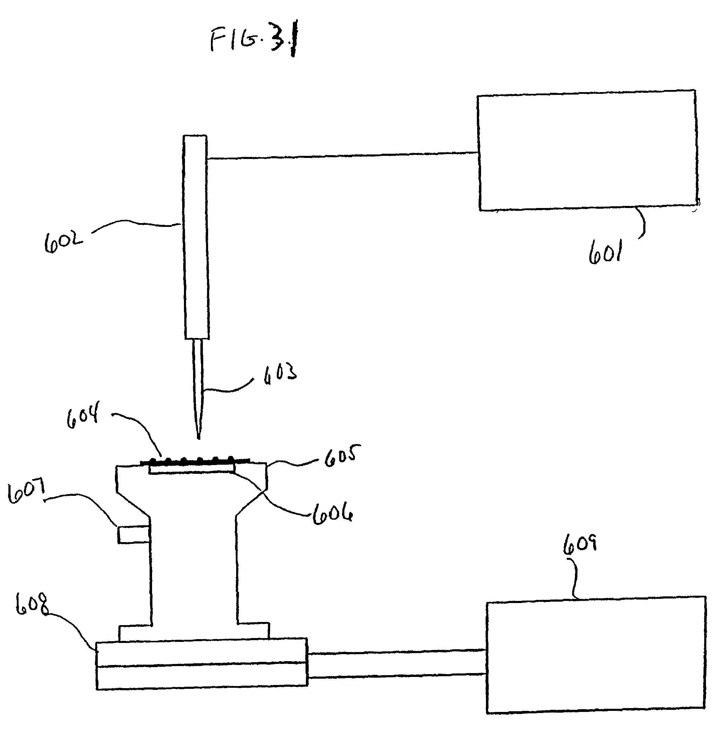

Plastic plates (often used in multi-well format) suitable for direct adsorption of DNA can be used to develop sampling platforms for the biochip. Chemically activated plates suitable to bind DNA by covalent linkage are also commercially available for use (CoStar, Cambridge, MA). Multiarrays of sample spots are produced, and liquid solutions of DNA are dispensed onto the sampling platform using a pneumatic Picopump (FIG. 31) (World Precision Instruments, Sarasota, FL). The Picopump is capable of producing regular microspots with diameter size range of 500-800 µm, which can be selected to match the size of the biochip detector elements. The DNA sample is loaded into a small diameter glass capillary, which is held a few millimeters above the sampling platform. The membrane is held in place on a vacuum flask fitted with a metal mesh frit. The vacuum procedure served two important purposes. First the vacuum applied to the membrane improves the reproducibility of the spotting by maintaining the membrane flat against the metal mesh surface. The vacuum procedure also has a shortening effect on the sample drying process, which decreased the sample spot size by preventing the spread of sample though the sampling platform. The biochip forma can be designed to be compatible to polymerase chain reaction (PCR™), which is an important technique allowing replication of defined DNA sequences, thereby amplifying the detection of these sequences.

## 4.4 BIOSENSOR PROBES

The development of biosensor technologies for detection of trace quantities of biological species in complex systems is important for many biomedical and environmental applications. Spectroscopic chemical sensors and biosensors have been developed using laser induced fluorescence, room temperature phosphorescence, surface enhanced Raman spectroscopy, antibody based immunofluorescence and gene probe Raman sensiing methods, including gene probes having surface-enhanced Raman scattering labels to enhance the selectivity and sensitivity of chemical sensors and biosensors (Vo-Dinh *et al.*, 1994).

The present invention uses spectroscopic techniques such as luminescence with visible and NIR labels is a useful detection scheme for gene biosensors without having the limitation of radioactive methods.

Non-radioactive probes, particularly gene probes, are desirable because of their selectivity in addition to avoiding the hazards involved with radioactive materials. Recognition and detection of biological species is based on the principle that cell specific nucleic acid sequences can be specifically recognized and can be combined with a receptor that specifically binds with that species. Such receptors include, for example, antibodies, enzymes, cells, bacterial probes, complementary nucleic acids, or nucleic acids that selectively hybridize with a cell-specific nucleic acid sequence. Receptors may be found and employed in the form of organelles, tissue components, chemoreceptors or even whole cells or microorganisms. Other types of receptors may include biomimetic materials such as cyclodextrins, molecular imprint materials, *etc.*

Gene probes operate on a hybridization process. Hybridization involves joining of a single strand of nucleic acid with a complementary probe sequence. Hybridization of a nucleic acid probe to a biotarget such as bacterial or viral DNA or RNA or selected gene segments, offers a high degree of accuracy for identifying nucleic acid sequences complementary to the probe. Nucleic acid strands tend to be paired with complementary strands, such as is typically found in double-stranded DNA structures. Therefore, a single-stranded DNA (or RNA) will seek out its complement in a complex mixture of DNA containing large numbers of other nucleic acid molecules. Nucleic acid probe or gene probe detection methods are specific to DNA sequences. Factors affecting the hybridization or reassociation of two complementary DNA strands include temperature, contact time, salt concentration, the degree of mismatch between the base pairs, and the length and concentration of the target and probe sequences.

## 4.5 PEPTIDE NUCLEIC ACID COMPOSITIONS

In certain embodiments, the inventors contemplate the use of peptide nucleic acids (PNAs) in the practice of the methods of the invention. PNA is a DNA mimic in which the nucleobases are attached to a pseudopeptide backbone (Good and Nielsen, 1997). PNAs are able to be utilized in a number methods that traditionally have used RNAs or DNAs. Often PNA sequences perform better in techniques than the corresponding RNA or DNA sequences and have utilities that are not inherent to RNA or DNA. An excellent review of PNA including methods of making, characteristics of, and methods of using, is provided by Corey (1997) and is incorporated herein by reference.

## 4.5.1 METHODS OF MAKING PNAS

According to Corey, PNAs have 2-aminoethyl-glycine linkages replacing the normal phosphodiester backbone of DNA (Nielsen *et al.*, 1991; Hanvey *et al.*, 1992; Hyrup and Nielsen, 1996; Neilsen, 1996). This chemistry has three important consequences: firstly, in contrast to DNA or phosphorothioate oligonucleotides, PNAs are neutral molecules; secondly, PNAs are achiral, which avoids the need to develop a stereoselective synthesis; and thirdly, PNA synthesis uses standard Boc (Dueholm *et al.,* 1994) or Fmoc (Thomson *et al.,* 1995) protocols for solid-phase peptide synthesis, although other methods, including a modified Merrifield method, have been used (Christensen *et al.,* 1995).

PNA monomers or ready-made oligomers are commercially available from PerSeptive Biosystems (Framingham, MA, USA). PNA syntheses by either Boc or Fmoc protocols are straightforward using manual or automated protocols (Norton *et al.,* 1995). The manual protocol lends itself to the production of chemically modified PNAs or the simultaneous synthesis of families of closely related PNAs.

As with peptide synthesis, the success of a particular PNA synthesis will depend on the properties of the chosen sequence. For example, while in theory PNAs can incorporate any combination of nucleotide bases, the presence of adjacent purines can lead to deletions of one or more residues in the product. In expectation of this difficulty, it is suggested that, in producing PNAs with adjacent purines, one should repeat the coupling of residues likely to be added inefficiently. This should be followed by the purification of PNAs by reverse-phase high-pressure liquid chromatography (Norton *et al.*, 1995) providing yields and purity of product similar to those observed during the synthesis of peptides.

Further discussed by Corey are desired modifications of PNAs for given applications. Modifications can be accomplished by coupling amino acids during solid-phase synthesis or by attaching compounds that contain a carboxylic acid group to the exposed N-terminal amine. Alternatively, PNAs can be modified after synthesis by coupling to an introduced lysine or cysteine. The ease with which PNAs can be modified facilitates optimization for better solubility or for specific functional requirements. Once synthesized, the identity of PNAs and their derivatives can be confirmed by mass spectrometry. Several studies have made and utilized modifications of PNAs (Norton *et al.*, 1995; Haaima *et al.*, 1996; Stetsenko *et al.*, 1996; Petersen *et al.*, 1995; Ulmann *et al.*, 1996; Koch *et al.*, 1995; Orum *et al.*, 1995; Footer *et al.*, 1996; Griffith *et al.*, 1995; Kremsky *et al.*, 1996; Pardridge *et al.*, 1995; Boffa *et al.*, 1995; Landsdorp *et al.*, 1996; Gambacorti-Passerini *et al.*, 1996; Armitage *et al.*, 1997; Seeger *et al.*, 1997; Rusckowski *et al.*, 1997). United States Patent No. 5,700,922 discusses PNA-DNA-PNA chimeric molecules and their uses in diagnostics, modulating protein in organisms, and treatment of conditions susceptible to therapeutics.

## 4.5.2 PHYSICAL PROPERTIES OF PNAs

In contrast to DNA and RNA, which contain negatively charged linkages, the PNA backbone is neutral. In spite of this dramatic alteration, PNAs recognize complementary DNA and RNA by Watson-Crick pairing (Egholm *et al.*, 1993), validating the initial modeling by Nielsen *et al.*, (1991). PNAs lack 3' to 5' polarity and can bind in either parallel or antiparallel fashion, with the antiparallel mode being preferred (Egholm *et al.*, 1993).

Hybridization of DNA oligonucleotides to DNA and RNA is destabilized by electrostatic repulsion between the negatively charged phosphate backbones of the complementary strands. By contrast, the absence of charge repulsion in PNA-DNA or PNA-RNA duplexes increases the melting temperature ($T_m$) and reduces the dependence of $T_m$ on the concentration of mono- or divalent cations (Nielsen *et al.*, 1991). The enhanced rate and affinity of hybridization are significant because they are responsible for the surprising ability of PNAs to perform strand invasion of complementary sequences within relaxed double-stranded DNA. In addition, the efficient hybridization at inverted repeats suggests that PNAs can recognize secondary structure effectively within double-stranded DNA. Enhanced recognition also occurs with PNAs immobilized on surfaces, and Wang *et al.,* have shown that support-bound PNAs can be used to detect hybridization events (Wang *et al.,* 1996).

One might expect that tight binding of PNAs to complementary sequences would also increase binding to similar (but not identical) sequences, reducing the sequence specificity of PNA recognition. As with DNA hybridization, however, selective recognition can be achieved by balancing oligomer length and incubation temperature. Moreover, selective hybridization of PNAs is encouraged by PNA-DNA hybridization being less tolerant of base mismatches than DNA-DNA hybridization. For example, a single mismatch within a 16 bp PNA-DNA duplex can reduce the $T_m$ by up to 15°C (Egholm *et al.,* 1993). This high level of discrimination has allowed the development of several PNA-based strategies for the analysis of point mutations (Wang *et al.*, 1996; Carlsson *et al.*, 1996; Thiede *et al.*, 1996; Webb and Hurskainen, 1996; Perry-O'Keefe *et al.*, 1996).

High-affinity binding provides clear advantages for molecular recognition and the development of new applications for PNAs. For example, 11-13 nucleotide PNAs inhibit the activity of telomerase, a ribonucleo-protein that extends telomere ends using an essential RNA template, while the analogous DNA oligomers do not (Norton *et al.*, 1996).

Neutral PNAs are more hydrophobic than analogous DNA oligomers, and this can lead to difficulty solubilizing them at neutral pH, especially if the PNAs have a high purine content or if they have the potential to form secondary structures. Their solubility can be enhanced by attaching one or more positive charges to the PNA termini (Nielsen *et al.*, 1991).

## 4.5.3 APPLICATIONS OF PNAS

Findings by Allfrey and colleagues suggest that strand invasion will occur spontaneously at sequences within chromosomal DNA (Boffa *et al.*, 1995; Boffa *et al.*, 1996). These studies targeted PNAs to triplet repeats of the nucleotides CAG and used this recognition to purify transcriptionally active DNA (Boffa *et al.*, 1995) and to inhibit transcription (Boffa *et al.*, 1996). This result suggests that if PNAs can be delivered within cells then they will have the potential to be general sequence-specific regulators of gene expression. Studies and reviews concerning the use of PNAs as antisense and anti-gene agents include Nielsen *et al.*, (1993b), Hanvey *et al.*, (1992), and Good and Nielsen (1997). Koppelhus *et al.*, (1997) have used PNAs to inhibit HIV-1 inverse transcription, showing that PNAs may be used for antiviral therapies.

Methods of characterizing the antisense binding properties of PNAs are discussed in Rose (1993) and Jensen *et al.*, (1997). Rose uses capillary gel electrophoresis to determine binding of PNAs to their complementary oligonucleotide, measuring the relative binding kinetics and stoichiometry. Similar types of measurements were made by Jensen *et al.*, using BIAcore™ technology.

Other applications of PNAs include use in DNA strand invasion (Nielsen *et al.*, 1991), antisense inhibition (Hanvey *et al.*, 1992), mutational analysis (Orum *et al.*, 1993), enhancers of transcription (Mollegaard *et al.*, 1994), nucleic acid purification (Orum *et al.*, 1995), isolation of transcriptionally active genes (Boffa *et al.*, 1995), blocking of transcription factor binding (Vickers *et al.*, 1995), genome cleavage (Veselkov *et al.*, 1996), biosensors (Wang *et al.*, 1996), *in situ* hybridization (Thisted *et al.*, 1996), and in a alternative to Southern blotting (Perry-O'Keefe, 1996).

## 4.6 ANTIBODY COMPOSITIONS AND METHODS OF MAKING

In particular embodiments, the inventors contemplate the use of antibodies, either monoclonal or polyclonal which bind to one or more of the polypeptides disclosed herein. Means for preparing and characterizing antibodies are well known in the art (See, *e.g.*, Harlow and Lane, 1988; The methods for generating monoclonal antibodies (mAbs) generally begin along the same lines as those for preparing polyclonal antibodies. Briefly, a polyclonal antibody is prepared by immunizing an animal with an immunogenic composition in accordance with the present invention and collecting antisera from that immunized, animal. A wide range of animal species can be used for the production of antisera. Typically the animal used for production of anti-antisera is a rabbit, a mouse, a rat, a hamster, a guinea pig or a goat. Because of the relatively large blood volume of rabbits, a rabbit is a preferred choice for production of polyclonal antibodies.

As is well known in the art, a given composition may *vary* in its immunogenicity. It is often necessary therefore to boost the host immune system, as may be achieved by coupling a peptide or polypeptide immunogen to a carrier. Exemplary and preferred carriers are keyhole limpet hemocyanin (KLH) and bovine serum albumin (BSA). Other albumins such as ovalbumin, mouse serum albumin or rabbit serum albumin can also be used as carriers. Means for conjugating a polypeptide to a carrier protein are well known in the art and include glutaraldehyde, *m*-maleimidobencoyl-*N*-hydroxysuccinimide ester, carbodiimide and bis-biazotized benzidine.

As is also well known in the art, the immunogenicity of a particular immunogen composition can be enhanced by the use of non-specific stimulators of the immune response, known as adjuvants. Exemplary and preferred adjuvants include complete Freund's adjuvant (a non-specific stimulator of the immune response containing killed *Mycobacterium tuberculosis),* incomplete Freund's adjuvants and aluminum hydroxide adjuvant.

The amount of immunogen composition used in the production of polyclonal antibodies varies upon the nature of the immunogen as well as the animal used for immunization. A variety of routes can be used to administer the immunogen (subcutaneous, intramuscular, intradermal, intravenous and intraperitoneal). The production of polyclonal antibodies may be monitored by sampling blood of the immunized animal at various points following immunization. A second, booster, injection may also be given. The process of boosting and tittering is repeated until a suitable titer is achieved. When a desired level of immunogenicity is obtained, the immunized animal can be bled and the serum isolated and stored, and/or the animal can be used to generate mAbs.

mAbs may be readily prepared through use of well-known techniques, such as those exemplified in US-A-4,196,265. Typically, this technique involves immunizing a suitable animal with a selected immunogen composition, *e. g.*, a purified or partially purified polypeptide or peptide. The immunizing composition is administered in a manner effective to stimulate antibody producing cells. Rodents such as mice and rats are preferred animals, however, the use of rabbit, sheep, or frog cells is also possible. The use of rats may provide certain advantages (Goding, 1986, pp. 60-61), but mice are preferred, with the BALB/c mouse being most preferred as this is most routinely used and generally gives a higher percentage of stable fusions.

Following immunization, somatic cells with the potential for producing antibodies, specifically B lymphocytes (B cells), are selected for use in the mAb generating protocol. These cells may be obtained from biopsied spleens, tonsils or lymph nodes, or from a peripheral blood sample. Spleen cells and peripheral blood cells are preferred, the former because they are a rich source of antibody-producing cells that are in the dividing plasmablast stage, and the latter because peripheral blood is easily accessible. Often, a panel of animals will have been immunized and the spleen of animal with the highest antibody titer will be removed and the spleen lymphocytes obtained by homogenizing the spleen with a syringe. Typically, a spleen from an immunized mouse contains approximately $5 \times 10^7$ to $2 \times 10^8$ lymphocytes.

The antibody-producing B lymphocytes from the immunized animal are then fused with cells of an immortal myeloma cell, generally one of the same species as the animal that was immunized. Myeloma cell lines suited for use in hybridoma-producing fusion procedures preferably are non-antibody-producing, have high fusion efficiency, and enzyme deficiencies that render them incapable of growing in certain selective media which support the growth of only the desired fused cells (hybridomas).

Any one of a number of myeloma cells may be used, as are known to those of skill in the art (Goding, pp.

65-66, 1986; Campbell, pp. 75-83, 1984). For example, where the immunized animal is a mouse, one may use P3-X63/Ag8, X63-Ag8.653, NS1/1.Ag 4 1, Sp210-Ag14, FO, NSO/U, MPC-11, MPCII-X45-GTG 1.7 and S194/5XX0 Bul; for rats, one may use R210.RCY3, Y3-Ag 1.2.3, IR983F and 4B210; and U-266, GM1500-GRG2, LICR-LON-HMy2 and UC729-6 are all useful in connection with human cell fusions.

One preferred murine myeloma cell is the NS-1 myeloma cell line (also termed P3-NS-1-Ag4-1), which is readily available from the NIGMS Human Genetic Mutant Cell Repository by requesting cell line repository number GM3573. Another mouse myeloma cell line that may be used is the 8-azaguanine-resistant mouse murine myeloma SP2/0 non-producer cell line.

Methods for generating hybrids of antibody-producing spleen or lymph node cells and myeloma cells usually comprise mixing somatic cells with myeloma cells in a 2:1 ratio, though the ratio may vary from about 20:1 to about 1:1, respectively, in the presence of an agent or agents (chemical or electrical) that promote the fusion of cell membranes. Fusion methods using Sendai virus have been described (Kohler and Milstein, 1975; 1976), and those using polyethylene glycol (PEG), such as 37% (vol./vol.) PEG, (Gefter *et al*., 1977). The use of electrically induced fusion methods is also appropriate (Coding, 1986, pp. 71-74).

Fusion procedures usually produce viable hybrids at low frequencies, about $1 \times 10^{-6}$ to $1 \times 10^{-8}$. However, this does not pose a problem, as the viable, fused hybrids are differentiated from the parental, unfused cells (particularly the unfused myeloma cells that would normally continue to divide indefinitely) by culturing in a selective medium. The selective medium is generally one that contains an agent that blocks the *de novo* synthesis of nucleotides in the tissue culture media. Exemplary and preferred agents are aminopterin, methotrexate, and azaserine. Aminopterin and methotrexate block *de novo* synthesis of both purines and pyrimidines, whereas azaserine blocks only purine synthesis. Where aminopterin or methotrexate is used, the media is supplemented with hypoxanthine and thymidine as a source of nucleotides (HAT medium). Where azaserine is used, the media is supplemented with hypoxanthine.

The preferred selection medium is HAT. Only cells capable of operating nucleotide salvage pathways are able to survive in HAT medium. The myeloma cells are defective in key enzymes of the salvage pathway, *e.g.*, hypoxanthine phosphoribosyl transferase (HPRT), and they cannot survive. The B-cells can operate this pathway, but they have a limited life span in culture and generally die within about two weeks. Therefore, the only cells that can survive in the selective media are those hybrids formed from myeloma and B-cells.

This culturing provides a population of hybridomas from which specific hybridomas are selected. Typically, selection of hybridomas is performed by culturing the cells by single-clone dilution in microtiter plates, followed by testing the individual clonal supematants (after about two to three weeks) for the desired reactivity. The assay should be sensitive, simple and rapid, such as radioimmunoassays, enzyme immunoassays, cytotoxicity assays, plaque assays, dot immunobinding assays, and the like.

The selected hybridomas would then be serially diluted and cloned into individual antibody-producing cell lines, which clones can then be propagated indefinitely to provide mAbs. The cell lines may be exploited for mAb production in two basic ways. A sample of the hybridoma can be injected (often into the peritoneal cavity) into a histocompatible animal of the type that was used to provide the somatic and myeloma cells for the original fusion. The injected animal develops tumors secreting the specific monoclonal antibody produced by the fused cell hybrid. The body fluids of the animal, such as serum or ascites fluid, can then be tapped to provide mAbs in high concentration. The individual cell lines could also be cultured *in vitro*, where the mAbs are naturally secreted into the culture medium from which they can be readily obtained in high concentrations. mAbs produced by either means may be further purified, if desired, using filtration, centrifugation and various chromatographic methods such as HPLC or affinity chromatography.

### 4.7 ELISAS

The techniques of ELISAs may be used in conjunction with the invention, when the product of interest to be detected is a polypeptide, and the substrate on the chip is an antibody. Alternatively, ELISA techniques may be utilized when the substrate on the chip is a polypeptide and the product of interest to be detected is an antibody. In either embodiment, the antibody or the polypeptide may be fluorescently labeled for detection by the biochip.

In an ELISA assay, proteins or peptides incorporating antigenic sequences are immobilized onto a selected surface, preferably a surface exhibiting a protein affinity such as the wells of a polystyrene microtiter plate. After washing to remove incompletely adsorbed material, it is desirable to bind or coat the assay plate wells with a nonspecific protein that is known to be antigenically neutral with regard to the test antisera such as bovine serum albumin (BSA), casein or solutions of milk powder. This allows for blocking of nonspecific adsorption sites on the immobilizing surface and thus reduces the background caused by nonspecific binding of antisera onto the surface.

After binding of antigenic material to the well, coating with a non-reactive material to reduce background, and washing to remove unbound material, the immobilizing surface is contacted with the antisera or clinical or biological extract to be tested in a manner conducive to immune complex (antigen/antibody) formation. Such conditions preferably include diluting the antisera with diluents such as BSA, bovine gamma globulin (BGG) and phosphate buffered saline (PBS)/Tween®. These added agents also tend to assist in the reduction of nonspecific background. The layered antisera is then allowed to incubate for from about 2 to about 4 hr, at temperatures preferably on the order of about 25°C to about 27°C. Following incubation, the antisera-contacted surface is washed so as to remove non-immunocomplexed material. A preferred washing procedure includes washing with a solution such as PBS/Tween®, or borate buffer.

Following formation of specific immunocomplexes between the test sample and the bound antigen, and subsequent washing, the occurrence and even amount of immunocomplex formation may be determined by subjecting same to a second antibody having specificity for the first. To provide a detecting means, the second antibody will preferably have an associated enzyme that will generate a color development upon incubating with an appropriate chromogenic substrate. Thus, for example, one will desire to contact and incubate the antisera-bound surface with a urease or peroxidase-conjugated anti-human IgG for a period of time and under conditions which favor the development of immunocomplex formation (*e.g.*, incubation for 2 hr at room temperature in a PBS-containing solution such as PBS Tween®).

After incubation with the second enzyme-tagged antibody, and subsequent to washing to remove unbound material, the amount of label is quantified by incubation with a chromogenic substrate such as urea and bromocresol purple or 2,2'-azino-di-(3-ethyl-benzthiazoline)-6-sulfonic acid (ABTS) and $H_2O_2$, in the case of peroxidase as the enzyme label. Quantitation is then achieved by measuring the degree of color generation, *e.g.*, using a visible spectra spectrophotometer.

## 4.8 METHODS FOR PREPARING MUTAGENIZED POLYNUCLEOTIDES

In certain circumstances, it may be desirable to modify or alter one or more nucleotides in one or more of the polynucleotide sequences disclosed herein for the purpose of altering or changing the insecticidal activity or insecticidal specificity of the encoded polypeptide. In general, the means and methods for mutagenizing a DNA segment are well-known to those of skill in the art. Modifications to such segments may be made by random, or site-specific mutagenesis procedures. The polynucleotides may be modified by the addition, deletion , or substitution of one or more nucleotides from the sequence encoding the insecticidally-active polypeptide.

Mutagenesis may be performed in accordance with any of the techniques known in the art such as and not limited to synthesizing an oligonucleotide having one or more mutations within the sequence of a particular region. In particular, site-specific mutagenesis is a technique useful in the preparation of mutants, through specific mutagenesis of the underlying DNA. The technique further provides a ready ability to prepare and test sequence variants, for example, incorporating one or more of the foregoing considerations, by introducing one or more nucleotide sequence changes into the DNA. Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Typically, a primer of about 17 to about 75 nucleotides or more in length is preferred, with about 10 to about 25 or more residues on both sides of the junction of the sequence being altered.

In general, the technique of site-specific mutagenesis is well known in the art, as exemplified by various publications. As will be appreciated, the technique typically employs a phage vector which exists in both a single stranded and double stranded form. Typical vectors useful in site-directed mutagenesis include vectors such as the M13 phage. These phage are readily commercially available and their use is generally well known to those skilled in the art. Double stranded plasmids are also routinely employed in site directed mutagenesis which eliminates the step of transferring the gene of interest from a plasmid to a phage.

In general, site-directed mutagenesis in accordance herewith is performed by first obtaining a single-stranded vector or melting apart of two strands of a double stranded vector which includes within its sequence a DNA sequence which encodes the desired promoter region or peptide. An oligonucleotide primer bearing the desired mutated sequence is prepared, generally synthetically. This primer is then annealed with the single-stranded vector, and subjected to DNA polymerizing enzymes such as *E. coli* polymerase I Klenow fragment, in order to complete the synthesis of the mutation-bearing strand. Thus, a heteroduplex is formed wherein one strand encodes the original non-mutated sequence and the second strand bears the desired mutation. This heteroduplex vector is then used to transform or transfect appropriate cells, such as *E. coli* cells, and clones are selected which include recombinant vectors bearing the mutated sequence arrangement A genetic selection scheme was devised by Kunkel *et al.*, (1987) to enrich for clones incorporating the mutagenic oligonucleotide. Alternatively, the use of PCR™ with commercially available thermostable enzymes such as *Taq* polymerase may be used to incorporate a mutagenic oligonucleotide primer into an amplified DNA fragment that can then be cloned into an appropriate cloning or expression vector. The PCR™-mediated mutagenesis procedures of Tomic *et al.*, (1990) and Upender *et al.*, (1995) provide two examples of such protocols. A PCR™ employing a thermostable ligase in addition to a thermostable polymerase may also be used to incorporate a phosphorylated mutagenic oligonucleotide into an amplified DNA fragment that may then be cloned into an appropriate cloning or expression vector. The mutagenesis procedure described by Michael (1994) provides an example of one such protocol.

As used herein, the term "oligonucleotide directed mutagenesis procedure" refers to template-dependent processes and vector-mediated propagation which result in an increase in the concentration of a specific nucleic acid molecule relative to its initial concentration, or in an increase in the concentration of a detectable signal, such as amplification. As used herein, the term "oligonucleotide directed mutagenesis procedure" also is intended to refer to a process that involves the template-dependent extension of a primer molecule. The term template-dependent process refers to nucleic acid synthesis of an RNA or a DNA molecule wherein the sequence of the newly synthesized strand of nucleic acid is dictated by the well-known rules of complementary base pairing (Watson, 1987), Typically, vector mediated methodologies involve the introduction of the nucleic acid fragment into a DNA or RNA vector, the clonal amplification of the vector, and the recovery of the amplified nucleic acid fragment. Examples of such methodologies are provided by US-A-4,237,224.

A number of template dependent processes are available to amplify the target sequences of interest present in a sample. One of the best known amplification methods is the polymerase chain reaction (PCR™) which is described in detail in US-A-4,683,195, US-A-4,683,202 and US-A-4,800,159.

Briefly, in PCR™, two primer sequences are prepared which are complementary to regions on opposite complementary strands of the target sequence. An excess of deoxynucleoside triphosphates are added to a reaction mixture along with a DNA polymerase (*e.g.*, *Taq* polymerase). If the target sequence is present in a sample, the primers will bind to the target and the polymerase will cause the primers to be extended along the target sequence by adding on nucleotides. By raising *and* lowering the temperature of the reaction mixture, the extended primers will dissociate from the target to form reaction products, excess primers will bind to the target and to the reaction products and the process is repeated. Preferably a reverse transcriptase PCR™ amplification procedure may be performed in order to quantify the amount of mRNA amplified. Polymerase chain reaction methodologies are well known in the art.

Another method for amplification is the ligase chain reaction (referred to as LCR), disclosed in EP-A-0 320 308 In LCR, two complementary probe pairs are prepared, and in the presence of the target sequence, each pair will bind to opposite complementary strands of the target such that they abut. In the presence of a ligase, the two probe pairs will link to form a single unit By temperature cycling, as in PCR™, bound ligated units dissociate from the target and then serve as "target sequences" for ligation of excess probe pairs. US-A-4,883,750 describes an alternative method of amplification similar to LCR for binding probe pairs to a target sequence.

Qbeta Replicase, described in WO-A-87/06270, may also be used as still another amplification method in the present invention. In this method, a replicative sequence of RNA which has a region complementary to that of a target is added to a sample in the presence of an RNA polymerase The polymerase will copy the replicative sequence which can then be detected.

An isothermal amplification method, in which restriction endonucleases and ligases are used to achieve the amplification of target molecules that contain nucleotide 5'-[$\alpha$-thio]triphosphates in one strand of a restriction site (Walker *et al*., 1992), may also be useful in the amplification of nucleic acids in the present invention.

Strand Displacement Amplification (SDA) is another method of carrying out isothermal amplification of nucleic acids which involves multiple rounds of strand displacement and synthesis, *i.e.* nick translation. A similar method, called Repair Chain Reaction (RCR) is another method of amplification which may be useful in the present invention and is involves annealing several probes throughout a region targeted for amplification, followed by a repair reaction in which only two of the four bases are present The other two bases can be added as biotinylated derivatives for easy detection. A similar approach is used in SDA.

Still other amplification methods described in GB-A-2 202 328 and in WO-A-89/09284 may be used in accordance with the present invention. In the former application, "modified" primers are used in a PCR™ like, template and enzyme dependent synthesis. The primers may be modified by labeling with a capture moiety (*e.g.*, biotin) and/or a detector moiety (*e.g.,* enzyme). In the latter application, an excess of labeled probes are added to a sample. In the presence of the target sequence, the probe binds and is cleaved catalytically. After cleavage, the target sequence is released intact to be bound by excess probe. Cleavage of the labeled probe signals the presence of the target sequence.

Other nucleic acid amplification procedures include transcription-based amplification systems (TAS) (Kwoh *et al*., 1989; WO-A-88/10315), including nucleic acid sequence based amplification (NASBA) and 3SR. In NASBA, the nucleic acids can be prepared for amplification by standard phenol/chloroform extraction, heat denaturation of a sample, treatment with lysis buffer and minispin columns for isolation of DNA and RNA or guanidinium chloride extraction of RNA. These amplification techniques involve annealing a primer which has target-specific sequences. Following polymerization, DNA/RNA hybrids are digested with RNase H while double stranded DNA molecules are heat denatured again. In either case the single stranded DNA is made fully double stranded by addition of second target polypeptide-specific primer, followed by polymerization. The double stranded DNA molecules are then multiply transcribed by a polymerase such as T7 or SP6. In an isothermal cyclic reaction, the RNAs are reverse transcribed into double stranded DNA, and transcribed once against with a polymerase such as T7 or SP6. The resulting products, whether truncated or complete, indicate target-specific sequences.

EP-A-0 329 822 disclose a nucleic acid amplification process involving cyclically synthesizing single-stranded RNA ("ssRNA"), ssDNA, and double-stranded DNA (dsDNA), which may be used in accordance with the present invention. The ssRNA is a first template for a first primer oligonucleotide, which is elongated by reverse transcriptase (RNA-dependent DNA polymerase). The RNA is then removed from resulting DNA:RNA duplex by the action of ribonuclease H (RNase H, an RNase specific for RNA in a duplex with either DNA or RNA). The resultant ssDNA is a second template for a second primer, which also includes the sequences of an RNA polymerase promoter (exemplified by T7 RNA polymerase) 5' to its homology to its template. This primer is then extended by DNA polymerase (exemplified by the large "Klenow" fragment of *E, coli* DNA polymerase I), resulting as a double-stranded DNA ("dsDNA") molecule, having a sequence identical to that of the original RNA between the primers and having additionally, at one end, a promoter sequence. This promoter sequence can be used by the appropriate RNA polymerase to make many RNA copies of the DNA. These copies can then re-enter the cycle leading to very swift amplification. With proper choice of enzymes, this amplification can be done isothermally without addition of enzymes at each cycle. Because of the cyclical nature of this process, the starting sequence can be chosen to be in the form of either DNA or RNA.

WO-A-89/06700 disclose a nucleic acid sequence amplification scheme based on the hybridization of a promoter/primer sequence to a target single-stranded DNA ("ssDNA") followed by transcription of many RNA copies of the sequence. This scheme is not cyclic; *i.e.* new templates are not produced from the resultant RNA transcripts. Other amplification methods include "RACE" (Frohman, 1990), and "one-sided PCRTh™" (Ohara, *et al.* 1989) which are well-known to those of skill in the art.

Methods based on ligation of two (or more) oligonucleotides in the presence of nucleic acid having the sequence of the resulting "di-oligonucleotide", thereby amplifying the di-oligonucleotide (Wu and Dean, 1996), may also be used in the amplification of polynucleotide sequences of the present invention.

**4.9 RIBOZYMES**

Ribozymes are enzymatic RNA molecules which cleave particular mRNA species. In certain embodiments, the inventors contemplate the selection and utilization of ribozymes capable of cleaving RNA segments, and the resulting detection of such mRNAs or ribozymes utilizing the methods and biochips of the present invention.

Six basic varieties of naturally-occurring enzymatic RNAs are known presently. Each can catalyze the hydrolysis of RNA phosphodiester bonds *in trans* (and thus can cleave other RNA molecules) under physiological conditions. In general, enzymatic nucleic acids act by first binding to a target RNA. Such binding occurs through the target binding portion of a enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The enzymatic nature of a ribozyme is advantageous over many technologies, such as antisense technology (where a nucleic acid molecule simply binds to a nucleic acid target to block its translation) since the concentration of ribozyme necessary to affect a therapeutic treatment is lower than that of an antisense oligonucleotide. This advantage reflects the ability of the ribozyme to act enzymatically. Thus, a single ribozyme molecule is able to cleave many molecules of target RNA. In addition, the ribozyme is a highly specific inhibitor, with the specificity of inhibition depending not only on the base pairing mechanism of binding to the target RNA, but also on the mechanism of target RNA cleavage. Single mismatches, or base-substitutions, near the site of cleavage can completely eliminate catalytic activity of a ribozyme. Similar mismatches in antisense molecules do not prevent their action (Woolf *et al.*, 1992). Thus, the specificity of action of a ribozyme is greater than that of an antisense oligonucleotide binding the same RNA site.

The enzymatic nucleic acid molecule may be formed in a hammerhead, hairpin, a hepatitis δ virus, group I intron or RNaseP RNA (in association with an RNA guide sequence) or Neurospora VS RNA motif Examples of hammerhead motifs are described by Rossi *et al.* (1992); examples of hairpin motifs are described by Hampel *et al.* EP-A-0 360 257), Hampel and Tritz (1989), Hampel *et al.* (1990) and Cech *et al.* (US-A- 5,631,359; an example of the hepatitis δ virus motif is described by Perrotta and Been (1992); an example of the RNaseP motif is described by Guerrier-Takada *et al.* (1983); Neurospora VS RNA ribozyme motif is described by Collins (Saville and Collins, 1990; Saville and Collins, 1991; Collins and Olive, 1993); and an example of the Group I intron is described by Cech *et al.* (US-A-4,987,071). All that is important in an enzymatic nucleic acid molecule of this invention is that it has a specific substrate binding site which is complementary to one or more of the target gene RNA regions, and that it have nucleotide sequences within or surrounding that substrate binding site which impart an RNA cleaving activity to the molecule. Thus the ribozyme constructs need not be limited to specific motifs mentioned herein.

The invention provides a method for producing a class of enzymatic cleaving agents which exhibit a high degree of specificity for the RNA of a desired target. The enzymatic nucleic acid molecule is preferably targeted to a highly conserved sequence region of a target mRNA such that specific treatment of a disease or condition can be provided with either one or several enzymatic nucleic acids. Such enzymatic nucleic acid molecules can be delivered exogenously to specific cells as required. Alternatively, the ribozymes can be expressed from DNA or RNA vectors that are delivered to specific cells.

Small enzymatic nucleic acid motifs (*e.g.*, of the hammerhead or the hairpin structure) may be used for exogenous delivery. The simple structure of these molecules increases the ability of the enzymatic nucleic acid to invade targeted regions of the mRNA structure. Alternatively, catalytic RNA molecules can be expressed within cells from eukaryotic promoters (*e.g.*, Scanlon *et al.*, 1991; Kashani-Sabet *et al.*, 1992; Dropulic *et al.*, 1992; Weerasinghe *et al.*, 1991; Ojwang *et al.*, 1992; Chen *et al.*, 1992; Sarver *et al.*, 1990). Those skilled in the art realize that any ribozyme can be expressed in eukaryotic cells from the appropriate DNA vector. The activity of such ribozymes can be augmented by their release from the primary transcript by a second ribozyme (WO-A-93/23569), and (WO-A-94/02595), Ohkawa *et al.*, 1992; Taira *et al.*, 1991; Ventura *et al.*, 1993).

Ribozymes may be added directly, or can be complexed with cationic lipids, lipid complexes, packaged within liposomes, or otherwise delivered to target cells. The RNA or RNA complexes can be locally administered to relevant tissues *ex vivo* or *in vivo* through injection, aerosol inhalation, infusion pump or stent, with or without their incorporation in biopolymers.

Ribozymes may be designed as described in (WO-A-93/23569), or (WO-A94/02595) and synthesized to be tested *in vitro* and *in vivo,* as described. Such ribozymes can also be optimized for delivery. While specific examples are provided, those in the art will recognize that equivalent RNA targets in other species can be utilized when necessary.

Hammerhead or hairpin ribozymes may be individually analyzed by computer folding (Jaeger *et al.,* 1989) to assess whether the ribozyme sequences fold into the appropriate secondary structure. Those ribozymes with unfavorable intramolecular interactions between the binding arms and the catalytic core are eliminated from consideration. Varying binding arm lengths can be chosen to optimize activity. Generally, at least 5 bases on each arm are able to bind to, or otherwise interact with, the target RNA.

Ribozymes of the hammerhead or hairpin motif may be designed to anneal to various sites in the mRNA message, and can be chemically synthesized. The method of synthesis used follows the procedure for normal RNA synthesis as described in Usman and Cedergren (1992) and in Scaringe *et al.* (1990) and makes use of common nucleic acid protecting and coupling groups, such as dimethoxytrityl at the 5'-end, and phosphoramidites at the 3'-end. Average stepwise coupling yields are typically >98%. Hairpin ribozymes may be synthesized in two parts and annealed to reconstruct an active ribozyme (Chowrira and Burke, 1992). Ribozymes may be modified extensively to enhance stability by modification with nuclease resistant groups, for example, 2'-amino, 2'-C-allyl, 2'-flouro, 2'-o-methyl, 2'-H (for a review see Usman and Cedergren, 1992). Ribozymes may be purified by gel electrophoresis using general methods or by high pressure liquid chromatography and resuspended in water.

Ribozyme activity can be optimized by altering the length of the ribozyme binding arms, or chemically synthesizing ribozymes with modifications that prevent their degradation by serum ribonucleases (*see e.g.,* and WO-A-92-07065; Perreault *et al* 1990; Pieken *et al.*, 1991; Usman and Cedergren, 1992: WO-A-93/ 15 187 WO-A-91/03102 EP-A-0 519 463; US-A-5,334,711; and WO-A-94/13688, which describe various chemical modifications that can be made to the sugar moieties of enzymatic RNA molecules), modifications which enhance their efficacy in cells, and removal of stem II bases to shorten RNA synthesis times and reduce chemical requirements.

WO-A-94/02595 describes the general methods for delivery of enzymatic RNA molecules. Ribozymes may be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres. For some indications, ribozymes may be directly delivered *ex vivo* to cells or tissues with or without the aforementioned vehicles. Alternatively, the RNA/vehicle combination may be locally delivered by direct inhalation, by direct injection or by use of a catheter, infusion pump or stent. Other routes of delivery include, but are not limited to, intravascular, intramuscular, subcutaneous or joint injection, aerosol inhalation, oral (tablet or pill form), topical, systemic, ocular, intraperitoneal and/or intrathecal delivery. More detailed descriptions of ribozyme delivery and administration are provided in (WO-A-94/02595 ) and (WO-A-93/23569).

Another means of accumulating high concentrations of a ribozyme(s) within cells is to incorporate the ribozyme-encoding sequences into a DNA expression vector. Transcription of the ribozyme sequences are driven from a promoter for eukaryotic RNA polymerase I (pol I), RNA polymerase II (pol II), or RNA polymerase III (pol III). Transcripts from pol II or pol III promoters will be expressed at high levels in all cells; the levels of a given pol II promoter in a given cell type will depend on the nature of the gene regulatory sequences (enhancers, silencers, *etc.*) present nearby. Prokaryotic RNA polymerase promoters may also be used, providing that the prokaryotic RNA polymerase enzyme is expressed in the appropriate rells (Elroy-Stein and Moss, 1990; Gao and Huang, 1993; Lieber et *al.*, 1993;

Zhou *et al.*, 1990). Ribozymes expressed from such promoters can function in mammalian cells (*e.g.* Kashani-Saber *et al.*, 1992; Ojwang *et al.*, 1992; Chen *et al.*, 1992; Yu *et al.*, 1993; L'Huillier *et al.*, 1992; Lisziewicz *et al.*, 1993). Such transcription units can be incorporated into a variety of vectors for introduction into mammalian cells, including but not restricted to, plasmid DNA vectors, viral DNA vectors (such as adenovirus or adeno-associated vectors), or viral RNA vectors (such as retroviral, semliki forest virus, sindbis virus vectors).

Ribozymes of this invention may be used as diagnostic tools to examine genetic drift and mutations within cell lines or cell types. They can also be used to assess levels of the target RNA molecule. The close relationship between ribozyme activity and the structure of the target RNA allows the detection of mutations in any region of the molecule which alters the base-pairing and three-dimensional structure of the target RNA. By using multiple ribozymes described in this invention, one may map nucleotide changes which are important to RNA structure and function *in vitro*, as well as in cells and tissues. Cleavage of target RNAs with ribozymes may be used to inhibit gene expression and define the role (essentially) of specified gene products in particular cells or cell types.

A further method for identifying polynucleotides is through the use of oligonucleotide probes. These probes are nucleotide sequences having a detectable label. As is well known in the art, if the probe molecule and nucleic acid sample hybridize by forming a strong bond between the two molecules, it can be reasonably assumed that the probe and sample are essentially identical. The probe's detectable label provides a means for determining in a known manner whether hybridization has occurred. Such a probe analysis provides a rapid method for identifying related, homologous, or substantially homologous polynucleotides.

The nucleotide segments which are used as probes according to the invention can be synthesized by use of DNA synthesizers using standard procedures. In the use of the nucleotide segments as probes, the particular probe is labeled with any suitable label known to those skilled in the art, including radioactive and non-radioactive labels. Typical radioactive labels include $^{32}$P, $^{125}$I, $^{35}$S, or the like. A probe labeled with a radioactive isotope can be constructed from a nucleotide sequence complementary to the DNA sample by a conventional nick translation reaction, using a DNase and DNA polymerase. The probe and sample can then be combined in a hybridization buffer solution and held at an appropriate temperature until annealing occurs. Thereafter, the membrane is washed free of extraneous materials, leaving the sample and bound probe molecules typically detected and quantified by autoradiography and/or liquid scintillation counting.

Non-radioactive labels include, for example, ligands such as biotin or thyroxin, as well as enzymes such as hydrolases or peroxidases, or the various chemiluminescers such as luciferin, or fluorescent compounds like fluorescein and its derivatives. The probe may also be labeled at both ends with different types of labels for ease of separation, as, for example, by using an isotopic label at the end mentioned above and a biotin label at the other end.

Duplex formation and stability depend on substantial complementarity between the two strands of a hybrid, and, as noted above, a certain degree of mismatch can be tolerated. Therefore, the probes of the subject invention include mutations (both single and multiple), deletions, insertions of the described sequences, and combinations thereof, wherein said mutations, insertions and deletions permit formation of stable hybrids with the target polynucleotide of interest. Mutations, insertions, and deletions can be produced in a given polynucleotide sequence in many ways, by methods currently known to an ordinarily skilled artisan, and perhaps by other methods which may become known in the future.

The potential variations in the probes listed is due, in part, to the redundancy of the genetic code. Because of the redundancy of the genetic code, *i.e.,* more than one coding nucleotide triplet (codon) can be used for most of the amino acids used to make proteins. Therefore different nucleotide sequences can code for a particular amino acid. Thus, amino acid sequences can be prepared by equivalent nucleotide sequences encoding the same amino acid sequence of the protein or peptide. Also, inverse or complement sequences are an aspect of the subject invention and can be readily used by a person skilled in this art. In addition it has been shown that proteins of identified structure and function may be constructed by changing the amino acid sequence if such changes do not alter the protein secondary structure (Kaiser and Kezdy, 1984). Thus, the subject invention includes mutants of the amino acid sequence depicted herein which do not alter the protein secondary structure, or if the structure is altered, the biological activity is substantially retained.

## 4.10 IMMOBILIZATION TECHNIQUES

The DNA probes may be directly or indirectly immobilized onto a transducer detection surface to ensure optimal contact and maximum detection. When immobilized onto a substrate, the probes are stabilized and therefore may be used repeatedly. In general terms, hybridization is performed on an immobilized nucleic acid target or a probe molecule is attached to a solid surface such as nitrocellulose, nylon membrane or glass. Numerous other matrix materials may be used, including reinforced nitrocellulose membrane, activated quartz, activated glass, polyvinylidene difluoride (PVDF) membrane, polystyrene substrates, polyacrylamide-based substrate, other polymers such as poly (vinyl chloride), poly(methyl methacrylate), poly(dimethyl siloxane), photopolymers (which contain photoreactive species such as nitrenes, carbenes and ketyl radicals capable of forming covalent links with target molecules (Saiki *et al.*, 1994).

Binding of the bioprobe to a selected support may be accomplished by any of several means. For example, DNA is commonly bound to glass by first silanizing the glass surface, then activating with carbodimide or glutaraldehyde. Alternative procedures may use reagents such as 3-glycidoxypropyltrimethoxysilane (GOP) or aminopropyltrimethoxysilane (APTS) with DNA linked *via* amino linkers incorporated either at the 3' or 5' end of the molecule during DNA synthesis. DNA may be bound directly to membranes using ultraviolet radiation. With nitrocellous membranes, the DNA probes are spotted onto the membranes. A UV light source (Stratalinker, from Stratagene, La Jolla, Ca.) is used to irradiate DNA spots and induce cross-linking. An alternative method for cross-linking involves baking the spotted membranes at 80°C for two hours in vacuum.

Specific bioprobes may first be immobilized onto a membrane and then attached to a membrane in contact with a transducer detection surface. This method avoids binding the bioprobe onto the transducer and may be desirable for large-scale production. Membranes particularly suitable for this application include nitrocellulose membrane (*e.g.*, from BioRad, Hercules, CA) or polyvinylidene difluoride (PVDF) (BioRad, Hercules, CA) or nylon membrane (Zeta-Probe, BioRad) or polystyrene base substrates (DNA.BIND™ Costar, Cambridge, MA).

### 4.11 POLYNUCLEOTIDE HYBRIDIZATION PROBES AND PRIMERS

In addition to their use in directing homologous or substantially homologous nucleic acid sequences, the polynucleotides described herein also have a variety of other uses. For example, they have utility as probes or primers in nucleic acid hybridization embodiments. The invention provides a method for detecting a nucleic acid sequence encoding a particular polypeptide. The method generally involves obtaining sample nucleic acids suspected of encoding a polypeptide of interest; contacting the sample nucleic acids with an isolated nucleic acid segment substantially complementary to the sample nucleic acids, under conditions effective to allow hybridization of substantially complementary nucleic acids; and detecting the hybridized complementary nucleic acids thus formed.

Nucleic acid molecules having sequence regions consisting of contiguous nucleotide stretches of about 23 to about 50, or even up to and including sequences of about 100-200 nucleotides or so, identical or complementary to the target DNA sequence, are particularly contemplated as hybridization probes for use in, *e.g.*, Southern blotting. Intermediate-sized fragments will also generally find use in hybridization embodiments, wherein the length of the contiguous complementary region may be varied, such as between about 25-30, or between about 30 and about 40 or so nucleotides, but larger contiguous complementarity stretches may be used, such as those from about 200 to about 300, or from about 300 to about 400 or 500 or so nucleotides in length, according to the length complementary sequences one wishes to detect. It is even possible that longer contigous sequence regions may be utilized including those sequences comprising at least about 600 700, 800, 900, 1000, 1100, 1200, 1300, 1400, 1500, or more contiguous nucleotides.

Of course, fragments may also be obtained by other techniques such as, *e.g.*, by mechanical shearing or by restriction enzyme digestion. Small nucleic acid segments or fragments may be readily prepared by, for example, directly synthesizing the fragment by chemical means, as is commonly practiced using an automated oligonucleotide synthesizer. Also, fragments may be obtained by application of nucleic acid reproduction technology, such as the PCR™ technology of US-A-4,683,195 and US-A-4,683,202by introducing selected sequences into recombinant vectors for recombinant production, and by other recombinant DNA techniques generally known to those of skill in the art of molecular biology.

Accordingly, the nucleotide sequences of the invention may be used for their ability to selectively form duplex molecules with complementary stretches of DNA fragments. Depending on the application envisioned, one will desire to employ varying conditions of hybridization to achieve varying degrees of selectivity of probe towards target sequence. For applications requiring high selectivity, one will typically desire to employ relatively stringent conditions to form the hybrids. "High stringency" hybridization conditions, *e.g.*, typically employ relatively low salt and/or high temperature conditions, such as provided by about 0.02 M to about 0.15 M NaCl at temperatures of about 50°C to about 70°C. Such selective conditions tolerate little, if any, mismatch between the probe and the template or target strand, and would be particularly suitable for isolating particular DNA segments. Detection of DNA segments via hybridization is well-known to those of skill in the art, and the teachings of US-A-4,965,188 and US-A-5,176,995 are exemplary of the methods of hybridization analyses. Teachings such as those found in the texts of Maloy *et al.*, 1990; Maloy 1994; Segal, 1976; Prokop, 1991; and Kuby, 1994, are particularly relevant.

Of course, for some applications, for example, where one desires to prepare mutants employing a mutant primer strand hybridized to an underlying template or where one seeks to isolate particular target polynucleotide sequences from related species, functional equivalents, or the like, less stringent hybridization conditions will typically be needed in order to allow formation of the heteroduplex. In these circumstances, one may desire to employ "low stringency" or "reduced stringency" hybridization conditions such as those employing from about 0.15 M to about 0.9 M salt, at temperatures ranging from about 20°C to about 55°C. Cross-hybridizing species can thereby be readily identified as positively hybridizing signals with respect to control hybridizations. In any case, it is generally appreciated that conditions can be rendered more stringent by the addition of increasing amounts of formamide, which serves to destabilize the hybrid duplex in the same manner as increased temperature. Thus, hybridization conditions can be readily manipulated, and thus will generally be a method of choice depending on the desired results. Regardless of what particular combination of salts (such as NaCl or NaCitrate and the like), organic buffers (including *e.g.*, formamide and the like), and incubation or washing temperatures are employed, the skilled artisan will readily be able to employ hybridization conditions that are "high," "medium," or "low" stringency, and will be able to interpret the results from hybridization analyses using such conditions to determine the relative homology of a target nucleic acid sequence to that of the particular polynucleotide sequence employed.

In certain embodiments, it will be advantageous to employ nucleic acid sequences of the present invention in combination with an appropriate means, such as a label, for determining hybridization. A wide variety of appropriate indicator means are known in the art, including fluorescent, radioactive, enzymatic or other ligands, such as avidin/ biotin, which are capable of giving a detectable signal. In preferred embodiments, one will likely desire to employ a fluorescent label or an enzyme tag, such as urease, alkaline phosphatase or peroxidase, instead of radioactive or other environmentally undesirable reagents. In the case of enzyme tags, colorimetric indicator substrates are known that can be employed to provide a means visible to the human eye or spectrophotometrically, to identify specific hybridization with complementary nucleic acid-containing samples.

In general, it is envisioned that the hybridization probes described herein will be useful both as reagents in solution hybridization as well as in embodiments employing a solid phase. In embodiments involving a solid phase, the test DNA (or RNA) is adsorbed or otherwise affixed to a selected matrix or surface. This fixed, single-stranded nucleic acid is then subjected to specific hybridization with selected probes under desired conditions. The selected conditions will depend on the particular circumstances based on the particular criteria required (depending, for example, on the G+C content, type of target nucleic acid, source of nucleic acid, size of hybridization probe, *etc.*). Following washing of the hybridized surface so as to remove nonspecifically bound probe molecules, specific hybridization is detected, or even quantitated, by means of the label.

## 4.12 DEVELOPMENT OF AN INTEGRATED CIRCUIT MICROCHIP (ICM)

### 4.12.1 ICM SYSTEM AND DESIGN

An important component of an ICM is the detection method and spectral range of sensing system. In the present work, several optical detection systems based on fluorescence of visible and near-infrared (NIR) dyes have been investigated for non-radioactive detection of tagged gene probes. Fluorescence detection means employing the integrated microchips of the present invention have been shown to selectively detect hybridized nucleic acids bound to the chip.. As shown previously, zeptomole ($10^{-21}$ mole) detection limit using fluorescence detection of dyes with laser excitation is possible (Vo-Dinh et *al.,* 1994), The miniaturization of optical biosensors is facilitated by the versatility of waveguide configurations. The figures show various configurations and uses of the DNA biochips.

### 4.12.2 ICM SYSTEM USING INTEGRATED PHOTOTRANSISTORS

The instrumental system discussed herein includes the design of integrated electroopuc sensing photodetectors for the biosensor microchips. Highly integrated biosensors are made possible partly through the capability of fabricating multiple optical sensing elements and microelectronics on a single integrated circuit (IC).

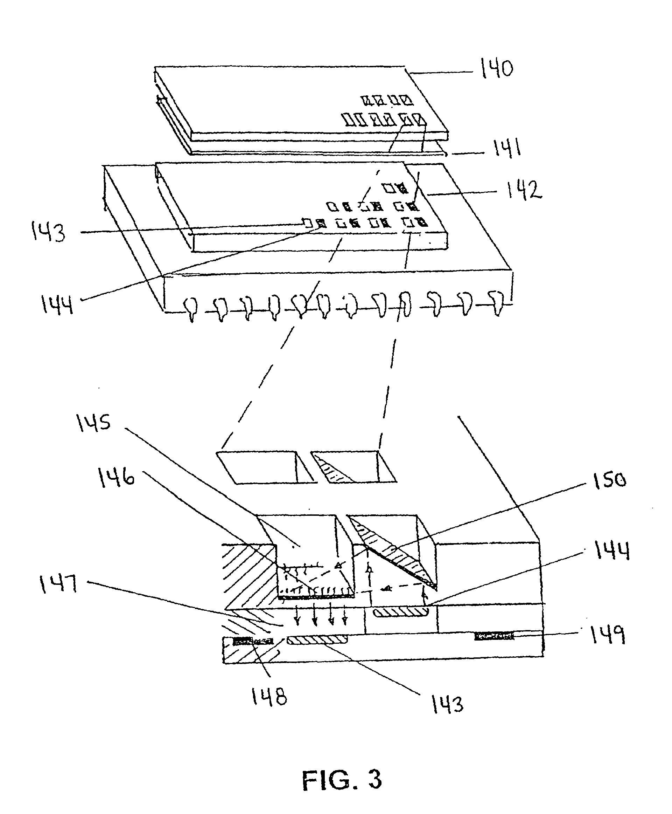

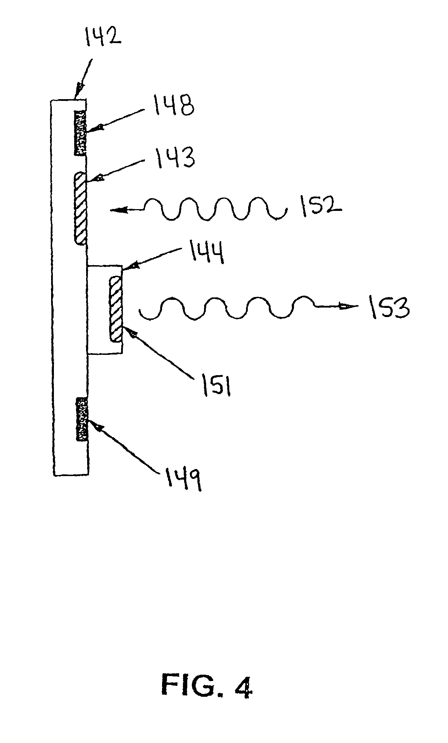

FIG. 3 and FIG. 4 show an example of such integration. This figure schematically shows a two-dimensional array of optical detector-amplifiers integrated on a single IC chip. The insert in this figure shows that each optical detector is a phototransistor coupled to a transimpedence amplifier followed by an amplifier. This block is repeated several times on the IC chip and combined with other electronic elements such as filters and amplifiers, which can also be integrated on the same IC.

The operational amplifier used with the phototransistor is a two-stage, unbuffered amplifier. The circuit is compact, occupying an area of only 185 µm x 200 µm, yet has moderately high performance. It was designed to be useful for wide-band amplification and low-level signals. The gain-bandwidth product is 70 Mhz and the amplifier is stable for gains greater than 10. Other typical characteristics include: input offset voltage less than 5 mV, DC gain of 220, positive slew rate of 80 V/µs and negative slew rate of 9V/µs. The circuit requires 2.5 mW from a single 5-V supply. In the preferred embodiement this IC chip performed the complete conversion from an optical signal to an electrical signal suitable for data digitization and capture by a computer.

FIG. 4 shows the physical layout of the phototransistor and amplifier circuit. The circuit was fabricated in a 2-µm, *p*-well CMOS process and occupied an area of 160,000 square microns. The phototransistor is composed of 220 phototransistor cells connected in parallel. An individual phototransistor cell occupied 760 square microns. The transimpedence amplifier had a gain of 100 kV/A. As the phototransistors were coupled with the 10-fold amplifier gain, the resulting gain was $10^6$ VIA. The phototransistors had a conversion gain on the order of 10 $\mu$A/$\mu$W, so the entire chain had an approximate conversion gain of 10 V/$\mu$W. The exact gain generally depends on the spectral region of interest and, to some extent, on the signal level being monitored.

The above described elements may be modified to tailor the devices to specific applications. Since the phototransistor is made from basic photocell elements, it can be connected to as many cells as needed to create the desired geometry or required number of channels to adapt the detector to a specific application. Similarly, the gain and bandwidth of the amplifiers can be adjusted using simple resistor or capacitor changes as the application requires. Other light sensing structures in addition to the phototransistor may be fabricated using standard Complementary Metal Oxide Semiconductor (CMOS) processing steps. Several photodiode structures are possible using the *p-n* junctions that would ordinarily form wells or transistors.

### 4.12.3 ICM SYSTEM WITH INTEGRATED PHOTOTRANSISTORS

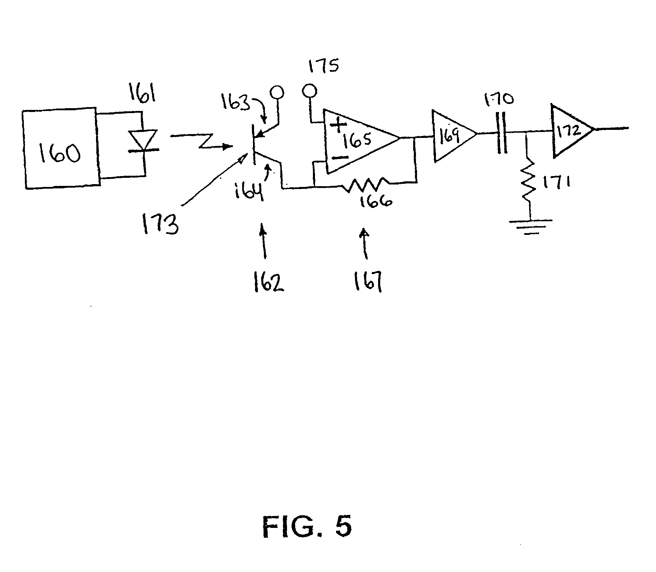

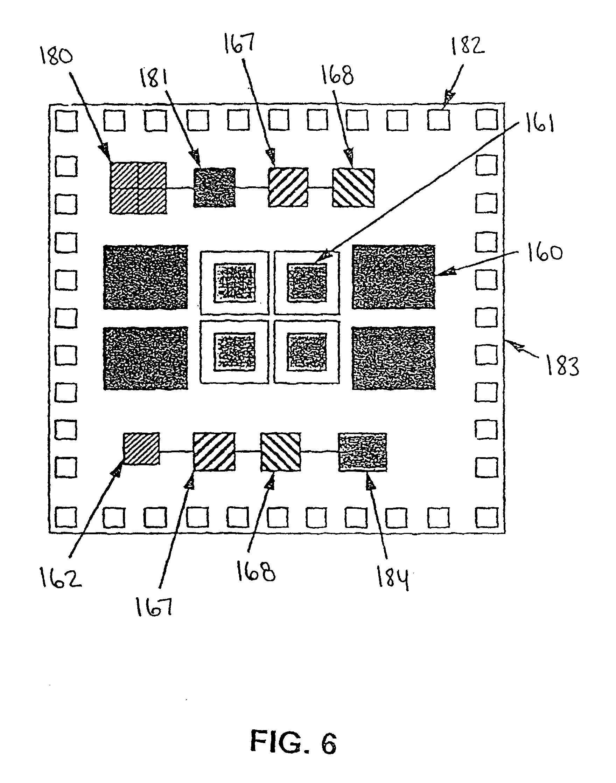

A biochip having multiple arrays of exciting light sources and detectors is shown in FIG. 3. A design of an ICM system for such a biochip with integrated light emitting diodes (LED) excitation sources is illustrated in FIG. 4. This ICM system contains both GaAs chips used as light sources and the phototransistor as the detector. The electrooptic circuit and layout of this device shown in FIG. 5 and FIG. 6 FIG.5 and FIG. 6 show the schematic diagram of the IC circuit and layout for the IR light source and the analog signal detection, respectively.

### 4.12.4 ICM SYSTEM WITH 4x4 *N-WELL* PHOTODIODE ARRAY

A second ICM system includes large-area, $4 \times 4$ *n*-well integrated amplifier-photodiode array that has been designed as a single, custom integrated circuit (IC), fabricated for the biochip. This IC device is coupled to the biosensor and is designed for monitoring very low light levels.

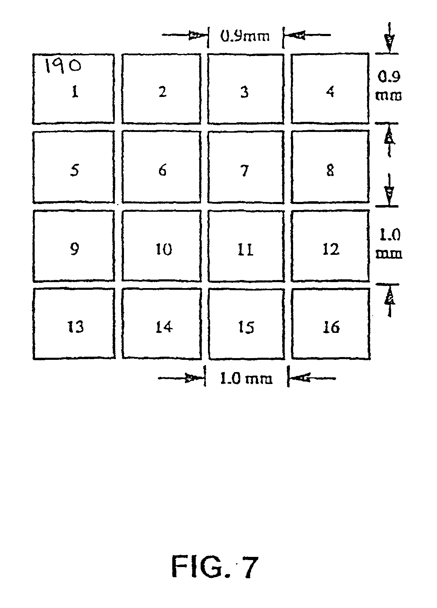

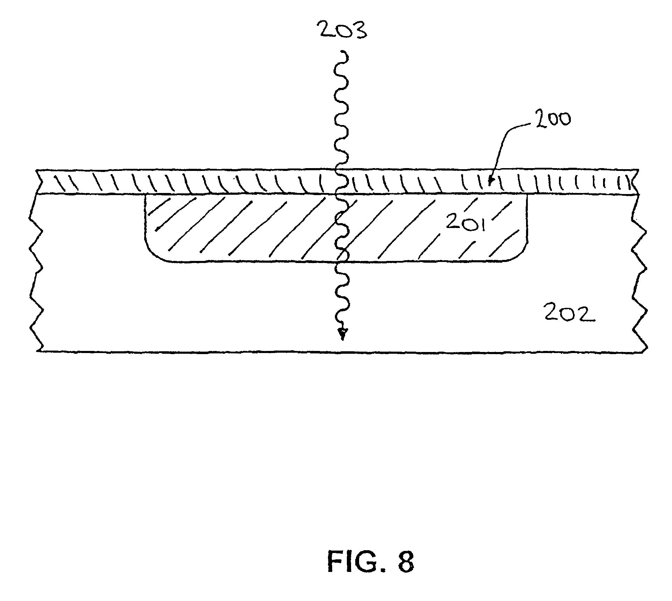

The physical layout of the IC array is illustrated in FIG. 7 shows the individual photodiodes have 0.9-mm square size and are arrayed on a 1-mm grid. The photodiodes and the accompanying electronic circuitry were fabricated using a standard 1.2-micron *n*-well CMOS process from Orbit Semiconductor (Sunnyvale, Calif.). The use of this type of standard process allows the production of photodiodes, phototransistors as well as other numerous types of analog and digital circuitry in a single IC chip. The photodiodes themselves are produced using the *n*-well structure that is generally used to make resistors or as the body material for a PMOS transistor. FIG. 8 shows a schematic cross-sectional drawing of the *n*-well photodiode. Since the anode of the diode is the p-type substrate material, which is common to every circuit on the IC chip, only the cathode is available for monitoring the photocurrent and the photodiode is constrained to operate with a reverse bias.

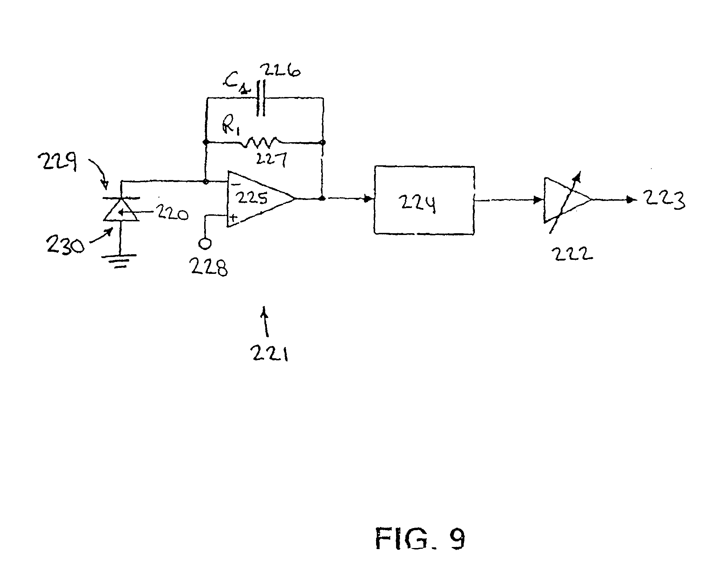

FIG. 9 shows a one-diode version of the circuit. The operational amplifier and feedback resistor $R_1$(227) form a transimpedence amplifier that is used to convert the photocurrent into a voltage. The conversion gain (V/A) is determined by the value of rasistor 227. The feedback capacitor performs two functions: (a) it prevents the amplifier circuit from oscillating, and (b) it limits the bandwidth of the circuit. Generally, the bandwidth should be no more than that necessary for the desired measurement. Reduction of the bandwidth decreases the noise present at the amplifier's output, so if this reduction can be accomplished without attenuating the signal, a net gain in signal-to-noise ratio is achieved. For this circuit, the signal bandwidth is given by $f=1/(2\pi R_1 C_1)$, where $R_1$ is the resistance and $C_1$ is the capacitance.

The voltage applied to the non-inverting input of the operational amplifier determines the reverse bias applied to the photodiode. The IC can be operated with a single %-v supply. For example, if 2 V is applied to the non-inverting input, then the dc level of the other input and the output will also be 2 V, so the reverse bias on the diode will be 2 V. Photocurrents flowing into the diode will also flow through the feedback resistor, causing the amplifier output to become more positive. As the operational amplifier output cannot exceed the positive supply, the maximum output will be approximately 5 V, so the maximum signal excursion is 3 V, which corresponds to a maximum current of 3 V / R 1.

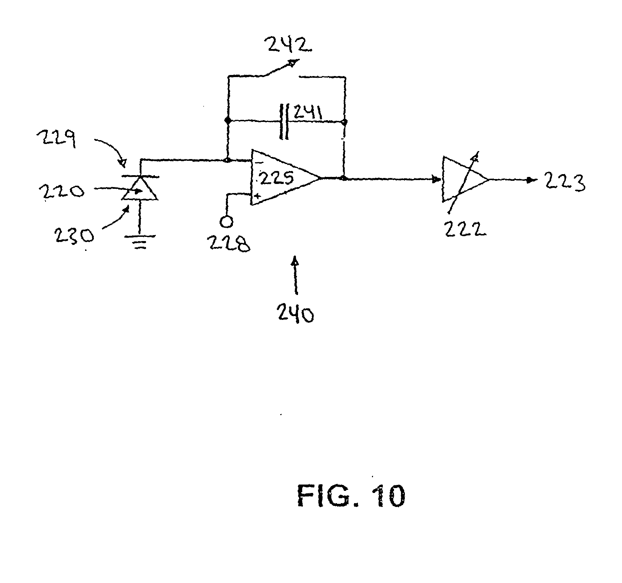

Alternative photodiode and integrated amplifier circuit block diagrams are shown in FIG. 10. Alternate to the transimpedence amplifier plus low-pass filter readout method is the use of an *integrating amplifier* as shown in FIG. 10. In this case, the amplifier integrates the current from the photodiode until the signal is converted to digital format. After conversion, the integrator is reset to its initial state and is capable of starting another measurement. This scheme has the advantage that the integration time can be controlled to allow adapting to various light (and therefore current) levels. The disadvantage of this scheme is that it requires coordination between the analog-to-digital conversion process and the integrator.

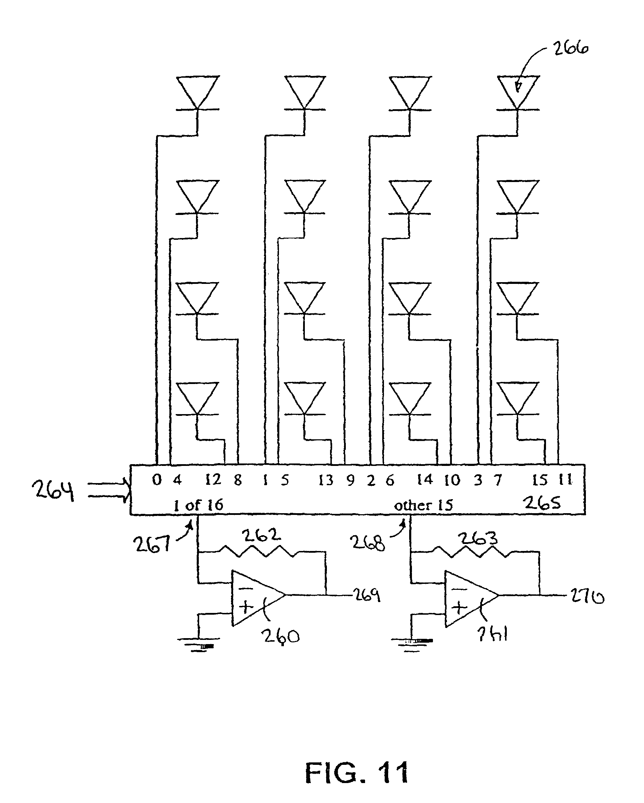

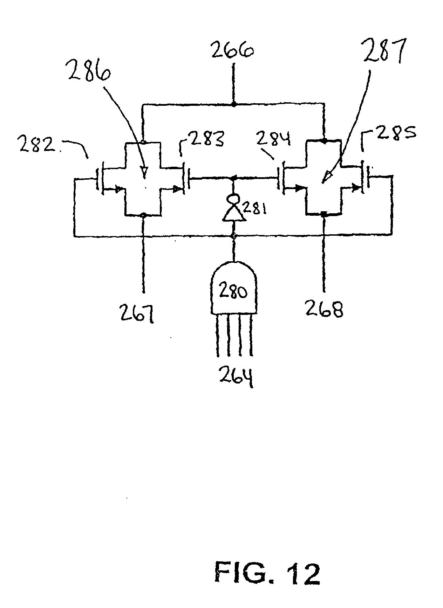

An analog multiplexer is designed to allow any of the elements in the array to be connected to an amplifier. In a specific embodiment, each photodiode could be supplied with its own amplifier. For this many applications, this feature is not necessary unless the additional data acquisition speed due to having parallel channels is required. The multiplexer is made from 16 cells as illustrated in FIG. 11. Each cell has two CMOS switches that are controlled by the output of the address decoder cell. Each cell has a unique 4-bit address. One switch is closed only when that particular cell is the one that is being addressed while the other switch is closed, except when that cell is the one being addressed. This process connects the addressed diode to one amplifier while all the others are connected in parallel to the other amplifier as shown in FIG. 12.

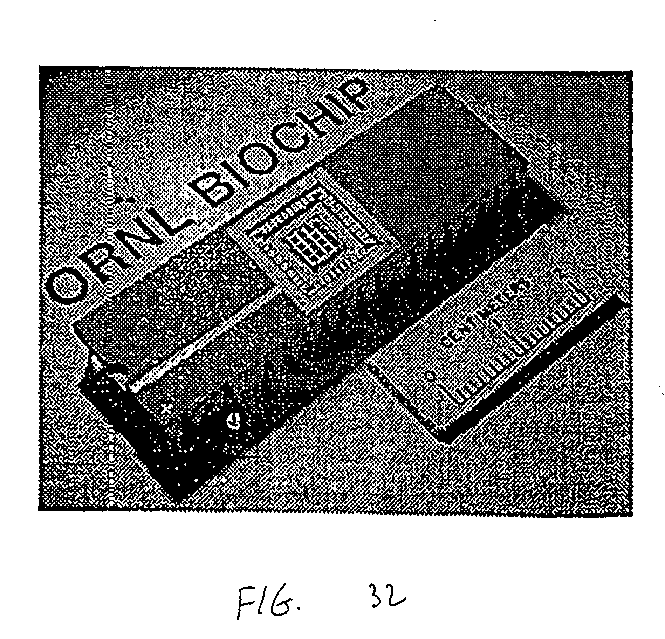

This arrangement allows connecting a 4 x 4 array of light sources (different fluorescent probes, for example) to the photodiode array and reading out the signal levels sequentially. With some modification, a parallel reading system can be designed. Using a single photodiode detector would require mechanical motion to scan the source array. The additional switches and amplifier serve to correctly bias and capture the charge generated by the other photodiodes. Failure to do this would result in erroneous measurements due to the addressed photodiode collecting current generated from elsewhere in the IC. Additionally, the additional amplifier and switches allow using the IC as a single, large area (nearly 4 mm square) photodetector (FIG. 32).

### 4.12.5 ALTERNATIVE PHOTODETECTORS

An avalanche photodiode (APD) provides an alternative solid-state method of detecting low light levels. Advantages of APD arrays over ordinarily used photodiode arrays include electron multiplication obtained by the avalanche process. This may improve the signal-to-noise ratio so that lower light levels are detected.

The process of making an APD and/or APD arrays from silicon includes fabricating structures that are not compatible with steps used in standard CMOS processing. A fully integrated microchip including avalanche photodiodes requires special semiconductor fabricating processes. Such processes are known in the art (Geiger *et al*., 1990; Aubert *et al.,* 1988).

### 4.12.6 ALTERNATE PHOTODIODE ARRAY READOUT SCHEMES

There are several alternate possibilities for reading out an array of photodiodes in addition to the multiplexed scheme that was implemented. For many applications, the low pass filter time constant is set to a large value to improve the signal-to-noise ratio. This requires a long time to acquire data for the whole array if each diode is read sequentially. For example, if the time constant is set to 1 second and the array is $4 \times 4$, then the minimum time to acquire data would be something $4 \times 4 \times 1$ second $\times 5 = 80$ seconds. The last factor of 5 allows for the amplifier and low pass filter to settle to better than 1% accuracy after its input is switched to another photodiode.

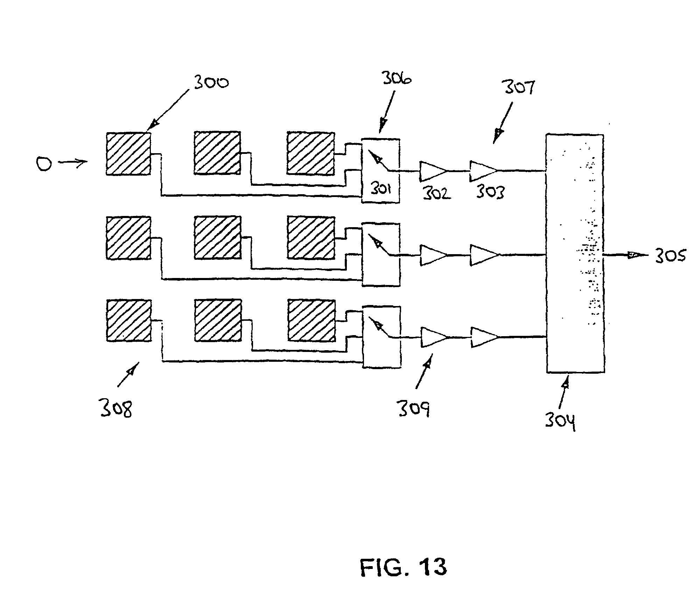

*Partially Parallel Readout Scheme.* According to one embodiement of the invention, the row of diodes are multiplexed into one amplifier/low pass filter circuit. Columns or other sub-units of the array could be used as well. The output of each filter is input to a multi-channel analog-to-digital converter (ADC), which can be implemented on the same IC as the photodiode array, amplifier and filter. Compared to the sequential or serial readout case, the time required is reduced by a factor of n for an $n \times n$ array. The schematic block diagram of this partially parallel readout system is shown in FIG. 13.

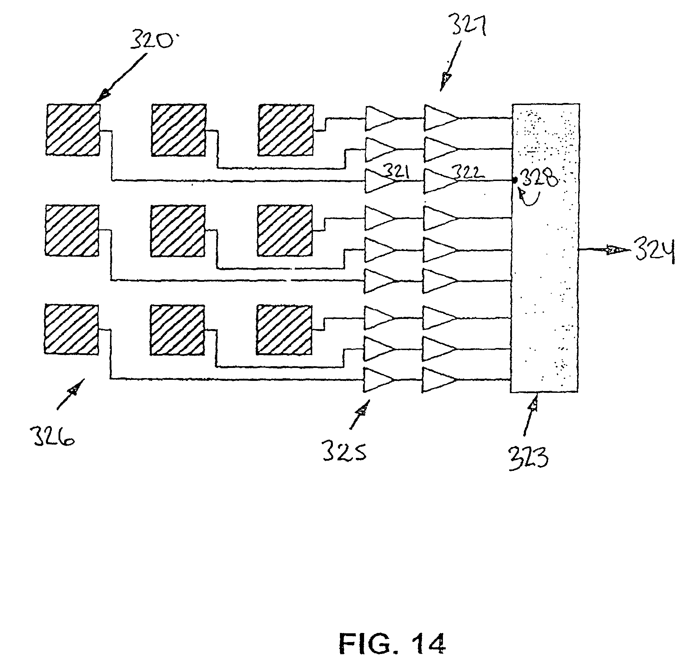

*Fully Parallel Readout Scheme.* This provides the fastest readout speed. Each diode is provided with its own amplifier, low-pass filter and ADC input channel. Compared to the serial readout case, the time required is reduced by a factor of $n^2$ for an $n \times n$ array. The block diagram at a fully parallel readout system is shown in FIG. 14.

### 4.12.7 PHOTODETECTOR ARRAY SIZES

One advantage of a custom biosensor IC is that the photodiodes can be made to physically match the probe. The prototype uses 0.9-mm square photodiodes on a 1 mm $\times$ 1 mm square grid, but arrays with a larger number of smaller photodiodes can be made. Using readily available 1.2-micron technology, photodiodes on a 20-micron grid could be made for a $10 \times 10$ array. This would give 100 photodiodes in an area only 0.04 $mm^2$, *i.e.* 2500 photodiodes per $mm^2$. Even more density is possible using 0.5-micron processes that are commercially available.

Using the serial readout scheme allows most of the chip area to be used for the photodiode array. It is likely that a 1000 element array will fit on a 5 mm $\times$ 6 min IC, which would be considered a medium-sized die format. For large arrays using the fully parallel readout scheme, the limiting factor on IC area is most likely the area required by the wiring and the readout electronics. Future advances in IC technology could increase the density of elements on the chip.

The partially parallel readout scheme is a compromise between the other two cases. Compared with the serial case for a large array, the die size might double to allow partially parallel readout.

## 4.12.8 ALTERNATIVE DETECTION CIRCUITS

Previous examples have shown use of a transimpedence amplifier to convert the current to a voltage and low-pass filters to improve signal-to-noise ratio for very low frequency or dc signals. Such filters may be implemented as digital filters or for different types of signals, such as a fluorescence or phosphorescence decay. A filter matched to the signal could be used to give optimal identification of the signal.

In another embodiment according to the invention, present low current levels may also be measured by other means such as integrating the current for a fixed time prior to measuring the voltage. Alternatively, one may integrate the current until a predetermined voltage level is reached and measure the time required for the integration. These methods use the following relations allowing the determination of current from measured voltages, capacitances and times.

$$\text{charge} = \text{current} \times \text{time}$$

$$\text{charge (on a capacitor)} = \text{capacitance} \times \text{voltage}$$

The circuit used to integrate current is similar to the transimpedence amplifier shown in FIG. 9, except that the feedback element of the amplifier is a capacitor shown as 241 in FIG 10 instead of a resistor.

Another integrating amplifier possibility is to make an amplifier that amplifies the voltage developed across a capacitor which receives the charge.

In yet another embodiment according to the present invention, another method of using an integrating amplifier is to employ an oscillator. The integrator integrates the unknown current to a preset voltage and then is reset by discharging the capacitor with a switch and the integrator is allowed to start again. This then cycles repeatedly. The frequency of oscillation is proportional to the input current. This common technique in circuit design may be used in the systems disclosed herein.

Several methods may be employed to implement the integrating capacitor for the described detection circuits. One may use a capacitor such as polysilicon that is compatible with CMOS technology. Or one may take advantage of the capacitance of the photodiode itself, using the amplifier to amplify the voltage across the photodiode. This has the advantage that fewer components are needed compared to using separate integrating capacitors. For an array of photodiodes, all photodiodes may be integrated simultaneously and one (fast) amplifier used to multiplex the outputs without depriving the circuit of significant measurement time.

## 4.12.9 EXCITATION LIGHT SOURCES

Light sources such as light-emitting diodes (LEDs) and semiconductor lasers may be used in connection with the integrated microchips herein described. One may also choose to employ alternative microlaser systems such as edge-emitting lasers and surface emitting lasers. Vertical-cavity surface-emitting (VCSELs) are particularly suitable light sources for integrated microchips. The linearity of laser arrays makes them ideal for compact 2-dimensional and 3-dimensional configurations in ICM systems. Quantum Cavity (CQ) lasers can also be used due to their small sizes. The QC lasers provide powerful mid-infrared semiconductor lasers that can be used in absorption and Raman mode.

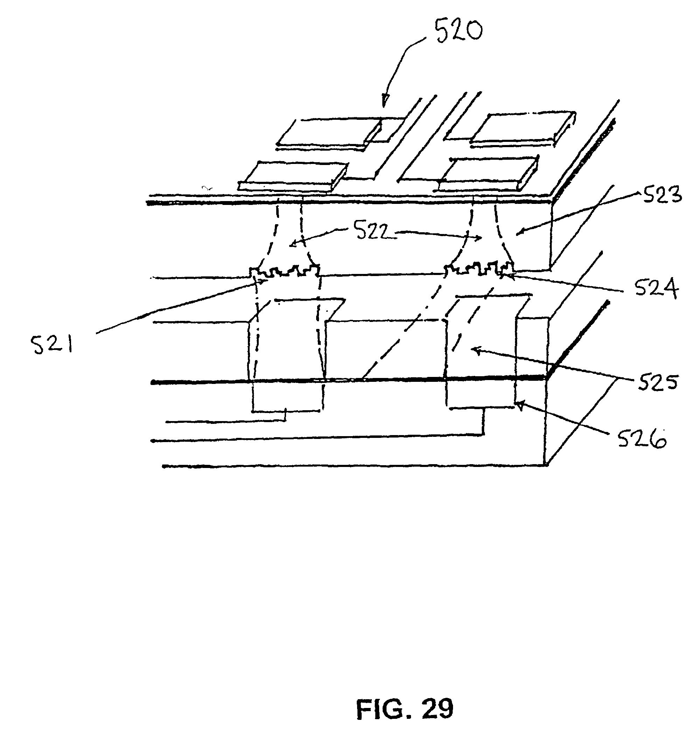

The ability to shape the diverging beam form a surface-emitting laser or a light-emitting diode is important for micro-optic illumination in ICM systems. One may use diffractive optical elements (DOE) systems that can be integrated into the ICBM devices. For example, a wide variety of optical elements may be designed and fabricated using current processing technologies. Compact DOEs and VCSELs can be integrated and fabricated on a single transparent substrate as illustrated in FIG 29. The DOEs can be etched directly into the substrate. Such a compact source-diffractive lens is ideal for miniature optical ICM array design.

## 4.13 EVALUATION OF VISIBLE AND NIR DYE LABELS FOR GENE PROBES

Many photodetectors used in IC chips operate in the red and near infrared (NIR) spectral region. It is therefore important to develop and evaluate DNA labels in the red and NIR region. The inventors have investigated gene probe sensing methods using both visible (350-700 nm) and NIR (700-1000 nm) spectral range fluorescent dye labels. Excitation and detection in the NIR range presents certain difficulties but also offers several advantages. Measurements in the NIR have less background fluorescence interference since very few species with NIR emission occur in samples such as sea water, tissue, serum and other body fluids. Since the intensity of scattered light exhibits frequency[4] (fourth-power) dependence, samples that appear opaque in the visible region may be more transparent in the NIR region.

Finally, an important advantage associated with NIR measurements is the availability of low-cost, miniaturized diode lasers which usually have emission lines in the red and NIR regions.

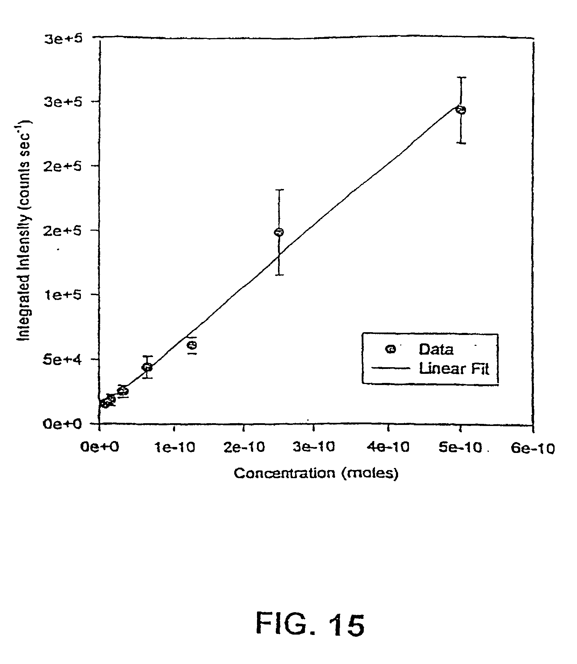

Thus, NIR dye labels in gene probe biosensors offer the advantages mentioned. In one model example, a waveguide multiprobe system with CCD detector configuration was used. The diode laser line of 780 nm at 9.5 mW was used for excitation. FIG. 15 shows a calibration curve for a NIR dye-labeled single-stranded DNA (5'-CCTCCTCCTTCCCAGCAGGG-3'; SEQ ID NO:1) over a concentration range from 1 pmol/μL to 3 fmol/μL. Excitation light was provided by a pen-size 9.5-mW laser diode (780 nm) and measurement times ranged from 1 to 3 min. This calibration curve was linear over the full range of the dye concentration investigated. The limit of optical detection, based on a signal equal to three times the standard deviation of the noise, was estimated to be in the 100 attomole/μL.

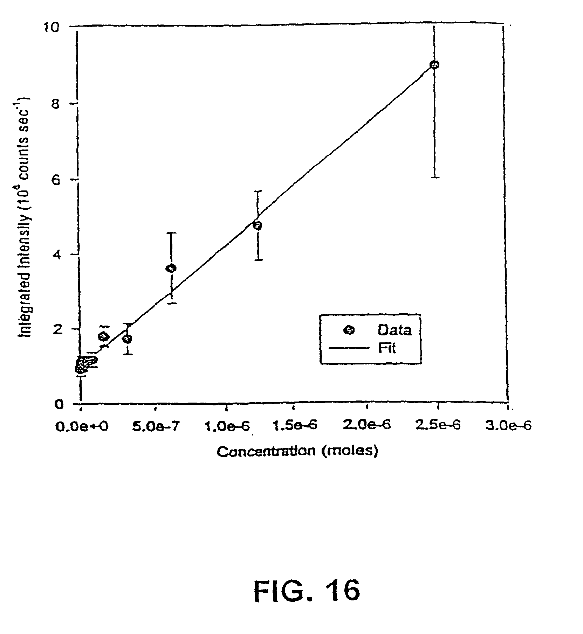

FIG. 16 shows the results of measurements of gene probes tagged with fluorescein, a dye label emitting in the visible range. Measurement times ranged from 0.05 to 2 s. The results show that the calibration curve is linear even with concentrations as low as approximately 2 nmol/μl. The higher detection limit is attributed to an increase in background fluorescence of the waveguide in the visible region.

## 4.14 EVALUATION OF THE ICM BIOSENSOR SYSTEMS

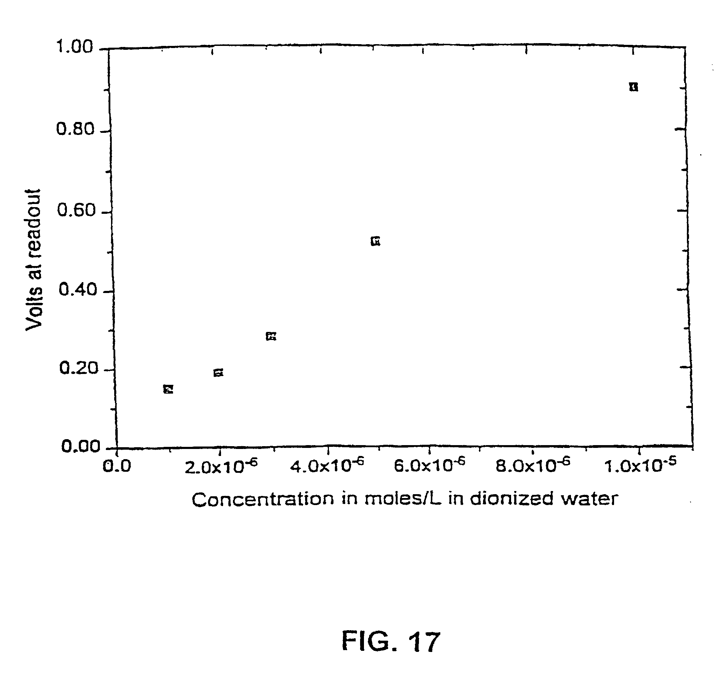

The microchips designed and fabricated for this study were evaluated by measuring the fluorescence signal of a fluorescent dye spotted onto the detector. FIG. 17 shows the performance of the specially designed ICM phototransistor and amplifier circuit (device No. ICI N551-CD2) which consisted of a 2-μm, p-well CMOS process and occupied an area of 160,000 square microns. As described in the experimental section, the phototransistor was actually composed of 220 phototransistor cells connected in parallel. The figure shows the signal output response for various concentration of the dye label Rhodamine-6G excited with a small helium-cadmium laser (8 mW, 325 nm). The results illustrate the linearity of the microchip detector with respect to the label concentration.

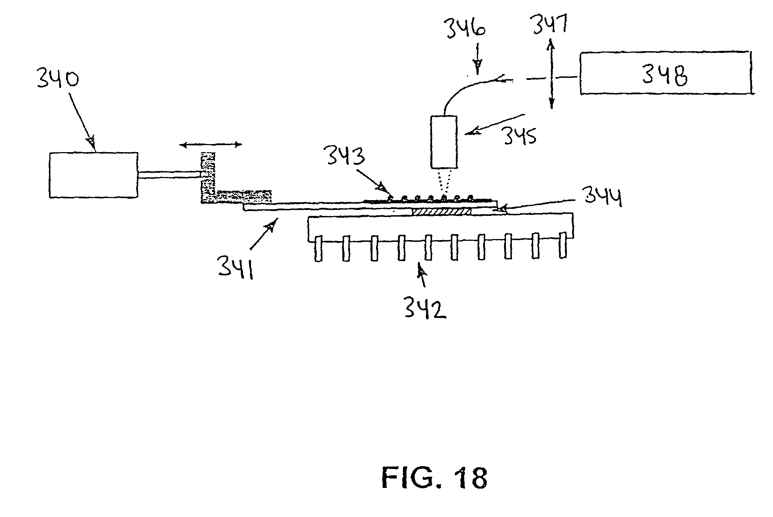

Measurements using an amplifier-phototransistor (APT) ICM device with $4 \times 4$ array of phototransistors (FIG. 18) have been performed. The photocurrent of each channel of the APT microchip was recorded using a digital photometer. The data from the photometer were transferred to a personal computer (PC) *via* an RS-232 link. The samples consisted of an array of microspots of fluorescein-labeled DNA on a membrane. The membrane was placed on a glass slide connected to a linear translation stage. The measurements were made while the translation stage moved the sample arrays over the stationary phototransistor device. Light from an argon ion laser at 488 um was transmitted *via* an optical fiber and focused onto a sample spot. An appropriate optical filter placed between the sample substrate and the detector array was used to reject the laser radiation.

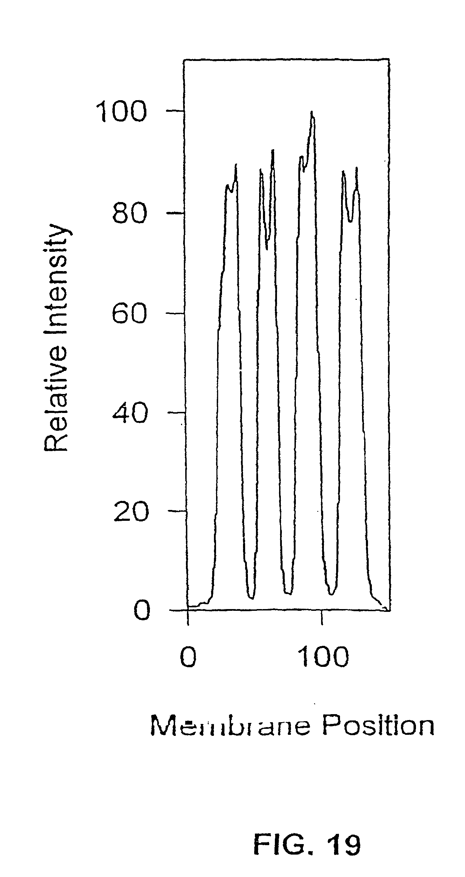

The data obtained with this experimental set up showed four resolved signals as the sample spots moved over an APT microchip channel (FIG. 19). Four sample spots of 1-μL of fluorescein-labeled DNA probes were placed on a nitrocellulose membrane, which was translated over a detection channel of an AP-ICM biochip. As the DNA spot passed over the photodetector, a fluorescence signal is detected. The 4 peaks in FIG. 19 illustrate the detection of the 4 DNA spots on the substrate by the ICM device. FIG. 20 shows the calibration curve of the fluorescein-labeled DNA using the APT microchip device. This demonstrates the possibility for quantitative measurements of the microchip device.

The photodiode array (PDA) microchip device using photodiode array was also evaluated and showed excellent sensitivity with the NIR dye labeled DNA. The results demonstrate the feasibility of the ICM technology for used in DNA biosensor applications.

## 5.0 EXAMPLES

The following examples are included to demonstrate preferred embodiments of the invention. It should be appreciated by those of skill in the art that the techniques disclosed in the examples which follow represent techniques discovered by the inventors to function well in the practice of the invention, and thus can be considered to constitute preferred modes for its practice.

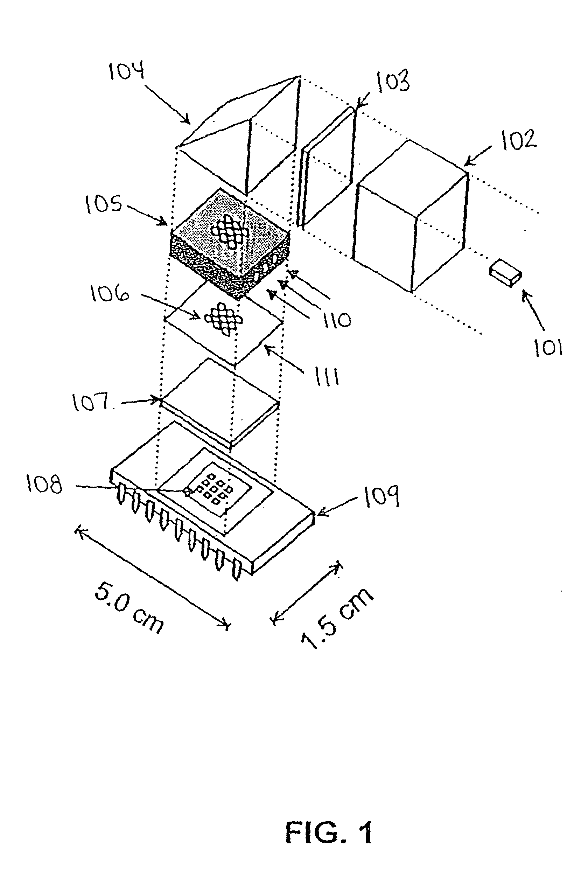

Preferred embodiments of the present invention are illustrated in FIGs. 1-29 of the drawings, like numerals being used to refer to like and corresponding parts of the various drawings.

## 5.1 EXAMPLE 1 -- NUCLEIC ACID BIOCHIP INTEGRATED DEVICE

**FIG. 1** shows an exploded, schematic, perspective view of an example of DNA biochip 109 that combines optical and electrical components. The components shown include a light source **101**, optical element **102**, filter **103**, reflective optic **104**, sampling platform **105**, for receiving and delivering sample **110** onto spot arrays **106** on a substrate **111**, filter **107**, and an array of photodetectors, **108**.

The optical elements shown in FIG. 1 may be positioned separately from the biochip **109**, but are preferably integral with the biochip. In the latter instance, a transparent shield or seal (*e.g.*, plate made with glass or quartz or plastic which is optically transparent at the wavelengths of interest for deletion) isolates the optical component from the biochemical components. Light from light source **101** travels through self-imaging optical element **102** (*e.g.*, defractive optics, binary optics or self-imaging optics systems that transform a point source into 2-dimensional array of light beams) and filter **103** to select and spectrally isolate the incident wavelength and is reflected by reflective optic **104** onto the sample platform **105**. A signal from a hybridization event passes through filter **107**, which blocks the light from the light source **101,** and is detected by the phototransistor array **108.**

## 5.2 EXAMPLE 2 -- OPTICAL DETECTOR AND AMPLIFIER CIRCUIT

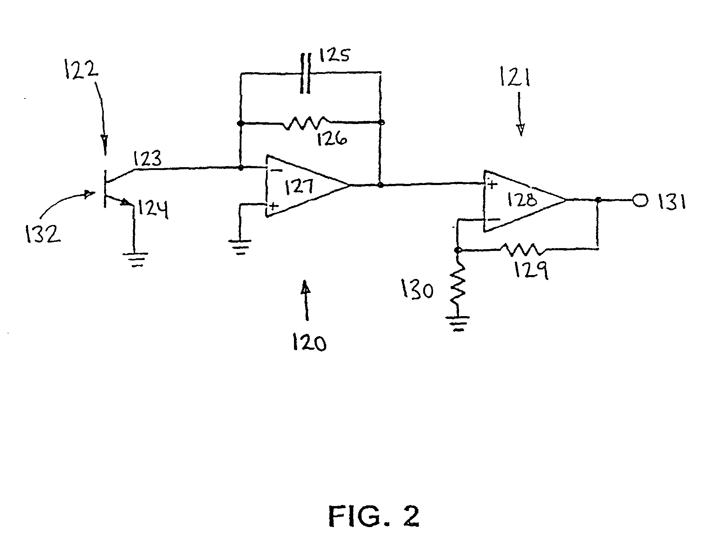

These optical detector and amplifier circuit shown in FIG. 2 may be implemented on an integrated circuit in order to convert an optical signal into an electrical signal suitable for data digitization and capture by a computer. The optical detector and amplifier includes a phototransistor **122** coupled to a transimpedence amplifier **120**, which converts a current signal into a voltage signal and which is followed by an amplifier **121**. The operational amplifier **127** in the transimpedence amplifier **120** is a two-stage, unbuffered amplifier.

In one embodiment, capacitor **125** has a value of 2 pF and resistor **126** has a value of 100 KΩ. The gain of amplifier **121** is equal to 1 + (Resistance **129** / Resistance **130**). Thus, in one specific embodiment for which the gain of amplifier **121** is 10, resistors **129** and **130** are chosen so that their ratio is 9. The circuit is compact (185 $\mu$m $\times$ 200 $\mu$m), but has moderately high performance. It is designed to be useful for wide-band amplification and low-level signals. The gain-bandwidth product is 70 MHz, and the amplifier is stable for gains greater than 10. In addition, the circuit has an input offset voltage less than 5 mV, a DC gain of 220, a positive slew rate of 80 V/$\mu$s, and a negative slew rate of 9 V/$\mu$s. The circuit requires 2.5 mW from a single 5 V supply. The circuit of FIG. 2 may be fabricated in a 2 $\mu$m, P-well CMOS process.

In one embodiment of such a circuit, phototransistor **122** is composed of 220 phototransistor cells that are connected in parallel. Each phototransistor cell occupies an area of 760 square microns, and the entire circuit occupies an area of 160,000 square microns. The transimpedence amplifier has a gain of 100 kV/A and is followed by an amplifier with a gain of 10. Thus, the total gain is $10^6$ V/A. The phototransistors have a conversion gain on the order of 10 $\mu$A/$\mu$W, and thus the entire circuit has an approximate conversion gain of 10 V/$\mu$W. The exact gain generally depends on the spectral region of interest and, to some extent, on the level of the signal being monitored.

The circuit described by FIG. 2 may be modified as required by specific applications. Since the phototransistor cell **122** is composed of basic photocell elements, it may be connected to as many cells as needed to create a desired geometry or a required number of channels needed to adapt the detector to a specific application. Other light sensing structures in addition to the phototransistor may be fabricated using standard CMOS processing steps. Several photodiode structures are possible using standard *pn* junctions.

An avalanche diode is an alternative solid-state device that is capable of detecting very low light levels. An advantage it has over an ordinary photodiode is the electron multiplication obtained by the avalanche process, which results in an improvement in the signal to noise ratio that enables smaller light levels to be detected. A disadvantage of this approach is that avalanche diodes require steps and structures that are not compatible with standard CMOS processing. A fully integrated microchip including avalanche photodiodes would require the development of a special semiconductor fabrication process. An APD differs from an ordinary photodiode in that the applied reverse bias is sufficient to cause electron multiplication. The most reliable way to cause this multiplication is by a large voltage (70-500V) to deplete a p-n junction where both the p- and n-type material is lightly doped. This results in a thick region of depleted material where the electric field is high enough to produce considerable electron multiplication, but not cause electrical breakdown. The applied voltage sets the gain (electron multiplication). In a standard low-voltage IC process, the doping levels are higher and breakdown voltages are lower. Voltages sufficient to cause considerable electron multiplication are too near the breakdown voltage to allow practical APDs. However, in a "high-voltage" process (50-100V breakdown) such as those used for some industrial electronics, it might be possible to fabricate a p-n junction that could be used as an APD while still allowing standard electronics to exist on the same IC. It might be necessary to add additional diffusion steps to the process to create the p-n junction for the APD while the standard process steps would be used to create signal processing electronics.

## 5.3 EXAMPLE 3 -- BIOCHIP

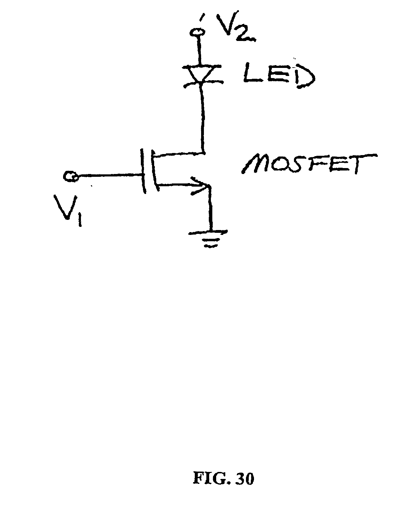

FIG. 3 shows a biochip having multiple arrays of exciting light sources and detectors. The biochip includes a sampling stage layer **140**, a filter and lens stage layer **141,** and a silicon die stage layer **142.** Light from light source **144** driven by a driver circuit **149,** see FIG. 30, is reflected from mirror coating **150.** The mirror surface is designated such that the light beam from **144** is focused and directed to cover the area of the microchamber surface which contain the DNA probe or DNA sample. If luminescence is the signal being detected, the resulting light travels through an optional filter and lens stage **147** and is detected by the photodetector **143** and the associated signal processing electronics **148** such as the system shown in FIG. 9.

### 5.4 EXAMPLE 4 -- BIOCHIP II

FIG. 4 shows a design of an integrated circuit microchip (ICM) system for a biochip as shown in FIG. 3 with substrate **142** and integrated light emitting diode excitation sources **144.** This ICM system contains both a Gallium Arsenide (GaAs) chip **151** used as a light source and a phototransistor **143** used as a detector. Light **153** from the sources **151** strikes luminescent tags, and light **152** from the tags strikes the phototransistor **143,** activating electronics **148.**

### 5.5 EXAMPLE 5 - INTEGRATED LED LIGHT SOURCE AND PHOTOTRANSISTOR DETECTION DEVICE

An LED 161 is driven by an LED driver 160 as shown in FIG. 5. The light is collected by the base **173** of phototransistor **162**. In one embodiment, the emitter **163** of phototransistor **162** is connected to a 5 V source. The current from collector **164** is converted to a voltage by transimpedence amplifier **167** (consisting of operational amplifier **165** with non-inverting input **175** tied to a 2.5 V source and feedback resistor **166**) and then amplified by a gain stage **169** and a power amplifier **172.** AC coupling with capacitor **170** and resistor **171** tied to ground is used to block the *dc* component of the signal. This circuit includes an LED for excitation. The circuit also can operate to detect pulsed or modulated light signals due to its AC coupling scheme.

### 5.6 EXAMPLE 6 -- LAYOUT OF AN INTEGRATED CIRCUIT FOR IMPLEMENTING LIGHT SOURCE AND DETECTION DEVICE

FIG. 6 illustrates an integrated circuit that may be used for implementing the light source and detection device of FIG. 1 or FIG. 5. Silicon die **183** with bonding pads **182** contains a GaAs infrared LED **161** that is driven by LED driver circuit **160.** LED array **180** is multiplexed by multiplexer **181** to amplifiers **167** and **168.** Detector **162** signal is amplified and compared to a reference level by comparator **184.** This may be used to give a simple indication of the total level of fluorescence present.

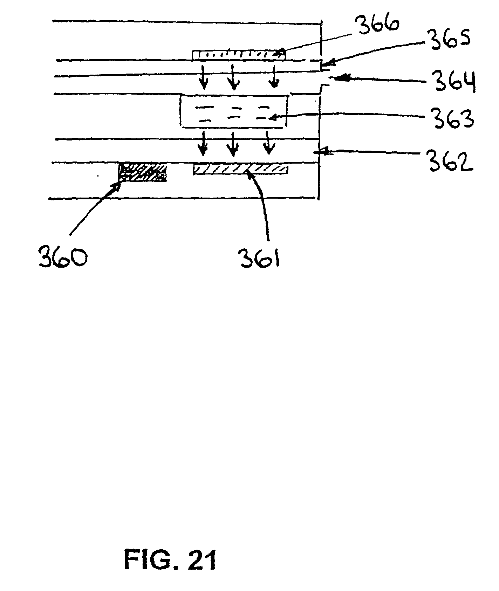

A second embodiment of the present invention includes a large-area, $4 \times 4$ *n*-well integrated amplifier-photodiode array that is designed as a single, custom integrated circuit. This integrated circuit is coupled to a biosensor and is designed for monitoring very low light levels. FIG. 7 shows the physical layout of such an array. The sixteen individual photodiodes are 0.9 mm by 0.9 mm in size and are arranged on a 4 mm by 4 mm grid. FIG. 7 is an example of a 4 x 4 array of detectors which can be used in systems shown in FIG. 21, FIG. 23, FIG. 25 and FIG. 29. The light sources are not shown in FIG. 7. The photodiodes and the accompanying electronic circuitry may be fabricated using a standard 1.2 micron *n*-well CMOS process such as that available from Orbit Semiconductor. The use of this type of standard process allows the production of photodiodes and phototransistors, as well as many other types of analog and digital circuitry, in a single integrated circuit. The photodiodes are produced using the *n*-well structure generally used to make resistors or the body material for a PMOS transistor. Details are shown in FIG. 8.

### 5.7 EXAMPLE 7 -- SCHEMATIC CROSS-SECTION OF AN *N*-WELL PHOTODIODE

FIG. 8 illustrates a schematic cross-section of an *n*-well photodiode that may be used in the photodiode array of FIG. 7. The light **203** travels through oxide layer **200.** Since the anode **202** of the photodiode **190** is the *p*-type substrate material that is common to each circuit on the chip, only the cathode 201 is available for monitoring the photocurrent, and the photodiode is constrained to operate with a reverse bias.

### 5.8 EXAMPLE 8 -- DETECTION CIRCUIT

FIG. 9 shows a photodiode instead of phototransistor circuit that establishes reverse bias of photodiode includes adjustable gain **222**. (Note that FIG. 2 and FIG. 5 show phototransistors instead of photodiodes.) The operational amplifier **225** and the feedback resistor **227** form a transimpedence amplifier **221** that converts the current from the photodiode **220** (with anode **230** tied to ground and cathode **229** tied to the inverting input of operational amplifier **225**) into a voltage. The conversion gain (in volts per amp) is determined by the value of the feedback resistor **227**. The feedback capacitor **226** prevents the amplifier **221** from oscillating and limits the bandwidth of the circuit, which should generally be no greater than required to obtain the desired measurement Reduction of the bandwidth decreases the noise that is present at the amplifier's output and thus, if the signal is not attenuated by the decreased bandwidth, results in a net gain of signal to noise ratio. The signal bandwidth for the circuit in FIG. 9 is $1/(2\pi R_1 C_1)$.

In one embodiment, feedback resistor **227** has a value of I MΩ and feedback capacitor **226** has a value of 100 pF. A bias voltage may be applied to the non-inverting input **228** of operational amplifier **225** to decrease the response time of photodiode **220**. A low pass filter **224** and an adjustable gain **222** precede the analog to digital converter **223** and are included to improve the signal to noise ratio for very low frequency or *dc* signals. The voltage applied to the non-inverting input of the operational amplifier determines the reverse bias applied to the photodiode. The integrated circuit may be operated with a single 5 V supply. If 2 V are applied to the non-inverting input, then the DC level of the other input and the output will also be 2 V, so the reverse bias on the photodiode will be 2 V. Current flowing into the photodiode will also flow through the feedback resistor, causing the amplifier output to become more positive. Since the output of the operational amplifier cannot exceed the value of the positive voltage supply, the maximum output will be approximately 5 V. Thus, the maximum signal excursion will be 3 V, which corresponds to a maximum current of $3/R_1$.

Although this embodiment has illustrated one method for measuring low current levels, many other methods are possible.

For example, one method of determining the current would be to integrate the current using an integrating amplifier for a fixed time and then measure the voltage or to integrate the current until a fixed voltage is reached and then measure the time. A second method to determine the current would be to use an oscillator. In particular, an integrator could continually integrate the unknown current until a preset a voltage was reached and then reset itself. The resulting frequency of oscillation would be proportional to the current. In addition to using standard CMOS capacitors, one could implement this second method using the capacitance of the photodiode itself. Initially, the switch is closed and the capacitance of the photodiode is discharged and there is zero voltage across the photodiode. When the switch is opened, light impinging on the photodiode creates charge which produces a voltage on the photodiode capacitance. More light increases the voltage which is amplified by the amplifier. When the output of the amplifier exceeds the reference voltage, the output of the comparator changes state, firing the one shot which in turn closes the switch and discharges the photodiode capacitance. This resets the amplifier input to the initial state and the comparator also returns to its initial state. When the one-shot times out, the switch opens and the process of charging can start again. A greater light level results in faster charging and therefore a higher frequency output of the oscillator. This results in an array of integrators that could be multiplexed to a single fast amplifier without the loss of significant measurement time.

### 5.9 EXAMPLE 9 -- ALTERNATIVE DETECTION CIRCUIT

FIG. 10 shows an alternative detection circuit that may be used in conjunction with the photodiode array of FIG. 7. The circuit in FIG. 10 uses an integrating amplifier **240** in place of the transimpedence amplifier **221** in FIG. 9. The low pass filter **224** in FIG. 9 is not required since the integrator **240** in FIG. 10 is an automatically tracking low pass filter during each sample period. In FIG. 10, the integrator **240** integrates the current from the photodiode **220** until the signal is sampled, at which point the integrator **240** is cleared by closing reset switch **242**. An advantage of this approach is that the integration time may be adapted to various light (and therefore current) levels. This approach requires coordination between the analog to digital conversion process and the integrator. The analog-to-digital converter must sample the output of the programmable gain amplifier when the integration time is completed and before the integrator is reset.

### 5.10 EXAMPLE 10 -- ANALOG MULTIPLEX

FIG. 11 shows an analog multiplexer **265** that may be used to allow any single element **266** in the photodiode array of FIG. 7 to be connected through output **267** to an amplifier **260**. One possible implementation of this multiplexer for use with 16 elements is shown in FIG. 12. Each element **266** has a unique four bit address and two CMOS switches **286** and **287** that are controlled by the output of the cell's address decoder **280**. Switch **286** is closed only when that particular cell is being addressed, and switch **287** is closed except when that particular cell is being addressed. In this way, the addressed photodiode is connected to one amplifier 260 through output **267** and the non-addressed photodiodes are connected in parallel to another amplifier **261** through output **268.** Switch **286** is composed of parallel CMOS switches **282** and **283,** and switch **287** is composed of parallel CMOS switches **284** and **285.** One gate in each pair is controlled by the output of the address decoder and the other gate is controlled by the inverse **281** of the output of the address decoder. This configuration allows the switching of signals that range from the ground potential to the supply voltage. (The individual CMOS gates are not forward biased at the peak of the signal swing).

### 5.11 EXAMPLE 11 -- LIGHT SOURCE ARRAY

Using the multiplexer in FIG. 11, FIG. 12 shows use of a 4 × 4 array of light sources (such as luminescent probes, for example) to be connected to a photodiode array from which signal levels are read sequentially. Using a single photodiode detector instead would require mechanical motion to scan the source array. The amplifier for the non-addressed photodiodes allows the charges from these photodiodes to be correctly biased and captured. Failure to use this amplifier may result in an erroneous measurement from the addressed photodiode due to its collection of currents from non-addressed photodiodes. Further, this multiplexed, dual amplifier approach allows the chip to function as a single, large area (nearly 4 mm square) photodetector.

For many applications, the low pass filter time constant is large in order to improve the signal to noise ratio. If the data are read sequentially, a large time constant can greatly increase the time needed to read the entire array. For example, if the time constant is set to 1 second and the array is $4 \times 4$, then the minimum time to acquire data would be 80 seconds, where a factor of 5 has been included to allow the amplifier and low pass filter to settle after their input is switched to another photodiode.

### 5.12 EXAMPLE 12 -- PARTIAL PARALLEL METHOD

As an alternative to the multiplexed method shown in FIG. 11 and FIG. 12, a partially parallel method may be used to obtain data from the photodiode array shown in FIG. 7. Such a partially parallel method is illustrated by FIG. 13. In this case, a row **310** of photodiodes **300** is multiplexed by multiplexer **301** into an amplifier **302** and a low pass filter **303**. Columns or other sub-units of the array may be used in place of rows. The output of each low pass filter is input into a multi-channel analog to digital converter **304**, which may be implemented on the same integrated circuit as the photodiode array, amplifier, and low pass filter. The A/D converter **304** produces output **305.** For an n $\times$ n array, such a partially parallel method reduces the time required for a sequential method by a factor of *n*.

### 5.13 EXAMPLE 13 -- FULLY PARALLEL READOUT

Yet another method that may be used to obtain data from the photodiode array shown in FIG. 7 is the fully parallel readout scheme shown in FIG. 14. In this case, each photodiode **320** is provided with its own amplifier **321,** low pass filter **322,** and analog to digital converter input **328.** For an *n $\times$ n array*, such a fully parallel method reduces the time required for a sequential method by a factor of $n^2$.

One advantage of a custom biosensor integrated circuit is that the photodiodes may be made to physically match the probe. The embodiment of FIG. 7 uses 0.9 mm square photodiodes on a 1 mm by 1 mm square grid, but arrays with a larger number of smaller photodiodes are also possible. Using the readily available 1.2 micron technology, a 10 by 10 array may be made using photodiodes on a 20 micron grid, thereby providing 100 photodiodes in an area of only 0.04 mm$^2$, which is a density of 2500 photodiodes per mm$^2$. An even greater density is possible using 0.5 micron processes or 0.25-micron (or less) processes, which are also commercially available. These processes use lithographic methods to fabricate these submicron structures to allow greater density of photodetector and light source arrays.

### 5.14 EXAMPLE 14 -- NIR DYE-LABELED NUCLEIC ACIDS

FIG. 15 shows a calibration curve for an NIR dye-labeled, single-stranded DNA (sequence: 5'-CCTCCTCCTTCCCAGCAGGG-3'; SEQ ID NO:1 ) over a concentration range from 1 pmol/μL to 3 fmol/μL. Excitation light was provided by a pen-size 9.5 mW laser diode (780 nm), and measurement times ranged from 1 to 3 minutes. Data were collected using a waveguide multiprobe system with a charge-coupled device (CCD) detector. This calibration curve was linear over the full range of the dye concentration investigated. The limit of optical detection, based on a signal equal to three times the standard deviation of the noise, was estimated to be in the range of 100 attomole/μL.

### 5.15 EXAMPLE 15 -- FLUORESCENCE MEASUREMENTS ON TAGGED GENE PROBES

FIG. 16 shows the results of measurements of gene probes tagged with fluorescein, a dye label emitting in the visible range. Measurement times ranged from 0.05 to 2 s. The results show that the calibration curve is linear down to a concentration of approximately 2 nmol/μL. The higher detection limit is attributable to an increase in background fluorescence of the waveguide in the visible region. The measurements were performed using the CCD detection system. The results demonstrate the possibility for quantitative analysis of fluorescein-labeled DNA probes.

### 5.16 EXAMPLE 16 -- SIGNAL OUTPUT RESPONSE FOR FLUORESCENT DYE

The present invention has been evaluated by measuring the fluorescence signal of a fluorescent dye spotted into the detector. FIG. 17 shows the performance of an ICM phototransistor and amplifier circuit consisting of a 2 μm, *p*-well CMOS process occupying an area of 160,000 square microns and 220 phototransistor cells connected in parallel.

Shown in FIG. 17 is the signal output response for various concentrations of the dye label Rhodamine-6G excited with a small helium-cadmium laser (8 mW, 325 nm) illustrating the linearity of the microchip detector with respect to label concentration.

### 5.17 EXAMPLE 17 -- EVALUATION OF A 4 × 4 ARRAY INTEGRATED CHIP

FIG. 18 illustrates a setup for the evaluation of a 4 × 4 array amplifier/phototransistor integrated circuit microchip (AP-ICM) device. The current in each channel was recorded using a digital photometer. The data from the photometer was transferred to a personal computer *via* a serial RS-232 line. The samples consisted of an array of microspots **343** of fluorescein-labeled DNA on a membrane. The membrane was placed on a glass slide **341** connected to a linear translation stage **340**. The measurements were made while the translation stage moved the sample arrays over the stationary phototransistor device **342.** Light from an argon ion laser 348 at 488 nm was transmitted *via* an optical fiber 346 through focusing optics **345** and **347** and was focused onto a sample spot. An appropriate optical filter 344 was placed between the sample substrate 343 and the detector array **342** in order to reject the laser radiation. The set-up described here was designed to evaluate the response of each individual photodetector on the chip. Application or systems are contemplated where multiple samples are scanned over individual detectors.

### 5.18 EXAMPLE 18 - DETECTION OF DNAS USING THE AP-ICM

FIG. 19 shows the results when four sample spots of 1 µL of fluorescein labeled DNA were placed on a nitrocellulose membrane that was translated over a detection channel of the AP-ICM device shown in FIG. 18. As the DNA spot passed over the photodetector, a fluorescence signal was detected. The four peaks in FIG. 19 illustrate the detection of the four DNA spots on the substrate by the AP-ICM device.

FIG. 20 shows the calibration curve of fluorescein-labeled DNA using the AP-ICM device shown in FIG. 18. The results demonstrate the capability of the AP-ICM chips to produce quantitative measurements of fluorescein-labeled DNA.

### 5.19 EXAMPLE 19 -- ABSORPTION AND REFLECTION MEASUREMENTS

FIG. 21 shows an embodiment of the present invention that is used for absorption and reflection measurements. Light from LED or diode laser **366** passes through an optional optical filter or lens stage **365** and the sample **363**, which can be accessed through an optional sample inlet **364.** Light from the sample **363** passes through an optical filter or lens stage **362** and impinges upon photodetector **361**. Signal processor **360** receives the output from photodetector **361.**

### 5.20 EXAMPLE 20 -- SIMULTANEOUS DETECTION OF FLUORESCENCE AND RAMAN

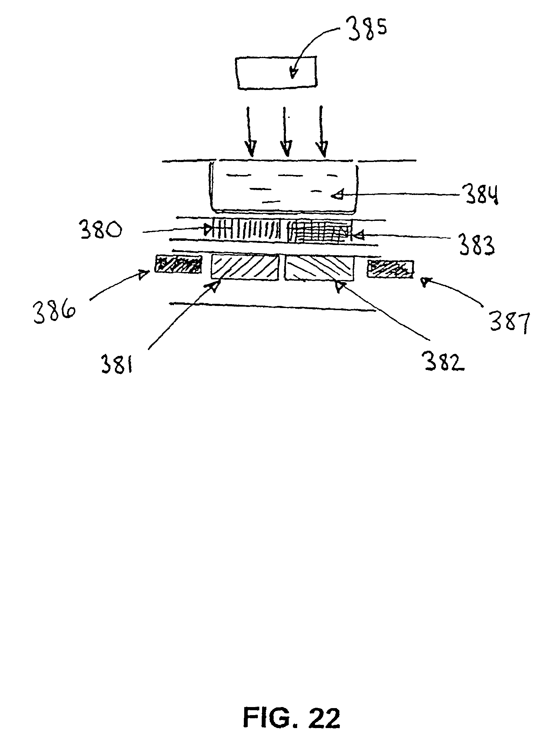

FIG. 22 shows an embodiment of the present invention that uses two photodetectors for simultaneous fluorescence and Raman measurements. Light from an excitation source **385** impinges upon sample **384**. Light from sample **384** passes through the lens stage and optical filter **380** for fluorescence measurement and through the lens stage and optical filter **383** for Raman measurement. Light passing through lens stage and optical filter **380** is measured by photodetector **381** and processed by signal processor **386.** Light passing through lens stage and optical filter **383** is measure by photodetector **382** and processed by signal processor **387.**

### 5.21 EXAMPLE 21 -- MULTI-DETECTION APPARATUS

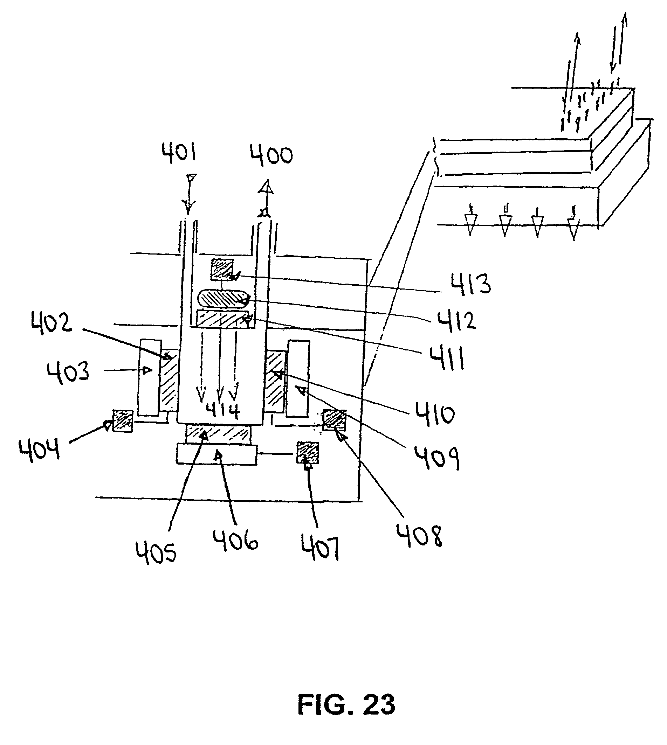

FIG. 23 shows an embodiment of the present invention that uses three photodetectors for simultaneous absorption, fluorescence, and Raman measurements. A sample enters the sample chamber **414** through sample inlet **401** and exits through sample outlet **400**. An LED or diode laser **412** receives power from a power supply **413** and directs light through an excitation filter **411** into sample chamber **414**. Fluorescence measurements are made by optical filter and lens **402**, photodetector **403**, and signal processor **404**. Absorption measurements are made by optical filter and lens **405**, photodetector **406**, and signal processor **407**. Raman measurements are made by optical filter and lens **410,** photodetector **409,** and signal processor **408.**

### 5.22 EXAMPLE 22 -- APPARATUS FOR MICROFLUIDIC DEVICES

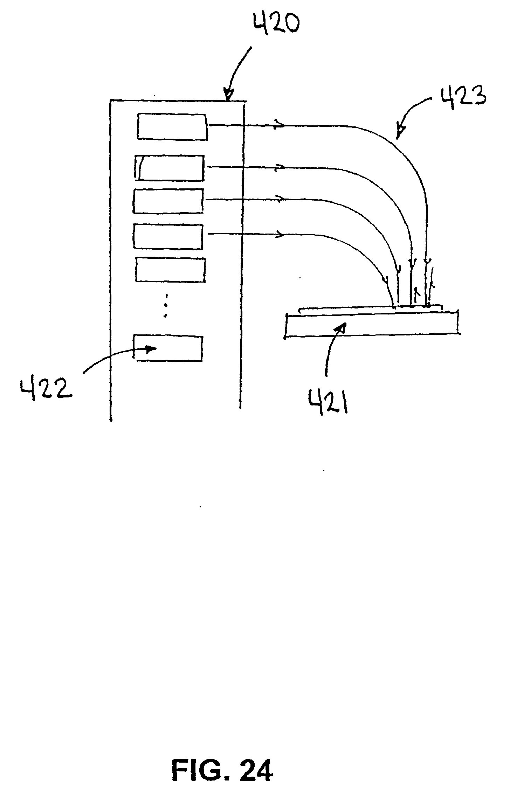

FIG. 24 shows an embodiment of the present invention that is used to detect samples from a microfluidic device such as a capillary electrophoresis array, a liquid chromatography array, a gas chromatography array, or a Lab-on-chip system. A microfluidic array **420** of microfluidic devices **422** direct samples through microfluidic channels **423** to the ICM chip **421.**

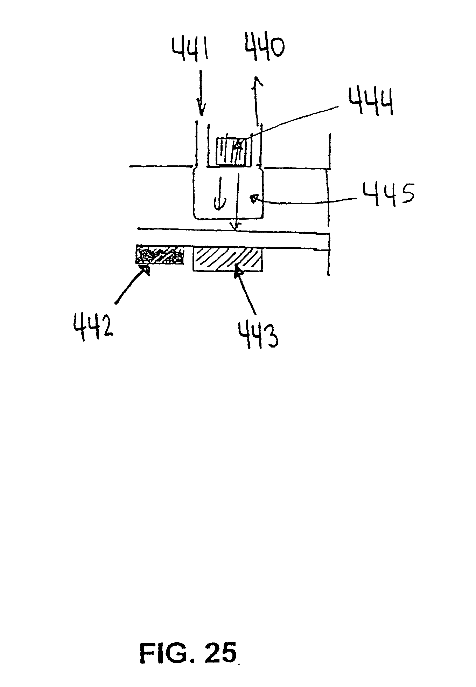

FIG. 25 Shows a detailed drawing of the ICM chip **421** used in the embodiment depicted by FIG. 24. The sample from the microfluidic device **422** enters sample chamber **445** through inlet **441** and exits through outlet **440**. Light from LED **444** enters sample chamber **445**, and the results are detected by photodetector **443** and signal processor **442**.

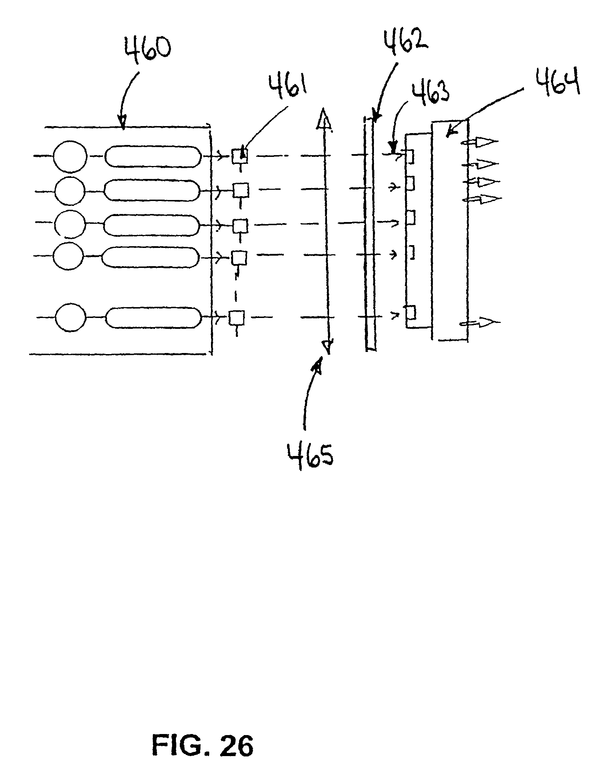

FIG. 26 shows an embodiment of the present invention that is used to detect samples from a microfluidic device using an imaging lens, binary optics, or a lens array to image each microfluidic channel onto a detector element of the ICM, Samples are directed from the microfluidics system **460** into sample chambers **461**. Light from the sample chambers **461** is directed through imaging lens, binary optics, or lens array **465** and through an optical filter **462** onto photodetectors **463** on an ICM chip **464.**

### 5.23 EXAMPLE 23 -- MICRO-ELECTROMECHANICAL SYSTEMS

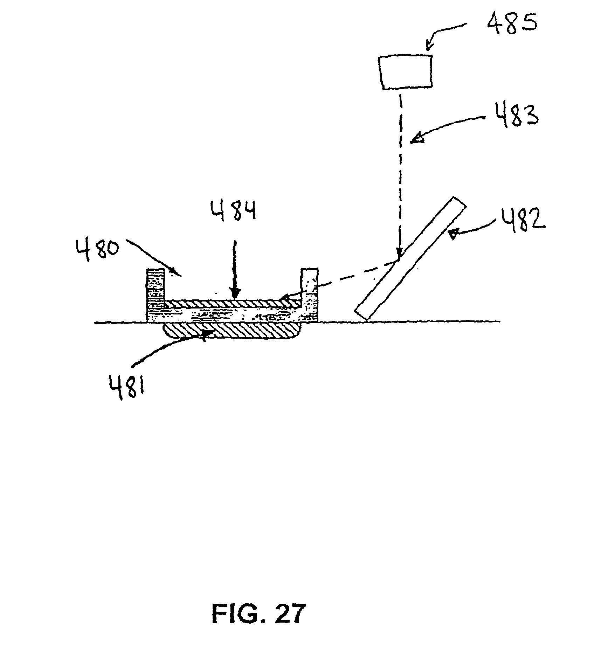

FIG. 27 shows a micro-electromechanical system (MEMS) that is used to construct an ICM with Random Access Microsensing. Light **483** from laser **485** is directed toward an electrically positionable MEMs mirror **482,** which then selectively directs the light onto DNA coated substrate **484** in a sample microchamber **480.** The results are detected by a photodiode **481.**

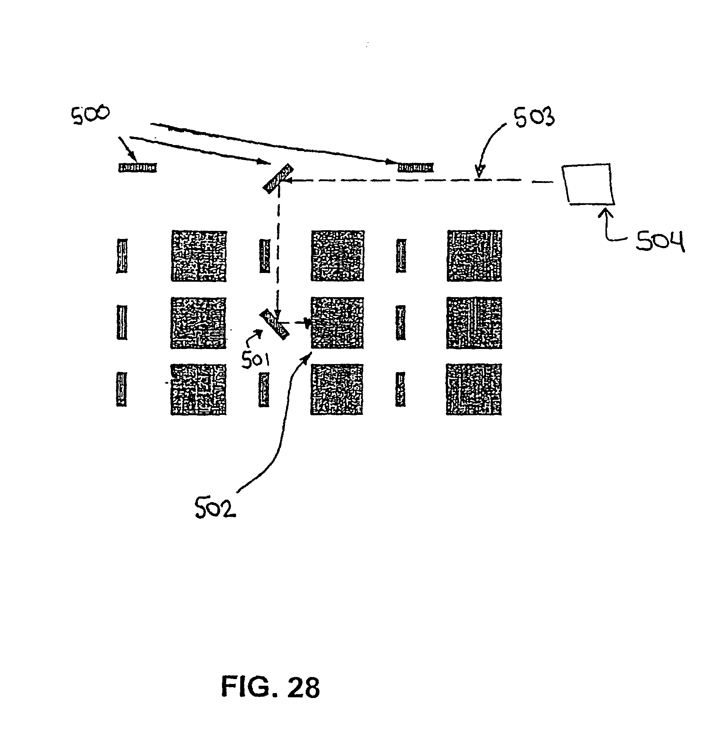

FIG. 28 shows an overview of an ICM system that uses the MEMS depicted in FIG. 27. Light **503** from laser **504** is selectively redirected by column select mirrors **500** onto a particular cell select mirror **501,** which then directs the light into the corresponding biosensor cell **502.** The mirrors as well as the diodes are etched and formed from silicon. The general fabrication steps for MEMs are similar to IC fabrication procedures using lithographic techniques. For MEMs, additional etching steps are performed to free to movage devices (*e.g.*, mirrors) from the substrate. The mirrors allow the light from the laser **504** to be directed toward any individual biosensor cell.

### 5.24 EXAMPLE 24 -- VERTICAL-CAVITY SURFACE-EMITTING LASER APPARATUS

FIG. 29 shows an embodiment of the present invention that uses individually addressable, integrated vertical-cavity surface-emitting lasers (VCSEL) and on-axis and/or off-axis diffractive lenses. The linearity of VCSEL arrays make them ideal for compact two-dimensional and three-dimensional configuration in ICM systems. The ability to shape a diverging beam from a surface-emitting laser or an LED is important for micro-optic illumination in ICM systems. The diffractive optical element (DOE) system shown in FIG. 29 allows for this ability and, as shown, may be integrated and fabricated with a VCSEL onto a single transparent substrate. Such a compact source-diffractive lens is ideal for a miniature optical ICM array. Note, as shown in FIG. 29, that a DOE system can be constructed to produce either on-axis or off-axis beams. An on-axis configuration is suitable for absorption measurements, whereas an off-axis configuration is suitable for emission measurements. The off-axis beam can be constructed so that it does not impinge the detector, thus minimizing scattered light.

Light from the VCSEL array **520** is directed through beam waists **522** constructed on the GaAs substrate **523** and then through either an on-axis diffractive lens **521** or an off-axis diffractive lens **524.** The results from sample chamber **525** are detected by photodetector **526**.

### 5.25 EXAMPLE 25 -- DEVELOPMENT OF A DNA BIOCHIP FOR GENE DIAGNOSIS

Rapid, simple, cost-effective medical devices for screening multiple medical diseases and infectious pathogens are essential for early diagnosis and improved treatments of many illnesses. An important factor in medical diagnostics is rapid, selective, and sensitive detection of biochemical substances (proteins, metabolites, nucleic acids), biological species or living systems (bacteria, virus or related components) at ultratrace levels in biological samples (*e.g.*, tissues, blood and other bodily fluids). To achieve the required level of sensitivity and specificity in detection, it is often necessary to use a biosensor that is capable of identifying and differentiating a large number of biochemical constituents in complex samples. Living systems possess exquisite recognition elements (*e.g.,* antibody, enzyme, gene probes, *etc.),* often referred to as bioreceptors, which allow specific identification and detection of complex chemical and biological species. Biosensors exploit this powerful molecular recognition capability of bioreceptors. Due to the exquisite specificity of the DNA hybridization process, there is an increasing interest in the development of DNA bioreceptor-based analytical systems (Vo-Dinh *et al.,* 1994; Isola *et al.,* 1996; Alarie *et al.*, 1992; Vo-Dinh *et al.,* 1987; Vo-Dinh *et al.,* 1991; Stevenson *et al.,* 1994). Gene probes using a novel detection scheme based on surface-enhanced Raman scattering (SERS) labels were recently developed in the inventors' laboratory to enhance both the selectivity and sensitivity of DNA biosensors (Vo-Dinh *et al.*, 1994). A fluorescence-based fiberoptic genosensor for *Mycobacterium tuberculosis* was reported (Isola *et al.*, 1996).

This work involves the development of a new generation of biosensors based on integrated circuit (IC) microchips using DNA bioreceptors designed to detect sequence-specific genetic constituents in complex samples. The design of the integrated electro-optic system on IC microchips, photodetector elements with amplifier circuitry, and their integration and use in DNA biosensor applications are discussed. The use of IC technology could lead to the development of extremely low-cost diagnostic biochips for medical applications. Measurements using the HIV 1 sequence-specific probes on the biochip device illustrate the application of the DNA biochip to the detection of a gene segment of the AIDS virus.

## 5.25.1 DEVELOPMENT OF THE INTEGRATED CIRCUIT OF THE PHOTODIODE ARRAY MICROCHIP

The biochip investigated in this example includes a large-area, $4 \times 4$ n-well integrated amplifier-photodiode array that has been designed as a single, custom integrated circuit (IC), fabricated for the biochip. This IC device is coupled to the multiarray sampling platform and is designed for monitoring very low light levels. The individual photodiodes have 900-μm square size and are arrayed on a 1-mm spacing grid. The photodiodes and the accompanying electronic circuitry were fabricated using a standard 1.2-micron n-well CMOS process. The use of this type of standard process allows the production of photodiodes and phototransistors as well as other numerous types of analog and digital circuitry in a single IC chip. The photodiodes themselves are produced using the n-well structure that is generally used to make resistors or as the body material for transistors. Since the anode of the diode is the p-type substrate material, which is common to every circuit on the IC chip, only the cathode is available for monitoring the photocurrent and the photodiode is constrained to operate with a reverse bias.

An analog multiplexer is designed to allow any of the elements in the array to be connected to an amplifier. In the final device, each photodiode could be supplied with its own amplifier. The multiplexer is made from 16 cells. Each cell has two CMOS switches that are controlled by the output of the address decoder cell. Each cell has a unique 4-bit address. One switch is open only when it is being addressed while the other switches are closed. This process connects the addressed diode to one amplifier while all the others are connected in parallel to the other amplifier.

This arrangement allows connecting a $4 \times 4$ array of light sources (different fluorescent probes, for example) to the photodiode array and reading out the signal levels sequentially. With some modification, a parallel reading system can be designed. Using a single photodiode detector would require mechanical motion to scan the source array. The additional switches and amplifier serve to correctly bias and capture the charge generated by the other photodiodes. The additional amplifier and switches allow the IC to be used as a single, large area (nearly 4 mm square) photodetector.

## 5.25.2 APPLICATION OF THE BIOCHIP TO HIV GENE FRAGMENT DETECTION

The inventors performed measurements using the biochip device with $4 \times 4$ photodiode array for detection of the HIV 1 gene. The signals from the photodiode microchip were directly recorded without the need of any electronic interface system or signal amplification device. The photocurrent of each sensing of the microchip was transmitted directly into a digital photometer or a strip chart recorder. The data from the photometer were linked to a personal computer (PC) *via* an RS-232 link. The sampling platform of the biochip contained a $4 \times 4$ array of microspots of NIR-labeled DNA on a nitrocellulose membrane.

The HIV1 gene probe is the 18-base oligonucleotide sequence complementary to the *gag* gene region. For this study, the inventors bound cDNA of specific sequences of the HIV1 virus onto the nitrocellulose membrane and hybridized them with Cy5 dye-labeled DNA strands having complementary sequences. The HIV1 measurements on the nitrocellulose was performed by spotting the H1V1 probe DNA in a $4 \times 4$ array onto the nitrocellulose sampling platform. The DNA was then crosslinked to the nitrocellulose using either a UV crosslinker or baked in vacuum at 80 degrees C for two h. With nitrocellulose membranes, the DNA solutions were spotted directly onto the membranes. The spots were subsequently subjected to either UV crosslinking using a Stratalinker (Stratagene, La Jolla, CA) or were baked in vacuum at 80°C for two h. The first step in membrane hybridization involved immobilization of single-stranded target nucleic acids to the selected surface of the probe. The probes were then treated with hybridization buffer to block nonspecific nucleic acid binding sites. The probe was introduced to samples containing labeled probe DNA and allowed to hybridize by reestablishing a double-stranded molecule with the complementary target sequences. After hybridization, excess unbound labeled probe was washed off, and the hybrid target sequences detected. In this work, the nitrocellulose membranes were prehybridized for one h at 37°C in 5 mL of 6X SSC (1X SSC=15 mM Sodium citrate, 150 mM sodium chloride pH 7.0) containing 1% BSA, 0.2% SDS (sodium dodecyl sulfate). After pre-hybridization, the hybridization probe was added to a final concentration of 100 ng/ml and hybridizations performed. After hybridization the membranes were washed in 5X SSC containing 0.1% SDS at room temperature for 10-15 min to reduce the background fluorescence levels.

Hybridization occurs between complementary DNA sequences was demonstrated by the fluorescence signals detected by the biochip. Only fluorescence signals were detected on the biochip channels where hybridization of labeled DNA HIV1 gene probes with complementary bound-DNA fragments had occurred. The 4 × 4 array signals of the 3-dimensional plot demonstrated positive hybridization with the HIVI probes used in this study. A reference system of negative blank samples (consisting of DNA probes which did not have the sequence of the HIVI gene) did not show fluorescent signals. This result illustrates the usefulness of the DNA biochip for detection of the HIV gene probe.

## 5.26 EXAMPLE 26 -- DEVELOPMENT OF A DNA BIOCHIP

### 5.26.1 PREPARATION OF OLIGONUCLEOTIDES

The inventors synthesized desired strands of oligonucleotides and labeled them with fluorescent labels (*e.g.*, fluorescein and Cy5 dyes) using procedures previously described (Vo-Dinh *et al*., 1994; Isola *et al*., 1998). All oligonucleotides were synthesized using an Expedite 8909 DNA synthesizer (Millipore, Bedford, MA). Fluorescein-labeled oligonucleotides were prepared with fluorescein CPG columns (for 3' labeling) or fluorescein phosphoramidite (for 5' labeling) using modified synthesis protocols as recommended by the manufacturers. Oligonucleotides with amino linkers were synthesized using either C3 aminolink CPG for 3' labeling or 5' amino modifier C6 (Glenn Research, Sterling, VA) for Y labeling. All oligonucleotides were synthesized using Expedite reagents (Millipore) and were deprotected and cleaved from the glass supports using ammonium hydroxide. The deprotected oligonucleotides were concentrated by evaporating the ammonium hydroxide in a Speedvac evaporator (Savant Instruments, Farmingdale, NY) and resuspended in 100 µl distilled $H_2O$. Further purification was performed by isopropanol precipitation of the DNA as follows: 10 µl of 3 M sodium acetate pH 7.0 and 110 µl isopropanol was added to 100 µl solution of DNA. The solution was then frozen at -70°C. Sodium acetate was used instead of ammonium acetate since the residual ammonium ions interfere with Cy5 linkage as well as with binding of DNA to solid supports through the amino linkers. The precipitate was collected by centrifugation at room temperature for 15 min and was washed three times with 50% isopropanol. Residual isopropanol was removed by vacuum drying in the Speedvat and the DNA resuspended in sterile distilled water at a final concentration of 10 µg µl$^{-1}$. These stock solutions were diluted in the appropriate buffer at a 1:10 dilution to give a DNA concentration of 1 µg µl$^{-1}$.

For labeling DNA with Cy5 dye (Amersham Life Sciences, Arlington Heights, IL), modified oligonucleotides containing alkyl amino groups were derivatized as follows (Vo-Dinh *et al*., 1994) 30 pmol of the DNA was dissolved in 250 µl 0.5 M sodium chloride and passed through a Sephadex G10 (1 cm diameter, 10 cm long) (Pharmacia, Piscataway, NJ) column equilibrated with 5 mM borate buffer (pH 8.0). The void volume containing the oligonucleotide was collected and concentrated by evaporation. This was dissolved in 100 µl 0.1 M carbonate buffer (pH 9.0). Cy5 (1 mg in carbonate buffer) was added to the oligonucleotide and the conjugation reaction was performed at room temperature for 60 min with occasional mixing. The conjugated oligonucleotide was separated from the free dye using a Sephadex G10 column as described above. The fractions containing the labeled DNA were collected and concentrated using a Speedvac evaporator.

### 5.26.2 RESULTS

### 5.26.2.1 DEVELOPMENT OF BIOCHIP INTEGRATED CIRCUITS

An important element in the development of the biochip involves the design and development of an IC electro-optic system for the microchip detection elements using the CMOS technology. With this technology, highly integrated biosensors are made possible partly through the capability of fabricating multiple optical sensing elements and microelectronics on a single IC. A two-dimensional array of optical detector amplifiers was integrated on a single IC chip. Such an integrated microchip system is not currently available commercially.

Two exemplary biochip IC systems based on photodiode circuitry were constructed, one system having 16 channels (4 × 4 array), and the other having 100 channels (10 × 10 array). The biochips include a large-area, *n*-well integrated amplifier-photodiode array that has been designed as a single, custom integrated circuit (IC), fabricated for the biochip. This IC device is coupled to the multiarray sampling platform and is designed for monitoring very low light levels. The individual photodiodes have 900 µm square size and are arrayed on a 1 mm spacing grid. The photodiodes and the accompanying electronic circuitry were fabricated using a standard 1.2 µ n-well CMOS process. The use of this type of standard process allows the production of photodiodes and phototransistors as well as other numerous types of analog and digital circuitry in a single IC chip. This feature is the main advantage of the CMOS technology in comparison to other detector technologies such as charge-coupled devices or charge-injection devices. The photodiodes themselves are produced using the *n*-well structure that is generally used to make resistors or as the body material for transistors. Since the anode of the diode is the p-type substrate material, which is common to every circuit on the IC chip, only the cathode is available for monitoring the photocurrent and the photodiode is constrained to operate with a reverse bias.

An analog multiplexer was designed that allows any of the elements in the array to be connected to an amplifier. In the final device, each photodiode could be supplied with its own amplifier. The multiplexer is made from 16 cells for the 4 × 4 array device. Each cell has two CMOS switches that are controlled by the output of the address decoder cell. Each cell has a unique 4-bit address. One switch is open only when it is being addressed while the other switches are closed. This process connects the addressed diode to one amplifier while all the others are connected in parallel to the other amplifier.

This arrangement allows connecting a 4 × 4 (or 10 × 10) array of light sources (different fluorescent probes, for example) to the photodiode array and reading out the signal levels sequentially. With some modification, a parallel reading system can be designed. Using a single photodiode detector would require mechanical motion to scan the source array. The additional switches and amplifier serve to correctly bias and capture the charge generated by the other photodiodes. The additional amplifier and switches allow the IC to be used as a single, large area (nearly 4 mm square) photodetector.

## 5.26.3 ILLUSTRATIVE APPLICATIONS INVOLVING BIOCHIP DEVICES

The human immunodeficiency virus (HIV) *gag* gene sequence was selected as a model template to evaluate the biochip sensing system. Infection with the human immunodeficiency virus Type I (HIV 1) results in a uniformly fatal disease. Unfortunately standard HIV serologic tests, including the enzyme-linked immunosorbent assay and the Western blot assay, are not useful in the diagnosis of HIV infection during early infancy because of the confounding presence of transplacentally derived maternal antibody in the infants blood. There is a need for a direct nucleic acid based test which detects the presence of HIV viral sequences. The inventors have utilized synthetic DNA templates (Isola *et al*., 1998) from the *gag* gene region of HIV 1 as a model system to illustrate the application of the biochip system for HIV gene detection.

The biochip device with 4 × 4 photodiode array was evaluated for the detection of HIV 1 gene fragments. The signals from the photodiode microchip were directly recorded without the need of any electronic interface system or signal amplification device. The photocurrent of each sensing of the microchip was transmitted directly into a digital photometer or a strip chart recorder. The data from the photometer were linked to a personal computer (PC) *via* an RS-232 link. The sampling platform of the biochip contained a 4 × 4 array of microspots of NIR-labeled DNA on a nitrocellulose membrane.

To illustrate the usefulness of the biochip in hybridization applications, an 18-base oligonucleotide sequence complementary to the *gag* gene was used region-of the HIV 1 gene (Isola *et al*., 1998). The inventors used various probe sequences selected in this region to design DNA probes for hybridization and detection using a novel technique based on surface-enhanced Raman scattering (Isola *et al*., 1998). For this study, the inventors have bound cDNA of specific sequences of the HIV I virus onto the nitrocellulose membrane used as the sampling platform, and hybridized them with Cy5 dye-labeled DNA strands having complementary sequences. The HIV 1 measurements on the nitrocellulose was performed by binding the HIV 1 probe DNA in a 4 × 4 array onto the nitrocellulose sampling platform. The DNA was then crosslinked to the nitrocellulose using either a UV crosslinker or baked in vacuum at 80°C for 2 h. The spots were subsequently subjected to either UV crosslinking using a Stratalinker (Stratagene, La Jolla, CA) or were baked in vacuum at 80°C for 2 h. The first step in membrane hybridization involved immobilization of single-stranded target nucleic acids to the selected surface of the probe. The probes were then treated with hybridization buffer to block nonspecific nucleic acid binding sites. The probe was introduced to samples containing labeled probe DNA and allowed to hybridize by reestablishing a double-stranded molecule with the complementary target sequences. After hybridization, excess unbound labeled probe was washed off, and the hybrid target sequences detected. In this work, the nitrocellulose membranes were prehybridized for 1 h at 37°C in 5 ml of 6 x SSC (1X SSC = 15 mM sodium titrate, 150 mM sodium chloride pH 7.0) containing 1% BSA, 0.2% SDS (sodium dodecyl sulfate). After pre-hybridization, the hybridization probe was added to a final concentration of 100 ng ml$^{-1}$ and hybridizations performed. Following hybridization under appropriate conditions, the membranes were washed in 5X SSC containing 0.1% SDS at room temperature for 10-15 min to reduce the background fluorescence levels.

Hybridization occurs between complementary DNA sequences was demonstrated by the fluorescence signals detected by the biochip. Data showed that fluorescence signals higher than the background were detected on the biochip channels where hybridization of labeled DNA HIV 1 gene probes with complementary bound-DNA fragments had occurred (FIG. 33). The 4 × 4 array signals of the 3-dimensional plot demonstrated positive hybridization with the HIV 1 probes used in this study. A reference system of negative blank samples (consisting of DNA probes which did not have the sequence of the HIV 1 gene) showed only weak background fluorescent signals. This result is an illustration the usefulness of the DNA biochip for detection of a specific HIV gene sequence. Detection of the HIV virus itself may also require simultaneous detection of multiple gene sequence regions of the virus. It has been observed that progression of the AIDS disease causes increase in the genotype diversity in HIV viruses. It has been reported that the HIV viruses appear to defeat the immune system by producing and accumulating these gene mutations as the disease progresses (Wolinsdy and Korber, 1996). In addition to the exemplary sequences used in this example, other sequence fragments of the HIV viruses could be used to detect particular genes or regions of the HIV genome.

Likewise, the polynucleotide probes used for hybridization applications of the biochip apparatus need not be necessarily DNA probes, nor do the particular polynucleotide targets to be detected need necessarily be DNA molecules. Because DNA:DNA, DNA:RNA, DNA:PNA, RNA:RNA, RNA:PNA, and PNA:PNA duplexes may be formed under the appropriate hybridization conditions and depending upon the degree of homology of the two strands, the inventors contemplate that a variety of hybridization biochips may be designed which will utilize either RNAs, DNAs, or PNAs in the detection of other homologous polynucleotide molecules. The preparation, synthesis, and use of ribozymes, RNAs, and PNAs as polynucleotide probes is described herein in Section 4.

As discussed, it is not necessary that bioprobes may be employed that are limited only to nucleic acids. Clearly there are numerous probes that could be considered for immobilization on the disclosed biochip. For example, Erdeniz *et al.* (1997) report cloning-free PCR™ based allele replacement methods. Allele transfer between yeast strains is described in which the desired allele is amplified by PCR™ with a pair of adaptamers. Adaptamers are chimeric oligonucleotides that used to amplify the selected allele and differentially tag its 5' and 3' ends. Such adaptamers depending upon the desired amplification, could be attached to the biochip and employed to track the amplification reaction.

Molecular imprinting is a technique used for creating selective recognition sites in synthetic polymers. A template molecule used in polymerization reactions of selected monomers. Many polymers obtained by this method show stereo and regio-specific selectivity, thus, enabling chiral separation of bioactive molecules, Mosbach (1994). It is contemplated that selected imprinted polymers could be designed to include one or more detectable labels that optionally may change their emission signal upon binding of a cognate molecule.

Cyclodextrins are well known for their utility in delivery of certain drugs particularly peptide and protein drugs. The cyclodextrins are also known for their ability to incorporate numerous types of compounds as guest molecules. Modified cyclodextrins such as those obtained by polymerization of ethylene oxide around a core of cyclodextrin have been employed as drug delivery systems. Polyethylene oxide modified cyclodextrins have been described by Topchieva *et al* (1998) as a new family of bouquet-like molecules with properties similar to the course cyclodextrins. Appropriately tagged cyclodextrins or modified cyclodextrins are also contemplated as having utility as bioprobes useful in connection with the disclosed biochip.

Lipiphilic polyamide dendrimers comprise another class of symmetrical macromolecules that are though to be potential drug carriers, Sakthivel *et al.* (1998). Because of their lipifilic properties, they have been investigated as gene transfer agents. Qin *et al.* (1998) have reported the use of Starburst dendrimers to increase plasmid-mediated gene transfer and efficiency. Polyamidoamine subunits assembled into Starburst dendrimers were used to augment plasmid-mediated gene transfer. Starburst dendrimers therefore may be useful in monitoring efficiency of gene transfer *in vivo* monitoring prolongation of allograft survival or other events in vivo where the detection of labeled tracers in biological fluids might be desirable. In addition to Starburst dendrimers, radio labeled dendrimers have been described which are macromolecular T2 contrast agents. Tagged dendrimers could be detected by choosing the appropriate bioprobe on the chip to pick up these agents and presence detected by measuring radioactivity. Bulte, *et al* (1998) have described Dysprosium-DOTA-PAMAM dendrimers which illustrate a particular example of AT2 contrast agent. Additional probes include biotentilated Starburst dendrimers might be used in antibody pretargeting as described by Wilbur *et al.* (1998).

Moreover, in addition to the illustrative embodiments described herein, the inventors contemplate the use of the biochip devices in the detection, quantitation, or identification of a variety of organisms and macromolecules of biological or medical interest. For example, the chips may be used to detect or quantitate or identify pathogens of medical interest. Such pathogens may be viral (such as EBV, HIV, FIV, parvoviruses, retroviruses, and the like), rickettsial, fungal (including organisms such as Candida and other yeast genera, dermatomycotic fungi (including *Epidermophyton* spp., *Microsporum* spp., and *Trichophyton* spp., and the like), systemic fungi (including *Blastomyces*, *Histoplasma, Coccidioides*, *Cryptococcus, Geotrichum, Histoplasma, Sporotrichum,* and the like); bacterial (such as *Mycobacteria* including *Mycobacterium tuberculosis*, *Vibrio* including *V. cholerae; Salmonella,* including S. *choleraesius, S. paratyphi, S. schottmulleri,* and *S. typhimurium; Treponema,* including *T. pallidum; Shigella,* including *S. dysenteriae* and *S. flexneri*, *Serratia,* including *S. marcescens; Yersinia,* including *Y. pestis* and *Y. pseudotuberculosis; Proteus,* including *P. mirabilis, Morganella,* including *M. morganii; Streptococcus,* including *S. faecalis, S. pneumoniae, S. salivarius, S. pyogenes,* and S. *sanguis; Staphylococcus,* including *S. aureus; Bacillus,* including B. *anthracis, B. coagulans, B. pasteurii, B. cereus* and *B. subtilis,* or other disease-causing bacteria including those of the genera *Leptospira, Clostridium, Neisseria, Brucella, Francisella,, Hemophilus, Corynebacterium,* and the like); or eukaryotic microorganisms such as *Giardia, including G. lamblia and G. intestinalis; Chlamydia,* including *C. psittaci and C. trachomatis,* and the like.

Alternatively, the detection of particular genes, such as the *p53* cancer gene (Vo-Dinh *et al.,* 1998), could also be performed using a single biochip or a plurality of individual biochips.

Another area of application of biochip technology is in functional genomics analysis. Provided that a reasonable degree of base complementarity exists, two nucleic acid strands from different sources (DNA and RNA) will undergo molecular hybridization under the proper conditions. This feature allows the biochip technology to be used also in the analysis of gene expression and function. Furthermore, the biochip can be also used to monitor the extent of genetic variation between individuals and their susceptibility to diseases.

The DNA biochip system offers a unique combination of performance capabilities and analytical features of merit not available in any other DNA analysis system currently available. With its multichannel capability, the DNA biochip technology described herein is the first system described to date based on an optical sensing microchip system having integrated an signal amplifier and data treatment on-board. The DNA biochip device allows simultaneous detection of multiple DNA targets simultaneously. The present DNA biochip offers several advantages in size, performance, fabrication, analysis and production cost due to its integrated optical sensing microchip. The small sizes of the probes (microliter to nanoliter) minimize sample requirement and reduce reagent and waste requirement. Highly integrated systems lead to a reduction in noise and an increase in signal due to the improved efficiency of sample collection and the reduction of interfaces. The capability of large-scale production using low-cost IC technology is an important advantage. The assembly process of various components is simplified by integration of several elements on a single chip. For medical applications, this cost advantage will allow the development of extremely low cost, disposable biochips that can be used for in-home medical diagnostics of diseases without the need of sending samples to a laboratory for analysis.

## 5.27 EXAMPLE 27 -- DNA BIOCHIPS USING A PHOTOTRANSISTOR IC

Recently, there is an increasing interest in the development of DNA bioreceptor-based analytical systems (Vo-Dinh *et al.,* 1994; Isola *et al.,* 1996; Schena *et al.,* 1995; Piunno *et al.,* 1995; Kumar *et al.,* 1994; Eggers *et al.,* 1994). An important area in biological monitoring is the sensitive diagnosis of diseases, biological species or living systems (bacteria, virus or related components) at ultratrace levels in biological samples (*e.g.*, tissues, blood and other bodily fluids) and environmental samples (*e.g.*, air, soil and water samples). To detect a compound in a "real life" sample, a biosensor must be able to recognize and differentiate various biochemical constituents of these systems in order to provide unambiguous identification and accurate quantification. Living systems possess exquisite recognition elements (*e.g.,* antibody, enzyme, gene probes, *etc.*), often referred to as bioreceptors, which allow specific identification and detection of complex chemical and biological species

This example describes a biosensor based on integrated circuit (IC) microchips using DNA bioreceptors designed to detect genetic constituents in complex samples of biomedical and environmental interest. In this system, phototransistors were used which are devices that can be miniaturized and fabricated on an integrated circuit. A phototransistor was developed that was composed of 220 phototransistor cells connected in parallel. This miniaturization technology is useful not only for the DNA probe substrates described here, but also for the integrated detector system that could be employed to produce microchip biosensor devices, *i.e.*, "biosensor-on-a-chip". The device has sensors, amplifiers, discriminators and logic circuitry on board. These highly integrated biosensors were produced using the capability of fabricating multiple optical sensing elements and microelectronics on a single IC.

Biosensors combine two important concepts that integrate "biological recognition" and "sensing". The basic principle of an optical biosensor is to detect this molecular recognition and to transform it into an optical signal using a transducer. As illustrated schematically in FIG. 1, the DNA biochip involves the combination of integrated circuit elements, electro-optics excitation/detection system, and DNA-based bioreceptor probes into a self-contained and integrated microdevice. A basic DNA biochip includes: 1) excitation light source with related optics, 2) a bioprobe, 3) a sampling element with sample platform and delivery system, 4) an optical detector with associated optics and dispersive device, and 5) a signal amplification/treatment system.

Construction of a DNA biochip involves integration of several basic elements of very different natures. The basic steps include: a) selection or development of the bioreceptor, b) selection of the excitation source, c) selection or development of the transducer, and d) integration of the excitation source-bioreceptor-transducer system.

The development of the DNA biochip in this example comprises three major elements. The first element involves the development of a bioreceptor probe system: a microarray of DNA probes on nitrocellulose sampling platform. The second element is focused on the development of non-radioactive methods for optical detection: the fluorescence technique. The third element involves the development of an integrated electro-optic IC system on a single chip for biosensing: phototransistor-amplifier microchip technology.

The design of the gene probe immobilization techniques on the biosensor substrates as well as the development of integrated electro-optic systems on IC biochips using a phototransistor multiarray system was demonstrated. Measurements of fluorescent-labeled DNA probes and hybridization studies with a HIV1 sequence-specific probe on the nitrocellulose sampling platform system illustrate the analytical figures of merit of this exemplary biochip device.

**5.27.1 EXPERIMENTAL AND MATERIALS**

**5.27.1.1 DEVELOPMENT OF OLIGONUCLEOTIDES**

Strands of oligonucleotides were synthesized and labeled with fluorescent labels (*e.g.*, fluorescein and Cy5 dyes). Oligonucleotides were synthesized using an Expedite 8909 DNA synthesizer (Millipore, Bedford, MA). Fluorescein-labeled oligonucleotides were prepared with fluorescein CPG columns (for 3' labeling) or fluorescein phosphoramidite (for 5' labeling) using modified synthesis protocols as recommended by the manufacturers. Oligonucleotides with amino linkers were synthesized using either $C_3$ aminolink CPG for 3' labeling or 5' amino modifier $C_6$ (Glenn Research, Sterling, VA) for 5' labeling. All oligonucleotides were synthesized using Expedite reagents (Millipore) and were deprotected and cleaved from the glass supports using ammonium hydroxide. The deprotected oligonucleotides were concentrated by evaporating the ammonium hydroxide in a Speedvac evaporator (Savant, Farmington, NY) and resuspended in 100 µL distilled $H_2O$. Further purification was performed by isopropanol precipitation of the DNA as follows: 10 µL of 3 M sodium acetate pH 7.0 and 110 µL isopropanol was added to 100 µL solution of DNA. The solution was then frozen at -70°C. Sodium acetate was used instead of ammonium acetate since the residual ammonium ions interfere with Cy3 and Cy5 linkage as well as with binding of DNA to solid supports through the amino linkers. The precipitate was collected by centrifugation at room temperature for 15 min and was washed 3 times with 50% isopropanol. Residual isopropanol was removed by vacuum drying in the Speedvac and the DNA resuspended in sterile distilled water at a final concentration of 10 µg/µL. These stock solutions were diluted in the appropriate buffer at a 1: 10 dilution to give a DNA concentration of 1 µg/µL.

For labeling DNA with Cy5 dye (Amersham Life Sciences, Arlington Heights, IL), modified oligonucleotides containing alkyl amino groups were derivatized as follows: 30 pmoles of the DNA was dissolved in 250 µL 0.5 M sodium chloride and passed through a Sephadex G10 (1 cm diameter, 10 cm long) (Pharmacia, Piscataway, NJ) column equilibrated with 5 mM borate buffer (pH = 8.0). The void volume containing the oligonucleotide was collected and concentrated by evaporation. This was dissolved in 100 µL 0.1 M carbonate buffer (pH = 9.0). Cy5 (1 mg in carbonate buffer) was added to the oligonucleotide and the conjugation reaction was performed at room temperature for 60 min with occasional mixing. The conjugated oligonucleotide was separated from the free dye using a Sephadex G10 column as described above. The fractions containing the labeled DNA were collected and concentrated using a Speedvac evaporator.

**5.27.1.2 IMMOBILIZATION OF DNA PROBES AND THE SAMPLING SUBSTRATES**

Biologically active DNA probes can be directly or indirectly immobilized onto a transducer detection surface to ensure optimal contact and maximum detection. When immobilized onto a substrate, the gene probes are stabilized and, therefore, can be reused repetitively. In one procedure, hybridization is performed on an immobilized target or a probe molecule attached on a solid surface such as nitrocellulose, or a nylon membrane, or a glass plate. Several methods can be employed to bind DNA to different supports. One method commonly used for binding DNA to glass involves silanization of the glass surface followed by activation with carbodiimide or glutaraldehyde.

Another approach consists of immobilizing the gene probe onto a membrane and subsequently attaching the membrane to the transducer detection surface. This approach has the advantage of avoiding a need to binding the bioreceptor onto the transducer. Several types of membranes are available for DNA binding: nitrocellulose, charge-modified nylon, *etc.* The gene probe was bound to the membrane using ultraviolet activation. With nitrocellulose membranes, the DNA solutions were spotted directly onto the membranes. The spots were subsequently subjected to either UV crosslinking using a Stratalinker (Stratagene, La Jolla, CA) or were baked in vacuum at 80°C for two hours.

**5.27.1.3 HYBRIDIZATION ON NITROCELLULOSE-BASED SAMPLING PLATFORM**

The first step in membrane hybridization involved immobilization of single-stranded DNA (ss-DNA) onto the selected surface of the sensor probe (*e.g.*, membrane or surface). The probes were then treated with hybridization buffer to block nonspecific nucleic acid binding sites. Samples containing fluorescent-labeled DNA were delivered to the sensor probe and allowed to hybridize by reestablishing a double-stranded molecule with the complementary target sequences. After hybridization, excess unbound labeled DNA was washed off, and the hybrid target sequences detected. In this work, the nitrocellulose membranes were pre-hybridized for one hour at 37°C in 5 mL of 6X SSC (1X SSC = 15 mM Sodium citrate, 150 mM sodium chloride pH 7.0) containing 1% BSA and 0.2% SDS (sodium dodecyl sulfate). After pre-hybridization, the hybridization probe was added to a final concentration of 100 ng/ml and hybridizations performed for 16 h. After hybridization the membranes were washed in 5X SSC containing 0.1% SDS at room temperature for 10-15 min and at higher temperatures if necessary to reduce the background fluorescence levels.

## 5.27.1.4 INSTRUMENTATION SYSTEM OF THE MICROARRAY PROBES

In addition to the integrated phototransistor IC device described in this example, an instrumental system was developed to evaluate the performance of the gene probes. A two-dimensional multiarray detection system using a charge-coupled device (CCD) was used with the initial system. The CCD experimental set-up consisted of a laser source, optical filters, lenses, the multiprobe waveguide and the CCD detector. For this evaluation, several lasers were used: a krypton ion laser (647.1 nm) was used for the Cy5 dye-labeled DNA probe, and an argon ion laser with the 514-nm line was used for a visible fluorescein-labeled DNA probe. The laser beam was focused into a 600 µm diameter fiber and transmitted to the sample substrate. The laser radiation was passed through a laser bandpass filter to remove unwanted laser lines or background fluorescence emission from the optical fiber. The fluorescence was passed through a Raman holographic filter to remove any remaining laser light, and was focused with a 1:2 50-mm lens and 2× macro focusing teleconverter onto the surface of a CCD detector. A 514.5-nm Raman holographic filter (Kaiser) was used to pass the fluorescence and block the laser light. for fluorescence measurements. A 670-nm longpass filter was used to block laser excitation and pass the Cy5 fluorescence. Several CCD systems were evaluated including those from: Photometrics, Ltd. (*e.g.*, Model PM-512) (Tucson, AZ), Princeton Instrument, (*e.g.*, Model RE-ICCD) (Princeton, NJ) and Santa Barbara Instruments Group (*e.g.*, Model ST-6) (Santa Barbara, CA).

## 5.27.2 RESULTS

## 5.27.2.1 DEVELOPMENT AND EVALUATION OF DNA MICROARRAYS

Extensive measurements were performed to evaluate the process of developing microarrays of DNA probes. A nitrocellulose membrane was used as the substrate for its ease in handling and its efficiency to bind DNA. Multiarrays of sample spots were produced using the device illustrated in FIG.31. Liquid solutions of ss-DNA sequences were dispensed onto the nitrocellulose (Zeta Probe®, Bio Rad, Hercules, CA) using a pV 830 pneumatic Picopump™ (World Precision Instruments, Sarasota, FL). The picopump™ was capable of producing regular microspots with diameter size range of 500-800 µm, which can be selected to match the size of detector elements. The DNA sample was loaded into a small diameter glass capillary, which was held a few mm above the cellulose membrane. The membrane was held in place on a vacuum flask fitted with a metal mesh frit. The vacuum procedure served two important purposes. First, the vacuum applied to the membrane improves the reproducibility of the spotting by maintaining the membrane flat against the metal mesh surface. The vacuum procedure also had a shortening effect on the sample drying process, which decreased the sample spot size by preventing the spread of sample though the membrane.

The Photometrics CCD (model CH 210) detection system was used to evaluate the DNA sample arrays since this system provides a 2-dimensional image of the probe array (Picard and Vo-Dinh, 1991). The results showed the image obtained using the CCD system of fluorescein-labeled DNA samples designed as a 4 × 4 array onto the nitrocellulose membrane. The sample was illuminated with the 514.5-nm line of the argon laser. A Kaiser 514.5-nm holographic filter was used to block the laser excitation from the CCD. The results illustrated the efficiency of the Picopump™ spotting system developed to produce DNA arrays on the nitrocellulose sampling platform. The diameter of the sample spot can be regulated by varying the sampling volumes, and can be reproduced with 5-10 % relative standard deviation. The amount of sample can be estimated by the intensity of the spot. For example, one spot on the array (location 1: 3) indicated a higher amount of fluorescent materials than all the other sample spot arrays.

The process of gene probe sensing using both visible and NIR (700-1000 nm spectral range) fluorescent dye labels was also evaluated. Excitation and detection in the NIR range present certain difficulties but also offer several advantages. Measurements in the NIR are less interfered by background fluorescence of the nitrocellulose substrate since very few species with NIR emission occur. An important advantage associated with NIR measurements is the availability of low-cost, miniaturized diode lasers which usually have emission lines in the red and NIR regions. The DNA probes were labeled with the Cy5 NIR dye following the procedure described above. The diode laser line of 780 nm at 9.5 mW was used for excitation. The calibration curve for the NIR dye-labeled single-stranded DNA over a concentration range from 1 pmol/µL to 3 fmol/µL. Excitation light was provided by a pen-size 9.5-mW laser diode (780 nm) and measurement times ranged from 1 to 3 min. This calibration curve was linear over the full range of the dye concentration investigated. The limit of optical detection, based on a signal equal to three times the standard deviation of the noise, was estimated to be in the 100 attomole/µL.

Hybridization studies were conducted to demonstrate the usefulness of the DNA array probes on the nitrocellulose sampling platform. The DNA probe's role is to identify the target compounds *via* molecular recognition. The operation of gene probes is based on the hybridization process. Hybridization involves the joining of a single strand of nucleic acid with a homologous complementary probe sequence. Hybridization of a nucleic acid probe to DNA biotargets (*e.g.*, gene sequences, bacteria, viral DNA) offers a very high degree of accuracy for identifying DNA sequences complementary to that of the probe. Nucleic acids strands tend to be paired to their complements in the corresponding double-stranded structure. Therefore, a single-stranded DNA molecule will seek out its complement in a complex mixture of DNA containing large numbers of other nucleic acid molecules. Hence, nucleic acid probe (*i.e.* gene probe) detection methods are very specific to DNA sequences. cDNAs of specific sequences of the HIV1 virus were bound onto the nitrocellulose membrane and hybridized them with Cy5-labeled DNA strands having complementary sequences. The HIV1 measurements on the nitrocellulose was performed by spotting the HIV1 probe DNA onto the nitrocellulose. The HIV1 gene probe is the 18-base oligonucleotide sequence complementary to the *gag* gene region (Clewley, 1989). Hybridization occurrence between complementary DNA sequences was demonstrated by positive detection of the fluorescence signals. The imaging system used the Photometrics CCD. The data showed the fluorescence signal obtained with the CCD detector resulting from the hybridization of HIV 1 labeled gene probe with complementary DNA bound on the nitrocellulose membrane. The $3 \times 3$ array signal of the 3-dimensional plot demonstrated positive hybridization with the HIVI probe. A reference system consisting of an array of control DNA probes, which do not have the sequence of the HIVI gene, did not exhibit fluorescent signals. This result illustrates the capability of the nitrocellulose membrane as the sampling platform for *in-situ* hybridization studies on the DNA biochip.

## 5.27.2.2 DEVELOPMENT AND EVALUATION OF THE PHOTOTRANSISTOR

## INTEGRATED CIRCUIT SYSTEM

An important element in the development of the biochip involves the design and development of an IC electro-optic system for the microchip detection elements using the phototransistor-amplifier technology. With this technology, highly integrated biosensors are made possible partly through the capability of fabricating multiple optical sensing elements and microelectronics on a single IC. A two-dimensional array of optical detector-amplifiers was integrated on a single IC chip. Each optical detector was a phototransistor coupled to a transimpedence amplifier followed by an amplifier. This block was repeated several times on the IC chip and combined with other electronic elements such as filters and amplifiers, which were also integrated on the same IC. The operational amplifier used with the phototransistor is a two-stage, unbuffered amplifier. The circuit is very compact, occupying an area of only 185 $\mu$m $\times$ 200 $\mu$m. It was designed to be useful for wide-band amplification and low-level signals. The gain-bandwidth product is 70 MHz and the amplifier is stable for gains greater than 10. Other typical characteristics include: input offset voltage less than 5 mV, dc gain of 220, positive slew rate of 80 V/$\mu$s and negative slew rate of 9 V/$\mu$s. The circuit requires 2.5 mW from a single 5-V supply. This IC chip performed the complete conversion from an optical signal to an electrical signal suitable for data digitization and capture by a computer.

The circuit was fabricated in a 2-$\mu$m, *p*-well complementary metal oxide semiconductor (CMOS) process and occupied an area of 160,000 square microns. Detector elements using single phototransistors are not sufficiently sensitive for trace detection. Therefore, the phototransistor was actually composed of 220 phototransistor cells connected in parallel. An individual phototransistor cell occupied 760 square microns. The transimpedence amplifier had a gain of 100 kV/A. As the phototransistors were coupled with the 10-fold amplifier gain, the resulting gain was $10^6$ V/A. The phototransistors had a conversion gain on the order of 10 $\mu$A/$\mu$W, so the entire chain had an approximate conversion gain of 10 V/$\mu$W. The exact gain generally depends on the spectral region of interest and, to some extent, on the signal level being monitored.

The above described elements may be modified to tailor the devices to specific applications. Since the phototransistor is made from basic photocell elements, it can be connected to as many cells as needed to create the desired geometry or required number of channels to adapt the detector to a specific application. Similarly, the gain and bandwidth of the amplifiers can be adjusted using simple resistor or capacitor changes as the application requires.

The microchips designed and fabricated were evaluated by measuring the fluorescence signal of a fluorescein-labeled DNA sample spotted onto the membrane probe. FIG. 20 shows the performance of the integrated phototransistor and amplifier circuit (device No. ICI N551-CD2). The figure shows the signal output response for various concentration of the fluorescein-labeled DNA excited with the Argon ion laser. The results illustrate the linearity of the microchip detector with respect to the fluorescein-labeled DNA concentrations and demonstrate the possibility for quantitative analysis.

## 5.27.2.3 EVALUATION OF THE BIOCHIP USING FLUORESCENT-LABELED DNA PROBES

In this example the inventors developed and performed measurements using an amplifier-phototransistor (APT) device with $4 \times 4$ array of phototransistors. Whereas the CCD instrument was used to obtain an image of the microarray sample spots and to record a plot of the intensity, the signals from the APT microchip were directly recorded without the need of any electronic interface system or signal amplification device. The photocurrent of each sensing element of the APT microchip was transmitted directly into a digital photometer. The data from the photometer were linked to a personal computer (PC) *via* an RS-232 link. The samples consisted of an array of microspots of fluorescein-labeled DNA on a nitrocellulose membrane. In the study of the probe arrays on nitrocellulose membranes described in previous sections, the inventors used the CCD system to obtain a two-dimensional image of the fluorescence signals. Since the sensing elements of the phototransistor chip provide only a signal intensity and not a signal profile, the inventors devised a device to record the response of the phototransistor detection elements as the sample spots were passed over the sensing elements. In this study, a nitrocellulose membrane having a series of fluorescein-labeled DNA sample spots was placed on a glass slide connected to a linear translation stage. The measurements were made while the translation stage moved the sample arrays over the stationary phototransistor device. The measurement system is schematically depicted in FIG. 18. Light from an argon ion laser at 488 nm was transmitted *via* an optical fiber and focused onto a sample spot. An appropriate optical filter placed between the sample substrate and the detector array was used to reject the laser radiation. The data showed four resolved signals as the sample spots moved over an APT microchip channel (FIG. 19). Four sample spots of 1-μL of fluorescein-labeled DNA were placed on a nitrocellulose membrane, which was translated over a detection channel of an APT biochip. As the DNA spot passed over the photodetector, a fluorescence signal is detected. The four peaks in FIG. 19 illustrates the detection of the four resolved DNA sample spots on the substrate by the APT biochip device.

## 6.0 REFERENCES

Affymetrix http://www.affymetrix.com; July 23, 1997.

Alarie, Stokes, Sutherland, Edwards, Vo-Dinh, "Intensified charge coupled device-based fiber-optic monitor for rapid remote surface-enhanced Raman scattering sensing, *Appl. Spectrose*., 46:1608-1612, 1992.

Anderson, McGall, Lipshutz, "Polynucleotide arrays for genetic sequence analysis," *In: Microsystem Technology in Chemistry and Life Science,* A. Manz, H. Becket (Eds.), Springer-Verlag, Berlin, pp 117-129, 1998.

Armitage *et al*., *Proc. Natl. Acad Sci. USA*, 94(23):12320-12325, 1997.

Aubert, Oguey, Vuillcumier, "Monolithic Optical Position Encoder with On-Chip Photodiodes," *IEEE J. Solid State Circuits,* 23(2):465-73, 1988.

Boffa, Carpaneto, Allfrey, *Proc. Natl. Acad Sci. USA,* 92:1901-1905, 1995.

Boffa. Morris, Carpaneto, Louissaint, Allfcey, *J. Biol. Chem*., 271:13228-13233, 1996.

Bulte, C. Wu, M. W. Brechbiel, R.A. Brooks, J. Vymazal, M. Holla and J.A. Frank, "Dysprosium-DOTA-PAMAM dendrimers as macromolecular T2 contrast agents. Preparation and relaxometry", *Invest Radio,* 33(11):841-5, 1998.

Campbell, *In: Monoclonal Antibody Technology, Laboratory Techniques in Biochemistry and Molecular Biology,* Burden and Von Knippenberg, Eds., Vol. 13, pp. 75-83, Elsevier, Amsterdam, 1984.

Carlsson *et al., Nature,* 380:207, 1996.

Chen *et al., Nucl. Acids Res.*, 20:4581-4589, 1992.

Chowrira and Burke, *Nucl. Acids Res.,* 20:2835-2840, 1992.

Christensen *et al.*, *J. Pept. Sci.,* 1(3):175-183, 1995.

Clewley, *J. Virol. Methods,* 25:179-194,1989.

Collins and Olive, *Biochem.*, 32:2795-2799, 1993.

Corey, *Trends Biotechnol.,* 15(6):224-229, 1997.

Dropulic *et al*., *J. Virol*., 66:1432-41, 1992.

Dueholm *et al., J. Org. Chem.,* 59:5767-5773, 1994.

Eggars, Hogan, Reich, Lamture, Ehrlich, Hollis, Kosicki, Powdrill, Beattie, Smith, Varma, Gangadharam, Mallik, Burke, Wallace, "A microchip for quantitative detection of molecules utilizing luminescent and radioisotope reporter groups," *Biotechniques,* 17:516-523, 1994.

Egholm *et al., Nature,* 365:566-568, 1993.

Elroy-Stein and Moss, *Proc. Natl. Acad. Sci. USA,* 87:6743-7, 1990.

Erdeniz, U.H. Mortensen and R. Rothstein, "Cloning-free PCR-based allele replacement methods", *Genome Res,* 7(12): 1174-83, 1997.

Fodor, Read, Pirrung, Stryer, Lu, Solas, "Light directed. spatially addressable parallel chemical synthesis," *Science,* 251:767-769, 1991.

Footer, Engholm, Kron, Coull, Matsudaira, *Biochemistry,* 35:10673-10679, 1996.

Frohman, M.A., In "PCR Protocols: A Guide to Methods and Applications," Academic Press, New York, 1990.

Gambacorti-Passerini *et al*., Blood, 88:1411-1417, 1996.

Gao and Huang, *Nucl. Acids Res.,* 21:2867-2872, 1993.

Gefter, Margulies, Scharff, "A simple method for polyethylene glycol-promoted hybridization of mouse myeloma cells," *Somat. Cell Genet.,* 3(2):231-236, 1977.

Geiger, Allen, Strader, *In: VLSI Design Techniques for Analog and Digital Circuits,* McGraw-Hill Publishing Co., New York, 1990.

Goding, "Monoclonal Antibodies: Principles and Practice," pp. 60-74. 2nd Edition, Academic Press, Orlando, FL, 1986.

Good and Nielsen, *Antisense Nucleic Acid Drug Dev.,* 7(4):431-437, 1997.

Graham, Leslie, Squirrell, "Gene probe assay on a fibre-optic evanescent wave biosensor," *Biosensors Bioelectronics,* 7:487-493, 1992.

Griffith *et al.*, *J. Am. Chem. Soc.,* 117:831-832, 1995.

Guerrier-Takada *et al.*, *Cell,* 35:849, 1983.

Haaima, Lohse, Buchardt, Nielsen, *Angew. Chem.*, *Int. Ed. Engl.,* 35:1939-1942, 1996.

Hacia, Brody, Chee, Fodor, Collins, *Nature Genetics,* 14:441-447, 1996.

Hampel and Tritz, *Biochem.,* 28:4929, 1989.

Hampel *et al.*, *Nucl. Acids Res.*, 18:299, 1990.

Hanvey *et al.*, *Science,* 258:1481-1485, 1992.

Harlow. and Lane, *In: Antibodies: A Laboratory Manual,* Cold Spring Harbor Laboratory, Cold Spring Harbor, NY, 1988.

Hyrup and Nielsen, *Bioorg. Med. Chem.*, 1996.

Isola, Alarie, Griffin, Vo-Dinh, "Development of a genosensor for Mycobacterium tuberculosis," *In: Biomedical Sensing, Imaging and Tracking Technologies,* Lieberman, Podbielska, Vo-Dinh, eds, SPIE Publishers, Bellingham, WA, Vol. 2676:228-240. 1996.

Isola, Stokes, Vo-Dinh, "Surface-enhanced Raman gene probe for HIV detection," *Anal. Chem.,* 70:1352-1356, 1998.

Jaeger *et al., Proc. Natl. Acad. Sci. USA ,* 86:7706-7710, 1989.

Jensen *et al., Biochemistry,* 36(16):5072-5077, 1997.

Kaiser and Kezdy, "Amphiphilic secondary structure design of peptide hormones," *Science,* 223(4633):249-255, 1984.

Kashani-Saber *et al., Antisense Res. Dev.,* 2:3-15, 1992.

Koch *et al., Tetrahedron Lett.,* 36:6933-6936, 1995.

Kohler and Milstein, "Derivation of specific antibody-producing tissue culture and tumor lines by cell fusion," *Eur. J. Immunol.,* 6(7):51 1-519, 1976.

Koppelhus, *Nucleic Acids Res.,* 25(11):2167-2173, 1997.

Kremsky *et al., Tetrahedron Lett.,* 37:4313-4316, 1996.

Kuby, *In: Immunology,* 2nd Edition. W.H. Freeman & Company, New York, 1994.

Kumar, Wilson, Valdes, Chambers, "Monitoring oligonucleotide hybridization using light-addressable potentiometric and evanescent wave fluorescence sensing," *Mat. Sci. Eng.*, CI:187-192, 1994.

Kunkel, Roberts, and Zakour, "Rapid and efficient site-specific mutagenesis without phenotypic selection," *Methods Enzymol.*, 154:367-382, 1987.

Kwoh *et al., Proc. Natl. Acad. Sci, USA,* 86(4):1173-1177, 1989.

L'Huillier *et al.*, *EMBO J.*, 11:4411-4418, 1992.

Landsdorp *et al., Hum. Mol. Genet.,* 5:685-691, 1996.

Lieber *et al., Methods Enzymol.,* 217:47-66, 1993.

Lisziewicz *et al., Proc. Nail. Acad. Sci. U. S. A.*, 90:8000-8004, 1993.

Maloy *et al., In: Microbial Genetics,* 2nd Edition. Jones and Barlett Publishers, Boston, MA, 1994.

Maloy, "Experimental Techniques in Bacterial Genetics" Jones and Bartlett Publishers, Boston, MA, 1990.McGall, Barone, Diggelmann, Fodor, Gentalen, Ngo, "The efficiency of light-directed synthesis of DNA arrays on glass substrates," *J. Am. Chem. Soc.,* 119:5081-5090, 1997.

Marttin, J.C. Verhoef and F.W. Merkus, "Efficacy, safety and mechanism of cyclodextrins as absorption enhancers in nasal delivery of peptide and protein drugs", *J. Drug Target,* 6(1):17-36, 1998

Mellors, Rinaldo, Gupta, White, "Prognosis in HIV-1 infection predicted by the quantity of virus in plasma," *Science,* 272:1167-1169, 1996.

Michael, *Biotechniques,* 16:410-412, 1994.

Mollegaard, Buchardt, Egholm, Nielsen, *Proc. Natl. Acad. Sci. USA,* 91:3892-3895, 1994.

Mosbach, "Molecular imprinting", *Trends Biochem. Sci.*, 19(1):9-14, 1994.

Neilsen, *In: Perspectives in Drug Discovery and Design 4*, Escom Science Publishers, pp. 76-84, 1996.

Nielsen, P. E., Egholm, M., Berg, R. H., and Buchardt, O, "Peptide nucleic acids (PNAs): Potential antisense and anti-gene agents," *Anti-Cancer Drug Design,* 8(1):53-63, 1993.

Nielsen, P. E., Egholm, M., Berg, R. H., and Buchardt, O, "Sequence-selective recognition of DNA by strand displacement with a thymine-substituted polyamide," *Science* 254, 1497-1500, 1991.

Norton, Piatyszek, Wright, Shay, Corey, *Nat. Biotechnol.,* 14:615-620, 1996.

Norton, Waggenspack, Varnum, Corey, *Bioorg. Med. Chem.,* 3:437-445, 1995.

Ohara *et al., Proc. Natl. Acad. Sci. USA,* 86(15):5673-5677, 1989.

Ohkawa *et al.*, *Nucl. Acids Symp. Ser.*, 27:15-6, 1992.

Ojwang *et al.*, *Proc. Natl. Acad. Sci. U. S. A.*, 89:10802-10806, 1992. Orum, Nielsen, Egholm, Berg, Buchardt, Stanley, *Nucl. Acids Res.,* 21:5332-5336, 1993.

Orum, Nielsen, Jorgensen, Larsson, Stanley, Koch, *BioTechniques,* 19:472-480, 1995.

Pardridge, Boado, Kang, *Proc. Natl. Acad Sci. USA,* 92:5592-5596, 1995.

Perrotta and Been, *Biochem.,* 31:16, 1992.

Perry-O'Keefe, Yao, Coull, Fuchs, Egholm, *Proc. Natl. Acad. Sci. USA,* 93:14670-14675, 1996.

Petersen, Jensen, Egholm, Nielsen, Buchardt, *Bioorg. Med. Chem. Lett.,* 5:1119-1124, 1995.

Picard and Vo-Dinh, "A multi-optical fiber array with charge-coupled device image detection for parallel processing of light signals and spectra," Rev. *Sci. Instr.*, 62:584, 1991.

Piunno, Krull, Hudson, Damha, Cohen, "Fiber optic biosensor for fluorimetric detection of DNA hybridization," *Anal. Chim. Acta,* 228:205-214, 1995.

Prokop and Bajpai, "Recombinant DNA Technology I," Conference on Progress in Recombinant DNA Technology Applications, Potosi, MI, June 3-8, 1990, *Ann. N.Y. Acad. Sci.,* 646:1-383, 1991.

Qin, D.R. Pahud, Y. Ding, A.U.Bielinska, J.F. Kukowska-Latallo, J.R. Baker, Jr., and J.S. Bromberg, "Efficient transfer of genes into murine cardiac grafts by Starburst polyamidoamine dendrimers", *Hum. Gene Ther.,* 9(4):553-60, 1998.

Rose, *Anal. Chem.,* 65(24):3545-3549, 1993.

Rossi *et al.*, *Aids Res. Hum. Retrovir.*, 8:183, 1992.

Ruskowski *et al.*, *Cancer,* 80(12 Suppl):2699-2705, 1997.

Saiki, Gelfand, Stoffel, Scharf, Higuchi, Horn, Mullis, Erlich, "Prime-directed enzymatic amplification of DNA with a thermostable DNA polymerase," *Science,* 239:487-491, 1988.

Sakthivel, I. Toth and A.T. Florence, "Synthesis and physicochemical properties of lipophilic polyamide dendrimers", *Pharm. Res.,* 15(5):776-82, 1998.

Sarver *et al.*, *Science,* 247:1222-1225, 1990.

Saville and Collins, *Cell,* 61:685-696, 1990.

Saville and Collins, *Proc. Natl. Acad. Sci. USA,* 88:8826-8830, 1991.

Scanlon *et al.*, *Proc. Nail. Acad Sci. USA*, 88:10591-10595, 1991.

Scaringe *et al., Nucl. Acids Res.,* 18:5433-5441, 1990.

Schena, Shalon, Davis, Brown, "Quantitative monitoring of gene expression patterns with a complementary DNA microarray," *Science,* 270:467-470, 1995.

Segal, "Biochemical Calculations" 2nd Edition. John Wiley & Sons, New York, 1976.

Shoemaker, Lashkari, Deval, Morris, Mittman, and Davis, "Quantitative phenotypic analysis of yeast deletion mutants using a highly parallel molecular bar-coding strategy," Nat. Genet., 14(4):450-456, 1996.

Sigust *et al.*, "Surface Immobilization of Biomolecules by Light," *Opt. Eng.*, 34:2338, 1995.

Stetsenko, Lubyako, Potapov, Azhikina, Sverdlov, *Tetrahedron Lett.,* 37:3571-3574, 1996.

Stevenson, Johnson, Vo-Dinh, "Synchronous luminescence: a new detection technique for multiple probes used for DNA sequencing," *Biotechniques,* 16:1104-1110, 1994.

Taira *et al.*, *Nucl. Acids Re*s., 19:5125-5130, 1991.

Thiede, Bayerdorffer, Blasczyk, Wittig, Neubauer, *Nucleic Acids Res.,* 24:983-984, 1996.

Thisted, Just, Petersen, Hyldig-Nielsen, Godtfredsen, *Cell Vision,* 3:358-363, 1996.

Thomson *et al.*, *Tetrahedron,* 51:6179-6194, 1995.

Tomic *et al.*, *Nucl. Acids Res.,* 12:1656, 1990.

Topchieva, P. Mischnick, G. K hn, V.A. Polyakov, S.V. Elezkaya, G.I. Bystryzky and K.I. Karezin, "Novel Derivatives of Cyclodextrins, Modified with Poly (ethylene Oxide and Their Complexation Properties, *Bioconjug. Chem.,* 9(6):976-682, 1998.

Ulmann, Will, Breipohl, Langner, Ryte, *Angew*. *Chem.*, *Int. Ed. Engl.*, 35:2632-2635, 1996.

Upender *et al.*, *Biotechniques,* 18:29-31, 1995.

Usman and Cedergren, *TIBS*, 17:34, 1992.

Ventura *et al.*, *Nucl. Acids Res.,* 21:3249-3255, 1993.

Veselkov, Demidov, Nielsen, Frank-Kamenetskii, *Nucl. Acids Res.,* 24:2483-2487, 1996.

Vickers, Griffith, Ramasamy, Risen, Freier, *Nucl. Acids Res.,* 23:3003-3008, 1995.

Vo-Dinh, Griffin, Sepaniak, "Fiber optic fluoroimmunosensors," *In: Fiber Optic Chemical Sensors and Biosensors,* O. S. Wolfbeis (Ed.), CRC Press, Boca Raton, FL, 1991.

Vo-Dinh, Houck, Stokes, "Surface-enhanced Raman gene probes," *Anal. Chem.,* 66:3379-3383, 1994.

Vo-Dinh, *In*. *Room Temperature Phosphorimetry for Chemical Analysis,* Wiley, New York, 1989.

Vo-Dinh, Isola, Alarie, Landis, Griffin, Allison, "Development of a multiarray biosensor for DNA diagnostics," *Instrum. Sci. Tech.,* 1998 (IN PRESS).

Vo-Dinh, Tromberg, Griffin, Ambrose, Sepaniak, Gardenhire, "Antibody-based fiberoptics biosensor for the carcinogen benzo[a]pyrene," *Appl. Spectrosc.*, 5:735-738, 1987.

Vo-Dinh, Wintenberg, Erickson, Isola, Alarie, "Development of a DNA biochip for gene diagnosis," Proceedings of the Conference on Biomedical Sensing and Imaging Technologies, San Jose, CA, Jan 26-29, 1998.

Walker *et al.*, *Proc. Nail. Acad Sci. USA,* 89(1):392-396, 1992. Wang, Stacy, Binder, Marin-Padilla, Sharpe, Speck, "Disruption of the *Cbfa2* gene causes necrosis and hemorrhaging in the central nervous system and blocks definitive hematopoiesis," *Proc. Natl. Acad. Sci. USA,* 93:3444-3449, 1996a.

Wang, Stacy, Miller, Lewis, Gu, Huang, Bushweller, Bories, Alt, Ryan, Liu, Wynshaw-Boris, Binder, Marin-Padilla, Sharpe, Speck, "The Cbfβ subunit is essential for CBFα2 (AML1) function *in vivo," Cell,* 87:697-708, 1996b.

Watson, J. D. *et al., Molecular Biology of the Gene,* 4th Ed., W. A. Benjamin, Inc., Menlo Park, CA, 1987.

Webb and Hurskainen, *J. Biomol. Screen.,* 1:119-121, 1996.

Weerasinghe *et al., J. Virol.*, 65:5531-5534, 1991.

Wilbur, P.M. Pathare, D.K. Hamlin, K.R. Buhler and R. L. Vessella, "Biotin Reagents for Antibody Pretargeting. 3. Synthesis, Radioiodination, and Evaluation of Biotinylated Starburst Dendrimers" , *Bioconjug. Chem.,* 9(6):813-825, 1998.Seeger *et al., Biotechniques,* 23(3):512-517, 1997.

Wolinsdy, Korber, Neumann *et al.,* "Adaptive evolution of human immunodeficiency virus-type I during natural course of infection," *Science,* 272:537-542, 1996.

Woolf *et al., Proc. Natl. Acad. Sci. USA*, 89:7305-7309, 1992.

Wu, S. J. and Dean, D. H., "Functional significance of loops in the receptor binding domain of *Bacillus thuringiensis* CryIIIA δ-endotoxin," *J Mol. Biol.* 255(4):628-640, 1996.

Yu *et al., Proc. Natl. Acad. Sci. USA*, 90:6340-6344, 1993.

Zhou *et al., Mol. Cell Biol.,* 10:4529-4537, 1990.

## Claims

1. An integrated circuit comprising:

   a) a detector capable of detecting an electromagnetic signal, said detector being integrated with the integrated circuit;
   b) an excitation source of electromagnetic radiation, said source of electromagnetic radiation being integrated with the integrated circuit;
   c) at least one sensing element operably associated with the integrated circuit wherein said sensing element selectively combines with a target molecule; and
   d) means for generating a signal.

2. The integrated circuit of claim 1 wherein the signal generated is detectable in the visible, ultraviolet, infrared or near-infrared range.

3. The integrated circuit of claim 1 further comprising a sampling element.

4. The integrated circuit of claim 1 wherein the sensing element is a molecular probe such as a chemical receptor, a bioreceptor, a polymer, a biopolymer, a biomimetic receptor such as a molecular imprint, a nucleic acid, an antibody, an enzyme, a cell receptor, or one or more cells.

5. The integrated circuit of any of the claims 1 to 4, wherein the excitation source of electromagnetic radiation is a light source selected from the group consisting of a light emitting diode or a laser.

6. The integrated circuit of any of the claims 1 to 5 further comprising a signal amplification and processing system integrated with the integrated circuit.

7. The integrated circuit of claim 6 wherein the signal amplification and processing system comprises a microprocessor and an amplifier.

8. The integrated circuit of any of the claims 1 to 7, wherein the detector capable of detecting an electromagnetic signal comprises photodetectors.

9. The integrated circuit of claim 8 wherein said photodetectors comprise photodiodes or phototransistors.

10. The integrated circuit of claim 8 or 9, wherein said detector further comprises one or more transimpedance amplifiers and low pass filters.

11. The integrated circuit of any of the claims 1 to 10, comprising:

    a) a plurality of excitation sources of electromagnetic radiation;
    b) a plurality of probes optionally tagged with a label that responds to said electromagnetic radiation from said sources by emitting or absorbing electromagnetic responses having a different frequency; and
    c) a plurality of detectors that detect said electromagnetic responses.

12. The integrated circuit of claim 11 wherein said probes are polynucleotide or polypeptide/nucleic acid probes.

13. The integrated circuit of claim 11 or 12 wherein said electromagnetic responses are selected from the group consisting of luminescence scattering, infrared absorption and ultraviolet absorption.

14. The integrated circuit of claim 4 wherein the probes respond to said electromagnetic radiation by emitting luminous responses.

**15.** The integrated circuit of claim 14 wherein the luminous responses are in the visible or near infrared spectral region.

**16.** An apparatus for detecting a polynucleotide, comprising:

a) an integrated circuit according to any of the claims 1 to 15 wherein the excitation source of electromagnetic radiation is a light source and wherein the detector capable of detecting an electromagnetic signal comprises a phototransducer,

b) a surface adapted to hold a sample of a material that may contain the particular nucleic acid sequence, said material containing a probe which has a tag that emits said light in response to light received from the source of light, said surface positioned on the integrated circuit to enable light from the source to be received by said material and light emitted by said tag to be received by said transducer.

**Patentansprüche**

**1.** Integrierter Schaltkreis, umfassend:

a) einen Detektor, mit dem ein elektromagnetisches Signal nachgewiesen werden kann, wobei der Detektor mit dem integrierten Schaltkreis integriert ist;
b) eine elektromagnetische Anregungsstrahlungsquelle, wobei die elektromagnetische Strahlungsquelle mit dem integrierten Schaltkreis integriert ist;
c) wenigstens ein mit dem integrierten Schaltkreis operativ assoziiertes Sensorelement, wobei sich das Sensorelement selektiv mit einem Zielmolekül verbindet; und
d) Mittel zur Erzeugung eines Signals.

**2.** Integrierter Schaltkreis nach Anspruch 1, wobei das erzeugte Signal im sichtbaren, ultravioletten oder infraroten Bereich oder im nahen Infrarotbereich nachweisbar ist.

**3.** Integrierter Schaltkreis nach Anspruch 1, weiterhin ein Probennahmeelement umfassend.

**4.** Integrierter Schaltkreis nach Anspruch 1, wobei es sich bei dem Sensorelement um eine molekulare Sonde, wie z.B. einen chemischen Rezeptor, einen Biorezeptor, ein Polymer, ein Biopolymer, einen biomimetischen Rezeptor wie etwa einen molekularen Abdruck, eine Nukleinsäure, einen Antikörper, ein Enzym, einen Zellrezeptor oder eine oder mehrere Zellen handelt.

**5.** Integrierter Schaltkreis nach einem der Ansprüche 1 bis 4, wobei es sich bei der elektromagnetischen Anregungsstrahlungsquelle um eine Lichtquelle, die aus der Gruppe bestehend aus einer Licht emittierenden Diode bzw. einem Laser ausgewählt ist, handelt.

**6.** Integrierter Schaltkreis nach einem der Ansprüche 1 bis 5, weiterhin ein Signalverstärkungs- und -verarbeitungssystem, das mit dem integrierten Schaltkreis integriert ist, umfassend.

**7.** Integrierter Schaltkreis nach Anspruch 6, wobei das Signalverstärkungs- und -verarbeitungssystem einen Mikroprozessor und einen Verstärker umfaßt.

**8.** Integrierter Schaltkreis nach einem der Ansprüche 1 bis 7, wobei der Detektor, mit dem ein elektromagnetisches Signal nachgewiesen werden kann, Photodetektoren umfaßt.

**9.** Integrierter Schaltkreis nach Anspruch 8, wobei die Photodetektoren Photodioden oder Phototransistoren umfassen.

**10.** Integrierter Schaltkreis nach Anspruch 8 oder 9, wobei der Detektor weiterhin einen oder mehrere Transimpedanzverstärker und Tiefpaßfilter umfaßt.

**11.** Integrierter Schaltkreis nach einem der Ansprüche 1 bis 10, umfassend:

a) mehrere elektromagnetische Anregungsstrahlungsquellen;

b) mehrere Sonden, die gegebenenfalls mit einer Markierung, die auf die elektromagnetische Strahlung aus diesen Quellen reagiert, indem sie elektromagnetische Anworten mit einer unterschiedlichen Frequenz emittiert oder .absorbiert, versehen sind; und

c) mehrere Detektoren, mit denen die elektromagnetischen Antworten nachgewiesen werden.

**12.** Integrierter Schaltkreis nach Anspruch 11, wobei es sich bei den Sonden um Polynukleotid- oder Polypeptid/Nukleinsäure-Sonden handelt.

**13.** Integrierter Schaltkreis nach Anspruch 11 oder 12, wobei die elektromagnetischen Antworten aus der Gruppe bestehend aus Lumineszenzstreuung, Infrarotabsorption und Ultraviolettabsorption ausgewählt sind.

**14.** Integrierter Schaltkreis nach Anspruch 4, wobei die Sonden auf die elektromagnetische Strahlung mit der Emission von Lumineszenzantworten reagieren.

**15.** Integrierter Schaltkreis nach Anspruch 14, wobei die Lumineszenzantworten im sichtbaren Spektralbereich oder nahen Infrarotspektralbereich liegen.

**16.** Vorrichtung zum Nachweis eines Polynukleotids, umfassend:

a) einen integrierten Schaltkreis gemäß einem der Ansprüche 1 bis 15, wobei es sich bei der elektromagnetischen Anregungsstrahlungsquelle um eine Lichtquelle handelt und wobei der Detektor, mit dem ein elektromagnetisches Signal nachgewiesen werden kann, einen Phototransducer umfaßt,

b) eine zum Halten einer Probe eines Materials, das die bestimmte Nukleinsäure enthalten kann, angepasste Oberfläche, wobei das Material eine Sonde mit einem Tag, das das Licht als Antwort auf von der Lichtquelle empfangenes Licht emittiert, enthält, wobei die Oberfläche auf dem integrierten Schaltkreis positioniert wird, so daß das Material Licht von der Quelle und der Transducer von dem Tag emittiertes Licht empfangen kann.

**Revendications**

**1.** Circuit intégré comprenant :

a) un détecteur capable de détecter un signal électromagnétique, ledit détecteur étant intégré au circuit intégré ;

b) une source d'excitation de rayonnement électromagnétique, ladite source de rayonnement électromagnétique étant intégrée au circuit intégré ;

c) au moins un élément sensible associé pour être commandé par le circuit intégré dans lequel ledit élément sensible se combine de manière sélective à une molécule cible ; et

d) des moyens pour générer un signal.

**2.** Le circuit intégré selon la revendication 1, dans lequel le signal généré est détectable dans le domaine du rayonnement visible, ultraviolet, infrarouge ou proche infrarouge.

**3.** Le circuit intégré selon la revendication 1, comprenant en outre un échantillonneur.

**4.** Le circuit intégré selon la revendication 1, dans lequel l'élément sensible est une sonde moléculaire telle qu'un chémorécepteur, un biorécepteur, un polymère, un biopolymère, un récepteur biomimétique tel qu'une empreinte moléculaire, un acide nucléique, un anticorps, un enzyme, un récepteur cellulaire ou une ou plusieurs cellules.

**5.** Le circuit intégré selon l'une quelconque des revendications 1 à 4, dans lequel la source d'excitation de rayonnement électromagnétique est une source optique choisie dans le groupe constitué par une diode électroluminescente ou un laser.

**6.** Le circuit intégré selon l'une quelconque des revendications 1 à 5, comprenant en outre un système d'amplification et de traitement de signaux intégré au circuit intégré.

**7.** Le circuit intégré selon la revendication 6, dans lequel le système d'amplification et de traitement de signaux comprend un microprocesseur et un amplificateur.

**8.** Le circuit intégré selon l'une quelconque des revendications 1 à 7, dans lequel le détecteur capable de détecter un signal électromagnétique comprend des photodétecteurs.

**9.** Le circuit intégré selon la revendication 8, dans lequel lesdits photodétecteurs comprennent des photodiodes ou des phototransistors.

**10.** Le circuit intégré selon la revendication 8 ou 9, dans lequel ledit détecteur comprend en outre un ou plusieurs amplificateurs à transimpédance et filtres passe-bas.

**11.** Le circuit intégré selon l'une quelconque des revendications 1 à 10, comprenant :

a) une pluralité de sources d'excitation de rayonnement électromagnétique ;

b) une pluralité de sondes éventuellement marquées par un traceur qui répond audit rayonnement électromagnétique émanant desdites sources par l'émission ou l'absorption des réponses électromagnétiques ayant une fréquence différente ; et

c) une pluralité de détecteurs qui détectent lesdites réponses électromagnétiques.

**12.** Le circuit intégré selon la revendication 11, dans lequel lesdites sondes sont des sondes à polynucléotides ou polypeptides / acides nucléiques.

**13.** Le circuit intégré selon la revendication 11 ou 12, dans lequel lesdites réponses électromagnétiques sont choisies dans le groupe constitué par la dispersion de luminescence, l'absorption dans l'infrarouge et l'absorption dans l'ultraviolet.

**14.** Le circuit intégré selon la revendication 14, dans lequel les sondes répondent audit rayonnement électromagnétique par l'émission de signaux de réponse lumineux.

**15.** Le circuit intégré selon la revendication 14, dans lequel les signaux de réponse lumineux sont dans la région du spectre visible ou proche infrarouge.

**16.** Appareil de détection d'un polynucléotide, comprenant :

a) un circuit intégré selon l'une quelconque des revendications 1 à 15, dans lequel la source d'excitation de rayonnement électromagnétique est une source optique et dans lequel le détecteur capable de détecter un signal électromagnétique comprend un phototransducteur ;

b) une surface adaptée à supporter un échantillon d'un matériau qui peut contenir la séquence d'acides nucléiques particulière, ledit matériau contenant une sonde qui comprend un indicateur qui émet ladite lumière en réponse à la lumière reçue à partir de la source de lumière, ladite surface étant positionnée sur le circuit intégré de manière à permettre à la lumière émanant de la source d'être reçue par ledit matériau et à la lumière émise par ledit indicateur d'être reçue par ledit transducteur.

FIG. 1

FIG. 2

FIG. 3

FIG. 4

**FIG. 5**

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

**FIG. 22**

**FIG. 23**

FIG. 24

FIG. 25

**FIG. 26**

FIG. 27

**FIG. 28**

FIG. 29

**FIG. 30**

FIG.3.1

602

601

603

604

605

606

607

608

609

EP 1 034 305 B1

FIG. 32

FIG. 33

SEQUENCE LISTING

<110> VO-DINH, TUAN
    WINTENBERG, ALAN
    ERICSON, MILTON N.

<120> INTEGRATED CIRCUIT BIOCHIP MICROSYSTEM

<130> ORNL:024P

<140> UNKNOWN

<141> 1998-11-25

<150> US08/979,762

<151> 1997-11-26

<160> 1

<170> PatentIn Ver. 2.0

<210> 1

<211> 20

<212> DNA

<213> Artificial Sequence

<220>

<223> Description of Artificial Sequence:SYNTHETIC

<400> 1

`cctcctcctt cccagcaggg` 20